# EUROPEAN PATENT APPLICATION

(11) **EP 4 721 774 A2**
(43) Date of publication of application: **08.04.2026**
(21) Application number: 25210362.7
(22) Date of filing: 17.11.2022
(51) Int. Cl.: A61M 1/00

(54) **ASPIRATION THROMBECTOMY SYSTEM**

(30) Priority: 17.11.2021 US 202163264207 P; 11.02.2022 US 202263267897 P
(62) Divisional of application: 22834811.6
(71) Applicant: RapidPulse, Inc., Miami, FL 33186 (US)
(72) Inventor: DEVILLE, Derek, Dee, Coral Gables, FL 33156 (US); PALMER, Matthew, Coral Gables, FL 33156 (US); BALES, William T., Miami, FL 33133 (US); MCBRAYER, Sean, Coral Gables, FL 33133 (US); PETERSEN, Eric, Homestead, FL 33031 (US); CARTLEDGE, Richard, Boca Raton, FL 33432 (US); BALES, Thomas, O., Jr., Miami, FL 33143 (US); RIVERA, Carlos, Cooper City, FL 33330 (US); CALDERON, Alejandro, Miami, FL 33155 (US)
(74) Representative: Forresters IP LLP

(57) **Abstract**

A method of operating an aspiration catheter having a proximal end and a distal end includes the acts of at least partially blocking the distal end of the aspiration catheter with an embolus, creating a vacuum at the distal end of the aspiration catheter with a vacuum source adjacent the proximal end of the aspiration catheter, at least partially relieving the vacuum at the distal end of the catheter by at least one of interrupting the vacuum from the vacuum source and venting the aspiration catheter with a vent fluid adjacent the proximal end, and repeating the acts of interrupting the vacuum and venting the aspiration catheter in a timing cycle that maximizes the time that the aspiration catheter is at vacuum and changes pressure at the distal end of the catheter from vacuum to at least atmospheric pressure during each cycle.

## Description

### FIELD OF THE INVENTION

The present systems, apparatuses, and methods lie in the field of thrombus removal. The present disclosure relates to an aspiration thrombectomy system and methods for thrombus removal with aspiration catheter.

### BACKGROUND

Ischemic strokes are usually caused by a blood clot that blocks or plugs a blood vessel in the brain. This blockage prevents blood from flowing to the brain. Within minutes, brain cells begin to die, which, if not treated rapidly, causes brain damage or death. The costs associated with removing a clot are significant. Most treatments involve thrombectomy: the removal of the clot by aspiration, mechanical retrieval, or some combination thereof.

Removal by aspiration is effected by placing a source of vacuum, e.g., an aspiration or vacuum catheter, upstream of the clot and drawing the clot into or against the distal end of the catheter. Conceptually, aspiration is effective but some significant problems occur in practice. The basic configuration for an aspiration catheter includes a length of hollow catheter having a proximal end fluidically connected to a vacuum or suction pump. In this configuration, operation of the suction pump causes fluid and particulates at the distal end of the catheter to enter the distal opening of the hollow lumen and travel to the proximal end of the lumen near or into the suction pump. Conventional aspiration catheters are threaded through a balloon guide catheter. In one procedure, the balloon of the guide catheter is guided into the internal carotid artery of the brain. The balloon is inflated to occlude the vessel. The aspiration catheter is threaded through the balloon guide catheter and out the distal end of the guide catheter past the balloon. The distal end of the aspiration catheter is advanced to the clot that is occluding the brain vessel. Suction connected to the aspiration catheter is turned on to cause flow reversal. Ideally, this system aspirates the clot entirely out of the neurovasculature and to the proximal end of the aspiration catheter so that extraction and re-establishment of blood flow could be confirmed. In practice, however, this rarely occurs.

Thrombi are frequently of a larger diameter than the catheter being used to aspirate them. For aspiration to be successful, the thrombus must deform to conform to the inner diameter of the aspiration catheter. During conventional aspirations, it is common for applied vacuum to partially draw a thrombus into the distal opening of the aspiration catheter's lumen, thereby deforming some of the thrombus to the catheter's inner diameter. At this point, the thrombus becomes lodged with the aspiration catheter, which is known as corking or being corked. In effect, the suction fixes the clot to the distal end of the catheter. When this occurs, a surgeon can use the aspiration catheter as a tether for pulling the clot out through the balloon guide catheter. However, one challenge of pulling the clot out through the balloon guide catheter is that some or all the clot material can break away uncontrollably. When this occurs, the detached clot material effectively re-embolizes and moves distally within the same vessel or another more distal vessel where it can be more difficult to remove. Such re-embolized clot material can become permanently lodged risking significant permanent brain damage or arterial ruptures.

Another option that has been considered is reversing the suction and pressurizing the clot. However, this risks forcibly and uncontrollably ejecting the clot material out of the distal end of the aspiration catheter. This can cause similar or even more significant complications than using the aspiration catheter as a tether for pulling the clot material proximally. For example, forcibly and uncontrollably ejecting the clot material is more likely to cause the clot material to move further distally within the vessel to a region with a smaller diameter where it is more difficult to remove.

Another challenge of aspirating clot material from a vessel is that it is difficult to confirm that the entire thrombus was removed. A significant disadvantage of current thrombus removal devices is that a surgeon often must fully withdraw a therapeutic device from a patient to ascertain whether the thrombus was adequately removed. For example, even when systems are capable of fully aspirating a thrombus, it may be difficult to assess the extent of thrombus removal because the reservoirs into which aspirated contents are deposited are located outside of the sterile field in an operating room setting where the thrombus is not readily visualized.

To confirm thrombus removal can require the surgeon to attempt aspiration again. The aspiration and balloon guide catheters have to be cleaned out, access to distal anatomy has to be re-established, and, when the aspiration catheter finally is located back at the embolism site, the same issues may be present again with whatever embolus material remains. A disadvantage of these procedures is the significant increase in procedure time, which not only significantly increases the cost (as each minute in an operating room is expensive), it also increases the stress on the operating team, which decreases the success rate of the operation.

The efficacy of thrombectomy systems is often measured using the "first-pass recanalization rate," which is the percentage of procedures in which the system fully removes the thrombus from the vessel with a single insertion of an aspiration catheter. Most current systems offer first-pass recanalization rates of between 30% and 60%. A system that increases the first-pass recanalization rate is valuable and desirable.

Aspiration systems using periodic cycling of vacuum have been described, but before the filing of U.S. Provisional Applications 62/701,086 and 62/750,011 owned by RapidPulse, Inc., such systems did not control positive pressures at the distal end of the catheter to avoid uncontrollably ejecting the thrombus from the distal end of the catheter. When positive pressure exists at the distal end of the aspiration catheter lumen, liquid from inside the lumen exits out from the distal end of the catheter in a distal direction. Such exit flow can drive thrombi from the distal end and risk ejecting thrombi further distally in the vasculature.

Thus, a need exists to overcome the problems with the prior art systems, designs, and processes as discussed above.

### SUMMARY

Aspiration thrombectomy systems and methods for thrombus removal in accordance with the present technology provide high first-pass recanalization rates by completely aspirating the thrombus from the patient.
Some embodiments provide an aspiration thrombectomy system that completely extracts the clot via aspiration so that clots are no longer dragged using the aspiration catheter as tether. The aspiration thrombectomy system suctions the clot material all the way to the proximal end of the vacuum channel and allows the surgeon to confirm recanalization of the vessel (for example, by injecting contrast through the catheter that remains in place after clot removal) and provides structure to indicate to the surgeon that the thrombus has been removed and that flow has been restored.

The aspiration thrombectomy systems can be coupled with conventional aspiration catheters to significantly increase the efficacy of such catheters. Two methods for indicating vacuum level include:
1) The absolute level of pressure, where a "high vacuum" approaches zero absolute pressure. In this "absolute pressure" method of measuring vacuum, a perfect vacuum would be zero and atmospheric pressure would be indicated by measuring the height of a column of mercury that can be supported by a standard atmosphere (760 mm Hg). Hence, lower values indicate an increased level of vacuum relative to the ambient atmospheric pressure.
2) The pressure relative to atmospheric pressure may be indicated, which is known as "gauge pressure." The most common way of measuring pressure in the vacuum realm (below atmospheric pressure) is by using a gauge calibrated so that one atmosphere reads zero (standard atmospheric pressure), and the highest possible level of vacuum in earth's atmosphere would be indicated as "29.92 inches of mercury." Common mechanical vacuum gauges work this way, so this usage has become common.
Herein, "gauge pressure" is used as method of indicating vacuum level; i.e., "zero inches of mercury" means atmospheric pressure (i.e., no suction at all). A high number (e.g., 25 inHg) means a high level of vacuum suction. "Vacuum" as used herein is a condition below normal atmospheric pressure. In the instant application, the units of pressure for vacuum is pounds per square inch ("PSI"), inches of mercury ("inHg") or millimeters of mercury ("mmHg"). Depending on the context of use of the word vacuum, a "high" vacuum is referred to herein as a negative gauge pressure that is significantly lower than atmospheric pressure. Vacuum also refers to negative pressures lower than atmospheric pressure, and a pressure above atmospheric pressure is referred to as a positive pressure. In some instances, however, use of the word "high" with respect to pressure can mean a greater negative or can mean a greater positive based on the context. Likewise, use of the words "low" or "lower" with respect to pressure can mean a lesser negative pressure or a lesser positive pressure based on the context.

For aspiration to fully aspirate the thrombus through the aspiration catheter, thrombus larger than the catheter diameter must deform to fit within the inner diameter of the aspiration catheter. When the thrombus is larger than the inner diameter of the aspiration catheter lumen, it is common for the applied vacuum to draw only a portion of the thrombus into the distal opening of the aspiration catheter lumen. At this point, some of the thrombus becomes stuck within the catheter and some of the thrombus protrudes from the distal end of the catheter.

Some embodiments of aspiration thrombectomy systems in accordance with the present technology prevent such clogging or unclog catheters after being clogged by temporarily halting the vacuum at the distal end of the aspiration catheter, reversing a fluid column in the catheter to push the thrombus distally relative to the catheter, and then reapplying vacuum in a short period of time such that the clot material is recaptured by the vacuum before the clot material is ejected uncontrollably from the distal end of the catheter. This sequence is repeated at a frequency of 2 Hz - 25 Hz, or 4 Hz - 20 Hz, or 6 Hz - 16 Hz, or 8 Hz - 14 Hz, and any suitable value therebetween. Upon reapplying the vacuum each cycle, the thrombus accelerates back into the catheter causing the clot material to deform such that it can be completely aspirated after one or more cycles.

In accordance with an added feature, the aspiration catheter is sized to fit within the Circle of Willis in a brain and the object is a blood clot adjacent the Circle of Willis.

In some embodiments, a clot removal system comprises a catheter having a distal end and a lumen filled configured to be filled with a liquid column having a proximal portion and a distal portion, a controllable vacuum valve, a vacuum source fluidically connected to the vacuum valve, a controllable vent valve having a vent liquid input configured to receive a vent fluid (e.g., a liquid), and a manifold connected to the catheter, the vacuum valve, and the vent valve. The manifold fluidically connects the proximal portion of the liquid column in the lumen to the vacuum source through the vacuum valve and to the vent fluid source through the vent valve. The clot removal system can further comprise a controller connected to the vacuum valve and the vent valve, and the controller is configured to selectively open and close the vacuum valve and the vent valve such that, responsive to opening the vacuum valve, the vacuum source is fluidically connected to the liquid column in the lumen and, responsive to opening the vent valve, the vent fluid source is fluidically connected to the liquid column in the lumen. The controller is also configured to cyclically open and close the vacuum valve and the vent valve such that closing the vacuum valve and opening the vent valve causes a distal shift of the fluid column and closing the vent valve and re-opening vacuum valve are timed to quell the distal shift of the fluid column such that an exit flow out from the distal end of the catheter during each cycle is in a range from at least approximately zero to a limited predetermined volume of liquid so that the reapplied vacuum recaptures the thrombus before the thrombus is uncontrollably ejected from the distal end of the catheter.

In some embodiments, a clot removal system comprises a catheter having a distal end and a lumen configured to be filled with a liquid column having a proximal portion and a distal portion, a controllable vacuum valve, a vacuum source fluidically connected to the vacuum valve, a controllable vent valve having a vent liquid input configured to receive a vent liquid, , and a manifold connected to the catheter, the vacuum valve, and the vent valve. The manifold fluidically connects the proximal portion of the liquid column in the lumen to the vacuum source through the vacuum valve and to a vent fluid source through the vent valve The system also comprises a controller connected to the vacuum valve and the vent valve, and the controller is configured to selectively open and close the vacuum valve and the vent valve such that responsive to opening the vacuum valve, the vacuum source is fluidically connected to the liquid column in the lumen and responsive to opening the vent valve, the vent fluid source is fluidically connected to the liquid column in the lumen. The controller is also configured to cyclically open and close the vacuum valve and the vent valve in a repeated cycle comprising a double-closed state in which the vacuum valve is closed and the vent valve is closed before opening the vacuum valve to reapply vacuum, and a time of the double-closed state is no greater than (a) approximately 10 ms - approximately 50 ms, (b) approximately 20 ms to 40 ms, or (c) approximately 30 ms.

In some embodiments, the catheter is inserted into the vessel while the vacuum valve and the vent valve are in a double-closed state until the distal end of the catheter is at least approximately adjacent to the clot material, and then the controller opens and closes the vacuum valve and the vent valve to initiate cyclic, pulsated aspiration.

In accordance with yet another feature, the controller is configured to selectively open and close the vacuum valve and the vent valve cycle in a repeated cycle comprising a vacuum-only state in which the vacuum valve is open and the vent valve is closed, a first double-closed state in which the vacuum valve is closed and the vent valve is closed, a vent-only state in which the vacuum valve is closed and the vent valve is open, and a second double-closed state in which the vacuum valve is closed and the vent valve is closed.

In accordance with yet a further feature, a time between an opening of the vent valve and a closing of the vent valve is between approximately 10 ms and approximately 50 ms.

In accordance with yet an added feature, a period of the cycle is between approximately 6 Hz and approximately 16 Hz.

In accordance with yet an additional feature, a period of the cycle is between approximately 8 Hz and 12 Hz.

In accordance with again another feature, the change in the level of vacuum at the distal end is greater than approximately 15 inHg in no greater than approximately 50 ms.

In accordance with again another feature, the change in the level of vacuum at the distal end is greater than approximately 20 inHg and no greater than approximately 30 ms; and
In accordance with again another feature, the change in the level of vacuum at the distal end is greater than approximately 25 inHg and no greater than approximately 20 ms.

In accordance with some embodiments, a period of cycle is from approximately 6 Hz to approximately 16 Hz, or approximately 8 Hz to approximately 14 Hz, or approximately 7 Hz to approximately 13 Hz, and the pressure differential is greater than approximately 15 inHg - approximately 25 inHg in less that approximately 20 ms, approximately 25 ms, approximately 30 ms or approximately 50 ms. In some embodiments, the period of cycle is approximately 7 Hz - approximately 15 Hz and the pressure differential is greater than approximately 18 inHg - approximately 22 inHg in less than approximately 15 ms - 25 ms.

In accordance with again an added feature, the lumen has an internal diameter of between approximately 0.038" and approximately 0.106" and the controller is configured to cyclically open and close the vacuum valve and the vent valve at a frequency of between 2 and 16 Hz.

In accordance with again an additional feature, the lumen has an internal diameter of between approximately 0.068" and approximately 0.088" and the controller is configured to cyclically open and close the vacuum valve and the vent valve at a frequency of between 2 and 16 Hz.

In accordance with still another feature, the controller is configured to cyclically open and close the vacuum valve and the vent valve in a repeated cycle by regulating timing of the vent valve.

In accordance with still a further feature, the controller is configured to cyclically open and close the vacuum valve and the vent valve to retain a level of pressure at the distal end at less than physiological pressure.

In accordance with a concomitant feature, there is provided a shaft and the vacuum valve and the vent valve are mounted together on the shaft.

Operation of a Rapid Onset Aspiration Repeater (ROAR) process in accordance with embodiments of the present technology successfully removes thrombi for several reasons. One is that the ROAR effect overcomes the static friction of a clot that is fixed or "stuck" on the catheter tip while under constant suction. Or, in the case of starting the ROAR process, directly without first using constant suction the ROAR effect can avoid static friction that would cause the clot to become fixed to the catheter tip. This is because the ROAR process oscillates the fluid column in the catheter such that the clot "shuttles" back and forth at the distal tip of the catheter to overcome or avoid static frictional force. Second, the oscillation of the fluid column morcellates the clot material at the distal end of the catheter such that different clot morphologies can be aspirated through overriding volume and diameter constraints of small catheters required to access the very narrow, tortuous vessels in the cerebral vasculature.

One aspect of some embodiments of systems and methods disclosed herein is that vacuum valve and the vent valve are operated in predetermine cycle based on the parameters of the catheter system such that the distal displacement of the fluid column in the catheter caused by opening the vent valve is quelled by timing the closing of the vent valve and opening of the vacuum valve such that pressure at the distal end does not reach a positive level that uncontrollably ejects the clot material distally into the vasculature before vacuum is reapplied to recapture the clot material.

In accordance with some embodiments, the distal portion of the liquid column exiting the distal end is limited to no more than approximately 2 microliters to approximately 20 microliters, or approximately 6 microliters to approximately 20 microliters, or approximately 8 microliters to approximately 16 microliters.

In accordance with some embodiments, a clot removal system comprises a catheter having a distal end and a lumen configured to be filled with a liquid column having a proximal portion and a distal portion, a vacuum source, a vent fluid input configured to be coupled to a vent liquid, and a vacuum and vent control system configured to cyclically fluidically connect to and disconnect from the proximal portion at least one of vacuum from the vacuum source and vent liquid from the vent fluid source, and thereby change a level of vacuum at the distal end such that distal displacement of the liquid column is limited to a volume of fluid that exits the distal end whereby reapplication of vacuum is able to recapture the clot material.

In accordance with some embodiments, a clot removal system comprises a catheter having a distal end and a lumen configured to be filled with a liquid column having a proximal portion and a vacuum and vent control system configured to cyclically connect to and disconnect from the proximal portion vacuum and vent fluid to create therein a pressure pulse and thereby reverse flow in the liquid column and substantially prevent the pressure pulse from reaching the distal end.

In accordance with some embodiments, a clot removal system comprises a catheter having a distal end and a lumen configured to be filled with a liquid column having a proximal portion and a vacuum and vent control system configured to cyclically connect to and disconnect from the proximal portion vacuum and vent fluid to create therein a pressure pulse and, before the pressure pulse reaches the distal end, reverse flow in the liquid column and thereby substantially prevent the pressure pulse from reaching the distal end.

In accordance with some embodiments, a clot removal system comprises a catheter having a distal end and a lumen configured to be filled with a liquid column having a proximal portion and a vacuum and vent control system configured to cyclically connect to and disconnect from the proximal portion vacuum and vent fluid and thereby allow the liquid column to move and stop to create therein a pressure pulse and, before the pressure pulse causes more than a limited amount of fluid to exit the distal end, alternate control to reverse flow in the liquid column and thereby control the pressure pulse such that no more than a limited volume of fluid exits the distal end before reapplication of vacuum is unable to recapture the clot material.

In accordance with some embodiments, the controller is configured to change the level of vacuum at the distal end in a cycle while simultaneously preventing distal movement of the distal portion of the liquid column.

In accordance with some embodiments, the controller is configured to selectively open and close the vacuum valve and the vent valve cycle in a repeated cycle comprising a first state in which the vacuum valve is open and the vent valve is closed, a second state in which the vacuum valve is closed and the vent valve is closed, a third state in which the vacuum valve is closed and the vent valve is open, and a fourth state in which the vacuum valve is closed and the vent valve is closed.

In accordance with some embodiments, a clot removal system comprises a catheter having a lumen configured to be filled with a liquid column from a proximal portion to a distal end and a water hammer controller configured to alternatively connect vacuum and/or fluid at atmospheric pressure or body pressure or a different pressure to the lumen, thereby allowing the liquid column to move and stop to create therein a water hammer and alternate control to reverse flow and thereby control the water hammer by substantially preventing the water hammer from causing more than a limited volume of fluid to exit the distal end before reapplication of vacuum is unable to recapture the clot material.

In accordance with some embodiments, a clot removal system comprises a catheter with a lumen, a vacuum source, a controllable vacuum valve, a vent fluid source, a controllable vent valve, a manifold connected to the catheter, to the vacuum valve, and to the vent valve, and a controller controlling the vacuum valve and the vent valve.

In accordance with some embodiments, the controller is configured to modulate the vacuum valve and the vent valve in a cycle that, responsive to vacuum being applied to the catheter, the compliance of the catheter causes a reduction in volume such that, when the vacuum is closed and the vent is open, the compliance acts as a spring and the lumen ingests vent fluid in a distal direction and, before a momentum induced by the ingested fluid cause more than a limited volume of fluid to exit the distal end of the catheter, the controller modulates the valves to reverse a direction and quell movement of the fluid of the fluid and prevent clot material from being uncontrollably ejected from the distal end of the catheter.

In accordance with some embodiments, the clot removal system comprises a fixed cycle with plurality of pinch valves and plurality of cams mechanically coupled to the valves so that orientations of the cams cannot be changed.

Although the systems, apparatuses, and methods are illustrated and described herein as embodied in an aspiration thrombectomy system and methods for thrombus removal with aspiration catheter, it is, nevertheless, not intended to be limited to the details shown because various modifications and structural changes may be made therein without departing from the spirit of the invention and within the scope and range of equivalents of the claims. Additionally, well-known elements of embodiments will not be described in detail or will be omitted so as not to obscure the relevant details of the systems, apparatuses, and methods.

Additional advantages and other features characteristic of the systems, apparatuses, and methods will be set forth in the detailed description that follows and may be apparent from the detailed description or may be learned by practice of embodiments. Still other advantages of the systems, apparatuses, and methods may be realized by any of the instrumentalities, methods, or combinations particularly pointed out in the claims.

Other features that are considered as characteristic for the systems, apparatuses, and methods are set forth in the appended claims. As required, detailed embodiments of the systems, apparatuses, and methods are disclosed herein; however, it is to be understood that the disclosed embodiments are merely examples of the systems, apparatuses, and methods, which can be embodied in various forms. Therefore, specific structural and functional details disclosed herein are not to be interpreted as limiting, but merely as a basis for the claims and as a representative basis for teaching one of ordinary skill in the art to variously employ the systems, apparatuses, and methods in virtually any appropriately detailed structure. Further, the terms and phrases used herein are not intended to be limiting; but rather, to provide an understandable description of the systems, apparatuses, and methods. While the specification concludes with claims defining the systems, apparatuses, and methods of the invention that are regarded as novel, it is believed that the systems, apparatuses, and methods will be better understood from a consideration of the following description in conjunction with the drawing figures, in which like reference numerals are carried forward.

### BRIEF DESCRIPTION OF THE DRAWINGS

The accompanying figures, which are not necessarily to scale, form part of the specification and disclose various principles and advantages of systems, apparatuses, and methods of the present technology. Like reference numbers can refer to identical or similar features that have similar structure and functionality through the separate views. Advantages of embodiments of the systems, apparatuses, and methods will be apparent from the following detailed description, which description should be considered in conjunction with the accompanying drawings in which:
FIG. 1 is a fragmentary, perspective view of some embodiments of a controller for a thrombectomy aspiration catheter in an unactuated state;
FIG. 2 is a fragmentary, perspective, longitudinal cross-sectional view of the controller of FIG. 1;
FIG. 3 is an enlarged, diagrammatic, side elevational view of a compression cam assembly of the controller of FIG. 1;
FIG. 4 is a fragmentary, longitudinal cross-sectional view of the controller of FIG. 1 with a compression roller removed;
FIG. 5 is a fragmentary, longitudinal cross-sectional view of the controller of FIG. 1 in an actuated state;
FIG. 6 is a fragmentary, enlarged, perspective view of a portion of an extrusion compressor of the controller of FIG. 1;
FIG. 7 is a fragmentary, perspective view of the controller of FIG. 1 in the actuated state;
FIG. 8 is a fragmentary, perspective and partially longitudinal cross-sectional view of the controller of FIG. 7;
FIG. 9 is a fragmentary, perspective and longitudinal cross-sectional view of the controller of FIG. 1 in an intermediate actuated state with the compression roller occluding the aspiration catheter and partially rolled to cause fluid column shift;
FIG. 10 is a fragmentary, longitudinal cross-sectional view of the controller of FIG.
FIG. 11 is a fragmentary, perspective and longitudinal cross-sectional view of the controller of FIG. 1 in the actuated state with the compression roller occluding the aspiration catheter and fully rolled to cause fluid column shift;
FIG. 12 is a fragmentary, longitudinal cross-sectional view of the controller of FIG. 11;
FIG. 13 is a longitudinal cross-sectional view of the controller of FIG. 9 with the compression roller and the aspiration catheter removed;
FIG. 14 is a fragmentary, perspective and longitudinal cross-sectional view of the controller of FIG. 1 in the unactuated state and diagrammatically connected to a distal portion of the aspiration catheter with a thrombus lodged in a distal opening of a vacuum channel;
FIG. 15 is a fragmentary, perspective and longitudinal cross-sectional view of the controller of FIG. 12 in the actuated state with the column shift that distally dislodges the thrombus from the distal opening of the vacuum channel;
FIG. 16 is a fragmentary, enlarged, perspective and longitudinal cross-sectional view of a distal portion of the controller of FIG. 1 and some embodiments of a vacuum booster disposed between the controller and a distal extent of the aspiration catheter with the vacuum booster in an energized state;
FIG. 17 is a fragmentary, enlarged, perspective and longitudinal cross-sectional view of the controller and the vacuum booster of FIG. 16 with the vacuum booster in a relaxed state;
FIG. 18 is a fragmentary, enlarged, perspective and partially transparent view of a proximal portion of the controller of FIG. 1 and some embodiments of a thrombus trap;
FIG. 19 is a fragmentary, enlarged, perspective view of the controller and thrombus trap of FIG. 18 with the intermediate shell of the thrombus trap removed;
FIG. 20 is a fragmentary, enlarged, perspective view of the controller and thrombus trap of FIG. 18;
FIG. 21 is a fragmentary, perspective and longitudinal cross-sectional view of the controller of FIG. 16, and the thrombus trap of FIG. 18;
FIG. 22 is a fragmentary, longitudinal cross-sectional view of some embodiments of a volume changing controller;
FIG. 23 is a vacuum circuit diagram of some embodiments of a vacuum booster and vacuum booster control device;
FIG. 24 is a cycle flow diagram of the operation of some embodiments of the controller with the vacuum booster and the thrombus trap;
FIG. 25 is a perspective view of an automatic aspiration thrombectomy system to be connected distally to an aspiration catheter and proximally to vacuum and vent lines and with a cam housing removed;
FIG. 26 is a fragmentary, top plan view of the aspiration thrombectomy system of FIG. 25 with diagrammatic illustration of the aspiration catheter and the vacuum and vent lines;
FIG. 27 is an elevational view of a proximal side of the aspiration thrombectomy system of FIG. 25;
FIG. 28 is an elevational view of a bearing side of the aspiration thrombectomy system of FIG. 25;
FIG. 29 is a perspective and longitudinally cross-sectional view of the aspiration thrombectomy system of FIG. 25 with a vacuum valve in a closed state, a vent valve in an open state, and a flag of a positional reset assembly in a zero-reset state;
FIG. 30 is a longitudinally cross-sectional view of the aspiration thrombectomy system of FIG. 29;
FIG. 31 is an enlarged cross-sectional view of a valve and cam set of the aspiration thrombectomy system of FIG. 25 with the cam in a rotational position to set an intermediate closing of the valve;
FIG. 32 is an enlarged cross-sectional view of the valve and cam set of FIG. 31 with the cam in a rotational position to close the valve;
FIG. 33 is a cross-sectional view of the aspiration thrombectomy system of FIG. 25 along section line 33-33 in FIG. 30 with the cam housing removed;
FIG. 34 is a perspective view of the aspiration thrombectomy system of FIG. 25 with the motor assembly housing removed;
FIG. 35 is a top plan view of the aspiration thrombectomy system of FIG. 26 with the motor assembly housing removed;
FIG. 36 is an elevational view of the aspiration thrombectomy system of FIG. 27 with the motor assembly housing removed;
FIG. 37 is an elevational view of the bearing side of the aspiration thrombectomy system of FIG. 28 with the motor assembly housing removed;
FIG. 38 is a perspective view of the aspiration thrombectomy system of FIG. 25 with the cam housing;
FIG. 39 is a top plan view of the aspiration thrombectomy system of FIG. 26 with the cam housing;
FIG. 40 is an elevational view of the aspiration thrombectomy system of FIG. 27 with the cam housing;
FIG. 41 is an elevational view of the bearing side of the aspiration thrombectomy system of FIG. 28 with the cam housing;
FIG. 42 is a fragmentary, partially hidden, perspective view of some embodiments of a rotational pintle valve to be employed with the aspiration thrombectomy system in a first valve state;
FIG. 43 is a fragmentary, cross-sectional view of the valve of FIG. 42;
FIG. 44 is a fragmentary, partially hidden, perspective view of the valve of FIG. 42;
FIG. 45 is a fragmentary, partially hidden, perspective view of the valve of FIG. 42 in a second valve state;
FIG. 46 is a fragmentary, cross-sectional view of the valve of FIG. 45;
FIG. 47 is a diagrammatic, cross-sectional view of some embodiments of an aspiration thrombectomy system;
FIG. 48 is a graph of some embodiments of a waveform for operating the system of FIG. 47 with a ROAR process to quell pressure pulses;
FIG. 49 is a graph illustrating some embodiments of one cycle of a waveform operation of a vacuum valve and a vent valve of the system of FIG. 47;
FIG. 50 is a graph illustrating pressure curves at a proximal portion and a distal portion of a lumen of a catheter of the system of FIG. 47 operating with the waveform of FIG. 49;
FIG. 51 is a graph illustrating the waveforms of FIG. 49 and 50 combined together in time;
FIG. 52 is a graph of some embodiments of a waveform for operating the system of FIG. 47 with a ROAR process to quell pressure pulses;
FIG. 53 is a graph illustrating positions of the vacuum and vent valves of the system of FIG. 47 for tuning the valves to create a ROAR effect;
FIG. 54 is a fragmentary, longitudinal cross-sectional view of a proximal manifold connector assembly for the system of FIG. 47;
FIG. 55 is a block diagram of some embodiments of a self-contained, aspiration thrombectomy system;
FIG. 56 is a block diagram of the system of FIG. 55 with some embodiments of a proximal manifold connector assembly having remote controls;
FIG. 57 is a perspective view of some embodiments of a self-contained, aspiration thrombectomy system with a collection canister and a vent liquid reservoir indicated diagrammatically;
FIG. 58 is a fragmentary, perspective view of a cassette connection assembly of the system of FIG. 57;
FIG. 59 is a top plan view of the system of FIG. 57;
FIG. 60 is a left side elevational view of the system of FIG. 57;
FIG. 61 is a right side elevational view of the system of FIG. 57;
FIG. 62 is a perspective view of some embodiments of a self-contained, aspiration thrombectomy system with a collection canister and a hanging vent liquid reservoir indicated diagrammatically;
FIG. 63 is a left side elevational view of the system of FIG. 62;
FIG. 64 is a right side elevational view of the system of FIG. 62;
FIG. 65 is a top plan view of the system of FIG. 62;
FIG. 66 is a front elevational view of the system of FIG. 57;
FIG. 67 is a fragmentary, front perspective view of cassette connection assembly and the hanging vent liquid reservoir of the system of FIG. 57;
FIG. 68 is a top plan view of some embodiments of a valve cassette for the systems of FIGS. 57 to 67 with hidden line views of fluid lumens;
FIG. 69 is a bottom plan view of the valve cassette of FIG. 69;
FIG. 70 is a bottom perspective view of the valve cassette of FIG. 69;
FIG. 71 is a bottom perspective view of the valve cassette of FIG. 69;
FIG. 72 is a diagrammatic illustration of some embodiments of a self-contained, aspiration thrombectomy system;
FIG. 73 is a fragmentary, enlarged portion of the graph of FIG. 50;
FIG. 74 is a fragmentary portion of a graph of characteristic pressure traces in the aspiration thrombectomy system when first contacting a clot at a distal end of a catheter;
FIG. 75 is a fragmentary portion of a graph indicating a pressure level at a distal end of a catheter operating with ROAR aspiration;
FIG. 76 is a fragmentary, enlarged portion of the graph of FIG. 75 showing approximately one cycle thereof;
FIG. 77 is a fragmentary portion of a graph indicating a pressure level at a distal end of a catheter operating with ROAR aspiration after pressure optimization tuning;
FIG. 78 is a fragmentary, enlarged portion of the graph of FIG. 77 showing approximately one cycle thereof;
FIG. 79 is a vertical cross-section of a geometric shape of some embodiments of a distal end of a valve actuator;
FIG. 80 is a vertical cross-section of a geometric shape of some embodiments of a distal end of a valve actuator;
FIG. 81 is a vertical cross-section of a geometric shape of some embodiments of a distal end of a valve actuator;
FIG. 82 is a vertical cross-section of a geometric shape of some embodiments of a distal end of a valve actuator;
FIG. 83 is a vertical cross-section of a geometric shape of some embodiments of a distal end of a valve actuator;
FIG. 84 is a diagrammatic illustration of some embodiments of a bubble test configuration of a self-contained, aspiration thrombectomy system;
FIG. 85 is a cross-section of some embodiments of a testbed for performing ROAR aspiration on a simulated clot;
FIG. 86 is a cross-section of another embodiment of a testbed for performing ROAR aspiration on a simulated clot;
FIG. 87 is a cross-section of a further embodiment of a testbed for performing ROAR aspiration on a simulated clot;
FIG. 88 is a perspective view of some embodiments of an aspiration thrombectomy system with a pole in a raised position and with an empty cassette slot;
FIG. 89 is a right side elevational view of the aspiration thrombectomy system of FIG. 88 with the pole in an intermediate position;
FIG. 90 is a front elevational view of the aspiration thrombectomy system of FIG. 88;
FIG. 91 is a rear elevational view of the aspiration thrombectomy system of FIG. 88;
FIG. 92 is a top plan view of the aspiration thrombectomy system of FIG. 88 with a vacuum canister removed;
FIG. 93 is a top plan view of the aspiration thrombectomy system of FIG. 88;
FIG. 94 is a bottom plan view of the aspiration thrombectomy system of FIG. 88;
FIG. 95 is a front elevational view of the aspiration thrombectomy system of FIG. 88 with the vacuum canister removed;
FIG. 96 is a perspective view of the aspiration thrombectomy system of FIG. 88 with the vacuum canister removed;
FIG. 97 is a left side elevational view of the aspiration thrombectomy system of FIG. 88 with a vacuum canister removed;
FIG. 98 is a vertical cross-sectional and perspective view of the vacuum canister of the aspiration thrombectomy system of FIG. 88 with a short float valve assembly having a float valve in a nearly closed position;
FIG. 99 is a vertical cross-sectional view of the vacuum canister of the aspiration thrombectomy system of FIG. 88 from a left side thereof with a tall float valve assembly having a float valve in a nearly closed position;
FIG. 100 is a left side elevational view of the vacuum canister of the aspiration thrombectomy system of FIG. 88;
FIG. 101 is a perspective view of the vacuum canister of the aspiration thrombectomy system of FIG. 88;
FIG. 102 is a front elevational view of the vacuum canister of the aspiration thrombectomy system of FIG. 88;
FIG. 103 is a rear elevational view of the vacuum canister of the aspiration thrombectomy system of FIG. 88;
FIG. 104 is a top plan view of a clot catching filter of the vacuum canister of the aspiration thrombectomy system of FIG. 88;
FIG. 105 is a top plan view of the vacuum canister of the aspiration thrombectomy system of FIG. 88;
FIG. 106 is a bottom plan view of the vacuum canister of the aspiration thrombectomy system of FIG. 88;
FIG. 107 is a perspective view of the clot catching filter of the vacuum canister of the aspiration thrombectomy system of FIG. 88;
FIG. 108 is a front perspective view of the clot catching filter of the vacuum canister of the aspiration thrombectomy system of FIG. 88;
FIG. 109 is a left side perspective view of the clot catching filter of the vacuum canister of the aspiration thrombectomy system of FIG. 88;

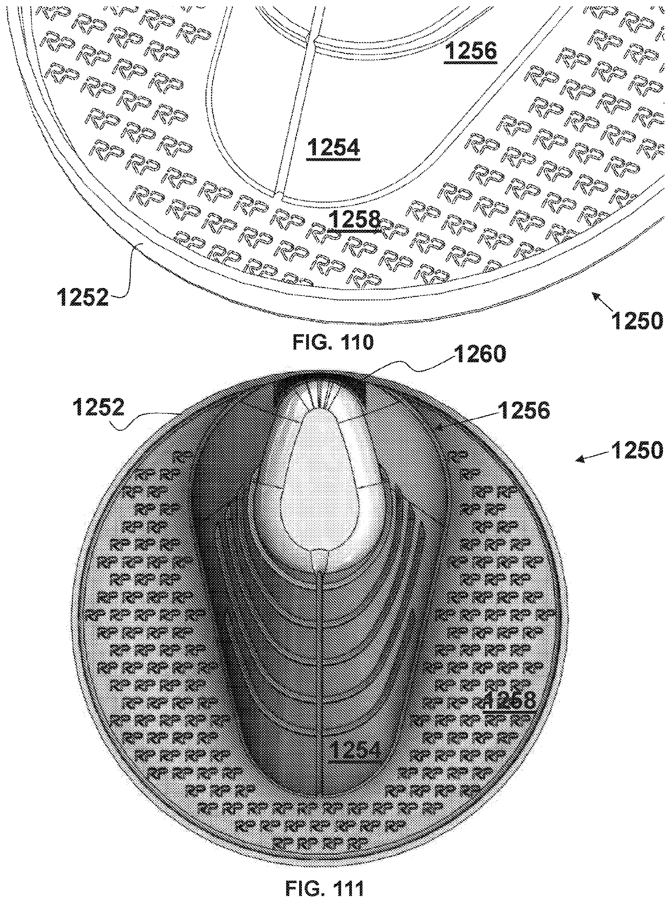

FIG. 110 is a fragmentary, top perspective view of a portion of an alternative embodiment of the clot catching filter of the vacuum canister of the aspiration thrombectomy system of FIG. 88;
FIG. 111 is a top plan view of the clot catching filter of FIG. 110;
FIG. 112 is a fragmentary, perspective view of some embodiments of a distal portion of a vacuum extension line with a remote control pendant of the aspiration thrombectomy system of FIG. 88;
FIG. 113 is an enlarged, plan view of the distal end of remote control pendant of FIG. 112;
FIG. 114 is a fragmentary, front elevational view of the distal portion of the vacuum extension line and remote control pendant of FIG. 112;
FIG. 115 is a fragmentary, side elevational view of the distal portion of the vacuum extension line and remote control pendant of FIG. 112;
FIG. 116 is a fragmentary, perspective view of another embodiment of a distal portion of a vacuum extension line with a remote control pendant of the aspiration thrombectomy system of FIG. 88 and some embodiments of a proximal manifold connector assembly or connection hub of a ROAR catheter;
FIG. 117 is a fragmentary, front elevational view of the distal portion of the vacuum extension line and remote control pendant of FIG. 116;
FIG. 118 is a fragmentary, side elevational view of the distal portion of the vacuum extension line and remote control pendant of FIG. 116;
FIG. 119 is a vertical cross-sectional elevational view of the aspiration thrombectomy system of FIG. 88 from a left side thereof with a cassette in the cassette slot;
FIG. 120 is a vertical cross-sectional perspective view of the aspiration thrombectomy system of FIG. 119;
FIG. 121 is a vertical cross-sectional elevational view of the aspiration thrombectomy system of FIG. 88 from a front side thereof with a cassette in the cassette slot;
FIG. 122 is a fragmentary, perspective view of some embodiments of an cassette assembly from above;
FIG. 123 is a fragmentary, perspective and hidden view of the cassette assembly of FIG. 122;
FIG. 124 is a fragmentary, top plan view of the cassette assembly of FIG. 122 with a top half removed;
FIG. 125 is a fragmentary, top plan and hidden view of the cassette assembly of FIG. 122;
FIG. 126 is a fragmentary, bottom plan view of the cassette assembly of FIG. 122;
FIG. 127 is a fragmentary, bottom plan and hidden view of the cassette assembly of FIG. 122;
FIG. 128 is a plan and hidden view of the cassette assembly of FIG. 122 from a side of the cassette facing an entrance of the cassette slot;
FIG. 129 is a fragmentary, exploded, and diagrammatic perspective view of a proximal manifold connector catheter hub and radio-frequency identification assembly for a catheter;
FIG. 130 is a top plan view of the proximal manifold connector catheter hub and a distal portion of a radio-frequency identification assembly of FIG. 129;
FIG. 131 is a distal plan view of the proximal manifold connector catheter hub and distal portion of the radio-frequency identification assembly of FIG. 130;
FIG. 132 is a side elevational view of the proximal manifold connector catheter hub and distal portion of the radio-frequency identification assembly of FIG. 130;
FIG. 133 is an exploded perspective view of the proximal manifold connector catheter hub and distal portion of the radio-frequency identification assembly of FIG. 130;
FIG. 134 is an exploded top plan view of the proximal manifold connector catheter hub and distal portion of the radio-frequency identification assembly of FIG. 130;
FIG. 135 is an exploded side elevational view of the proximal manifold connector catheter hub and distal portion of the radio-frequency identification assembly of FIG. 130;
FIG. 136 is a fragmentary, perspective view of some embodiments of an aspiration thrombectomy system with a pole in a raised position, with a cassette outside a cassette slot, with power and vacuum lumens partially fragmented, and with a two-part, rotatable, remote-control pendant; and
FIG. 137 is a fragmentary, exploded, enlarged view of the remote-control pendant of FIG. 136.

### DETAILED DESCRIPTION

Systems, apparatuses, and methods of the present technology are disclosed herein; however, the disclosed embodiments are merely examples of various embodiments of the present technology. Therefore, specific structural and functional details disclosed herein are not to be interpreted as limiting, but merely as a basis for the claims and as a representative basis for teaching one skilled in the art to variously employ the systems, apparatuses, and methods in virtually any appropriately detailed structure. Further, the terms and phrases used herein are not intended to be limiting; but rather, to provide an understandable description of the systems, apparatuses, and methods. While the specification concludes with claims defining the features of the systems, apparatuses, and methods that are regarded as novel, it is believed that the systems, apparatuses, and methods will be better understood from a consideration of the following description in conjunction with the drawing figures, in which like reference numerals are carried forward.

In the following detailed description, reference is made to the accompanying drawings which form a part hereof, and in which are shown by way of illustration embodiments that may be practiced. It is to be understood that other embodiments may be utilized and structural or logical changes may be made without departing from the scope. Therefore, the following detailed description is not to be taken in a limiting sense, and the scope of embodiments is defined by the appended claims and their equivalents.

Alternate embodiments may be devised without departing from the spirit or the scope of the invention. Additionally, well-known elements of embodiments of the systems, apparatuses, and methods will not be described in detail or will be omitted so as not to obscure the relevant details of the systems, apparatuses, and methods.

Before the systems, apparatuses, and methods are disclosed and described, it is to be understood that the terminology used herein is for the purpose of describing particular embodiments only and is not intended to be limiting. The terms "comprises," "comprising," or any other variation thereof are intended to cover a non-exclusive inclusion, such that a process, method, article, or apparatus that comprises a list of elements does not include only those elements but may include other elements not expressly listed or inherent to such process, method, article, or apparatus. An element proceeded by "comprises ... a" does not, without more constraints, preclude the existence of additional identical elements in the process, method, article, or apparatus that comprises the element. The terms "including" and/or "having," as used herein, are defined as comprising (i.e., open language). The terms "a" or "an", as used herein, are defined as one or more than one. The term "plurality," as used herein, is defined as two or more than two. The term "another," as used herein, is defined as at least a second or more. The description may use the terms "embodiment" or "embodiments," which may each refer to one or more of the same or different embodiments.

The terms "coupled" and "connected," along with their derivatives, may be used. It should be understood that these terms are not intended as synonyms for each other. Rather, in particular embodiments, "connected" may be used to indicate that two or more elements are in direct physical or electrical contact with each other. "Coupled" may mean that two or more elements are in direct physical or electrical contact (e.g., directly coupled). However, "coupled" may also mean that two or more elements are not in direct contact with each other, but yet still cooperate or interact with each other (e.g., indirectly coupled).

For the purposes of the description, a phrase in the form "A/B" or in the form "A and/or B" or in the form "at least one of A and B" means (A), (B), or (A and B), where A and B are variables indicating a particular object or attribute. When used, this phrase is intended to and is hereby defined as a choice of A or B or both A and B, which is similar to the phrase "and/or". Where more than two variables are present in such a phrase, this phrase is hereby defined as including only one of the variables, any one of the variables, any combination of any of the variables, and all of the variables, for example, a phrase in the form "at least one of A, B, and C" means (A), (B), (C), (A and B), (A and C), (B and C), or (A, B and C).

Relational terms such as first and second, top and bottom, and the like may be used solely to distinguish one entity or action from another entity or action without necessarily requiring or implying any actual such relationship or order between such entities or actions. The description may use perspective-based descriptions such as up/down, back/front, top/bottom, and proximal/distal. Such descriptions are merely used to facilitate the discussion and are not intended to restrict the application of disclosed embodiments. Various operations may be described as multiple discrete operations in tum, in a manner that may be helpful in understanding embodiments; however, the order of description should not be construed to imply that these operations are order dependent.

As used herein, the term "about" or "approximately" applies to all numeric values, whether or not explicitly indicated. These terms generally refer to a range of numbers that one of skill in the art would consider equivalent to the recited values (i.e., having the same function or result). In many instances these terms may include numbers that are rounded to the nearest significant figure. As used herein, the terms "substantial" and "substantially" means, when comparing various parts to one another, that the parts being compared are equal to or are so close enough in dimension that one skill in the art would consider the same. Substantial and substantially, as used herein, are not limited to a single dimension and specifically include a range of values for those parts being compared. The range of values, both above and below (e.g., "+/" or greater/lesser or larger/smaller), includes a variance that one skilled in the art would know to be a reasonable tolerance for the parts mentioned.

It will be appreciated that embodiments of the systems, apparatuses, and methods described herein may be comprised of one or more conventional processors and unique stored program instructions that control the one or more processors to implement, in conjunction with certain non-processor circuits and other elements, some, most, or all of the functions of the systems, apparatuses, and methods described herein. The non-processor circuits may include, but are not limited to, signal drivers, clock circuits, power source circuits, and user input and output elements. Alternatively, some or all functions could be implemented by a state machine that has no stored program instructions, or in one or more application specific integrated circuits (ASICs) or field-programmable gate arrays (FPGA), in which each function or some combinations of certain of the functions are implemented as custom logic. Of course, a combination of these approaches could also be used. Thus, methods and means for these functions have been described herein.

The terms "program," "software," "software application," and the like as used herein, are defined as a sequence of instructions designed for execution on a computer system or programmable device. A "program," "software," "application," "computer program," or "software application" may include a subroutine, a function, a procedure, an object method, an object implementation, an executable application, an applet, a servlet, a source code, an object code, any computer language logic, a shared library/dynamic load library and/or other sequence of instructions designed for execution on a computer system.

Herein various embodiments of the systems, apparatuses, and methods are described. In many of the different embodiments, features are similar. Therefore, to avoid redundancy, repetitive description of these similar features may not be made in some circumstances. It shall be understood, however, that description of a first-appearing feature applies to the later described similar feature and each respective description, therefore, is to be incorporated therein without such repetition.

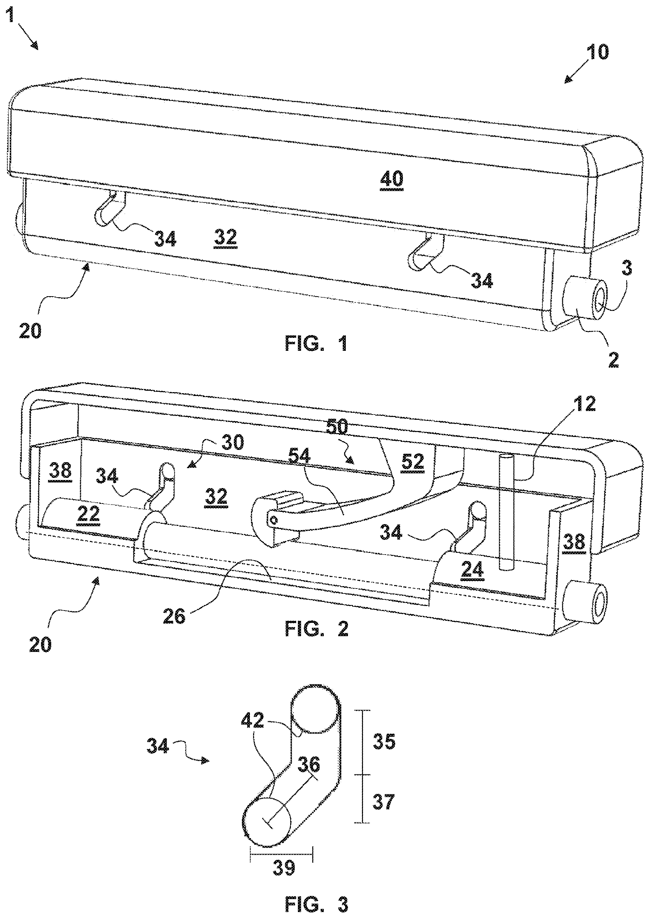

Described now are several embodiments of the present technology. Referring now to the figures of the drawings in detail and first, particularly to FIGS. 1 to 13, there is shown an embodiment of a one-handed controller 10 for an aspiration thrombectomy system 1 utilizing a vacuum tube 2. The controller 10 comprises a first handle part 20 and a second handle part 40. The first handle part 20 is connected to and holds the vacuum tube 2 and, therefore, is also referred to as a handle base. The second handle part 40 moves with respect to the first handle part 20 and, therefore, the second handle part 40 is also referred to as a compressor-actuator 40.

In some embodiments, the first handle part 20 has a distal tube anchor 22 and a proximal tube anchor 24. In this embodiment, the distal and proximal tube anchors 22, 24 are in the form of hollow tubes through which the vacuum tube 2 traverses. The distal and proximal tube anchors 22, 24 hold the vacuum tube 2 therein substantially without compressing the vacuum tube 2 (and thereby does not reduce or close the inner vacuum channel 3). The vacuum tube 2 can be of many materials, including latex, silicone, Pebax^{®}, polyurethane, polyvinyl chloride, or other synthetic rubber. Suitable sizes for the vacuum tube 2 have an inner diameter (I.D.) of approximately 0.055 to 0.095 inches. Some embodiments for retaining the vacuum tube 2 is an adhesive that bonds the material of the vacuum tube 2 to the interior lumens of the tubular tube anchors 22, 24. In such embodiments, the vacuum tube 2 is fixed to the first handle part 20. In an alternative embodiment, the first handle part 20 is a clamshell having two first handle part halves (not illustrated) that open to receive the cylindrical vacuum tube 2 and, when closed thereupon, the tube anchors 22, 24 tightly grip the vacuum tube 2 therein substantially without closing or occluding the vacuum channel 3 of the vacuum tube 2. In a clamshell embodiment, the first handle part 20 is split horizontally at the dashed line in FIG. 2 with a hinge, allowing a portion of the vacuum tube 2 to be inserted into and removed from the distal and proximal tube anchors 22, 24. A lock secures the vacuum tube 2 therein until the user desires removal. The hinge is useful to allow the surgeon to reposition the controller 10 along the vacuum tube 2.

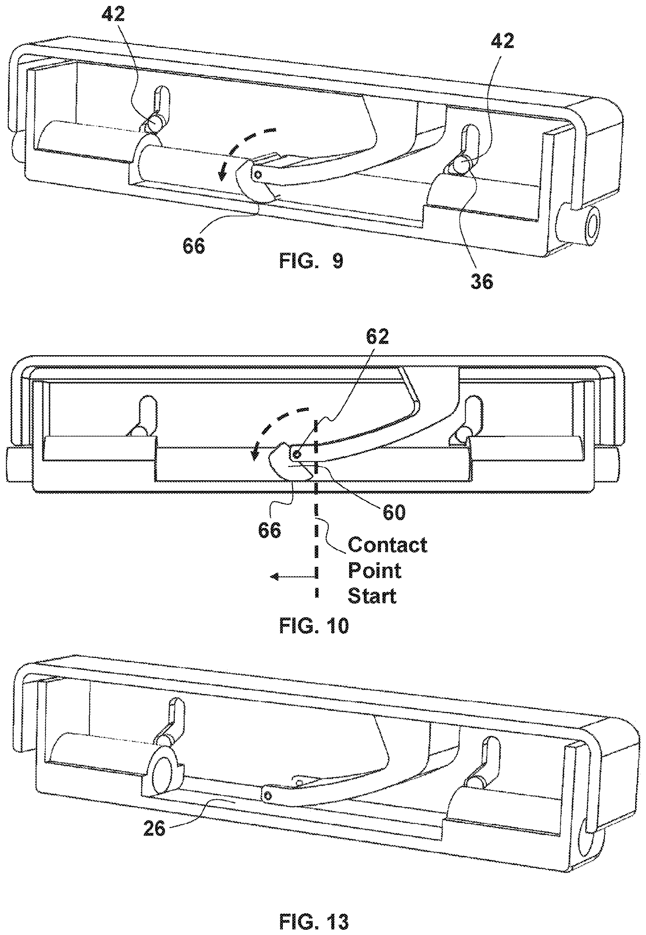

The distal and proximal tube anchors 22, 24 can be separated from one another over a distance. Between the distal and proximal tube anchors 22, 24 of the first handle part 20 is a compression floor 26. When installed within the first handle part 20, the vacuum tube 2 lays against the compression floor 26 between the distal and proximal tube anchors 22, 24 substantially without closing or occluding the vacuum channel 3. FIG. 13 illustrates the compression floor 26 of first handle part 20 with the vacuum tube 2 removed.

The first handle part 20 has a hollow interior that defines a set of parallel lateral walls 32 on either side of the vacuum tube 2. The first handle part 20 comprises a compression cam assembly 30 that permits the second handle part 40 to move in two directions with respect to the first handle part 20. More specifically, in some embodiments, the compression cam assembly 30 comprises a set of slots 34 formed in the lateral walls 32 of the first handle part 20. As shown in the enlarged view of FIG. 3, these slots 34 have a vertical extent 35 and an angled extent 36. The vertical extent 35 has a vertical length and the angled extent 36 has a vector length that is comprised of a second vertical extent 37 and a horizontal extent 39. Accordingly, as explained below, the slots 34 provide a cam surface for movement of the second handle part 40 in the same shape as the slot 34.

In some embodiments, to contact the first and second handle parts 20, 40 together, the second handle part 40 has a hollow interior into which the first handle part 20 is inserted and projects. (In an alternative embodiment, the first handle part 20 has a hollow interior into which the second handle part 40 is inserted and projects.) A width between interior facing lateral surfaces of the hollow compartment of the second handle part 40 is approximately equal to the width of the exterior surfaces of the lateral walls 32 such that the second handle part 40 can move up and down on the first handle part 20 tightly but smoothly with little or substantially no friction. In comparison, the length between interior facing longitudinal surfaces of the hollow compartment of the second handle part 40 is greater than the length of the exterior surfaces of the longitudinal walls 38. The difference in length is sufficiently long enough to allow the second handle part 40 to move along the horizontal extent 39 longitudinally parallel with the vacuum tube 2 throughout the horizontal extent 39.

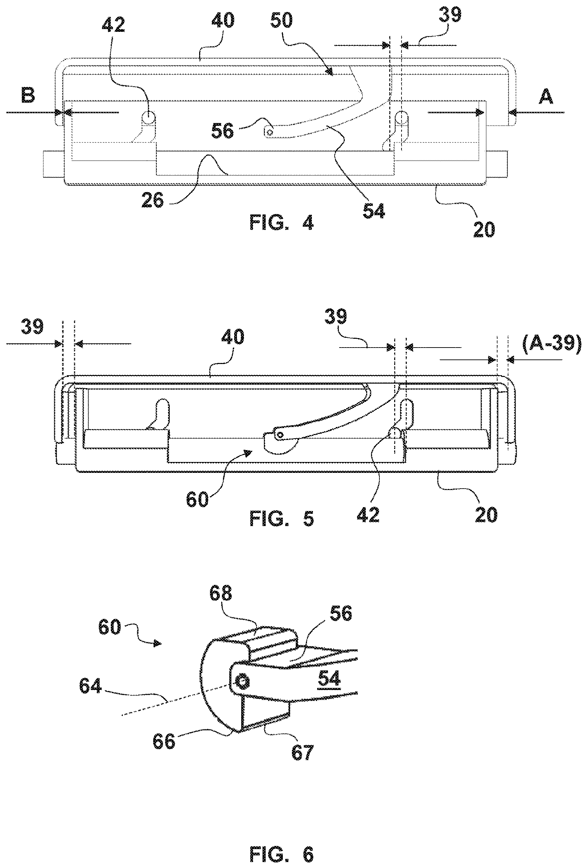

Movement of the compressor-actuator 40 with respect to the handle base 20 follows the slots 34 by providing the compressor-actuator 40 with bosses 42 protruding from the interior facing surfaces of the lateral walls 42 of the hollow compartment of the compressor-actuator 40; one circular boss 42 is associated with each of the slots 34. In this way, movement of the compressor-actuator 40 is guided by and restricted by the shape of the slots 34. In an unactuated state of the controller 10, shown in FIGS. 1, 2, and 4, the bosses 42 reside at the end of the vertical extent 35, which can be at the uppermost end of the slot 34. (It is noted that the embodiment shown in FIGS. 1 to 13 provide four slots 34 and four bosses 42. This number is merely one example. The cam surface of the slots 34, the extents 35, 36 of the slots 34, and the cam follower of the bosses 42 can take any form or shape that causes the controller to operate as described herein.) As seen most clearly in FIG. 4, a distance A between an interior of the proximal longitudinal wall 38 of the compressor-actuator 40 and an exterior of the proximal wall of the first handle part 20 is longer than the horizontal extent 39 (i.e., |A| > |39|). When the compressor-actuator 40 is fully actuated as shown in FIG. 5, the bosses 42 travel to the opposite (lowermost) end of the slot 34. The compressor-actuator 40, therefore, has traveled a vertical distance equal to the vertical movement of the bosses 42 within the vertical and angled extents 35, 36 and has traveled a horizontal distance equal to the horizontal extent 39. Some embodiments of the first and second handle parts 20, 40 have the interior surface of the distal longitudinal wall of the compressor-actuator 40 touching the exterior surface of the distal longitudinal wall of the handle base 20, this touch being indicated with arrows B in FIG. 4 (i.e., |B| = 0). When the compressor-actuator 40 is fully actuated, therefore, these two distal longitudinal walls separate to a distance equal to the horizontal extent 39. Likewise, the distance between an exterior surface of the proximal longitudinal wall of the handle base 20 and an interior surface of the proximal longitudinal wall of the compressor-actuator 40 shortens from A by a length equal to the horizontal extent 39 (i.e., (A-|39|)), which is illustrated in FIG. 5. Alternately, a four-bar linkage could be provided to join 20 and 40 to create the same motion as the cam slots and bosses.

What becomes apparent from movement of the compressor-actuator 40 following the slots 34 is how an extrusion compressor 50 connected to the compressor-actuator 40 operates during this movement. The extrusion compressor 50 in FIGS. 1, 2 and 4 to 13 can have the extrusion compressor 50 project from an interior surface of a ceiling of the hollow compartment of the compressor-actuator 40 downwards towards the handle base 20. In particular, the extrusion compressor 50 projects downwards towards the compression floor 26 of the handle base 20. The extrusion compressor 50 has a base 52 attached to the second handle part 40. A flex arm 54 projects from the base 52 and extends towards the compression floor 26. The flex arm 54 can be thinner than the base 52. A material from which the base 52 and flex arm 54 are made is not substantially rigid and, therefore, responsive to moving downwards to have a portion of the extrusion compressor 50 touch the compression floor 26 before the entire vertical movement of the compressor-actuator 40 is complete, the flex arm 54 flexes. Example materials for the base 52 and flex arm 54 include ABS, polycarbonate and Nylon^{®}, polypropylene, polyurethane, or other thermoplastic or thermoplastic elastomer and/or fiber filled ABS, polycarbonate and Nylon^{®}. At a distal end of the flex arm 54 is a gear flange 56 shaped to hold thereat a compression roller 60. The gear flange 56 has axle ports in which an axle 62 of the compression roller 60 resides. When installed between the interior sides of the gear flange 56, the compression roller 60 becomes fixed to the gear flange 56 in all directions except for rotational movement of the compression roller 60 about a rotation axis 64 of the roller 60; in other words, the roller 60 is allowed to rotate about the axis 64.

The illustrated extrusion compressor 50 is one example of a compressor. Different mechanical structures performing the same function can be used. For example, the base 52 and flex arm 54 can be replaced with a single beam that is hinged to the ceiling of the interior hollow of the compressor-actuator 40 and biased with a bias device (e.g., a spring) towards the compression floor 26 such that the point of the compression roller 60 touches the vacuum tube 2 as shown in FIG. 2 enough to grip the vacuum tube 2 but substantially not reduce the cross-sectional area of the vacuum channel 3.

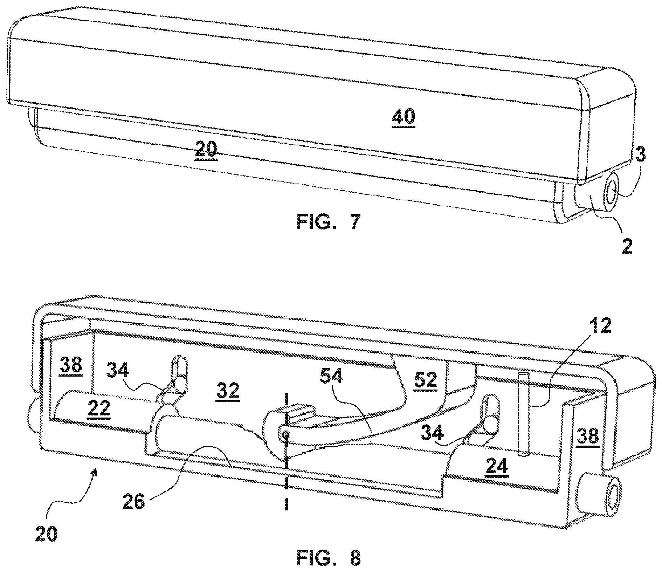

Rotation of the roller 60 is dependent upon how the roller 60 moves towards the vacuum tube 2 and along the vacuum tube 2. In this regard, the compression roller 60 has an exterior contact surface 66 that contacts the vacuum tube 2 in various ways when the compressor-actuator 40 is moved towards the handle base 20. As shown in FIG. 6, a longitudinal cross-section of the exterior surface 66 is approximately in the shape of a nautilus (alternatively, the shape can be cylindrical). The exterior surface 66 has a contact point 67, which is in contact with the exterior surface of the vacuum tube 2 in the unactuated state of the compressor-actuator 40 as shown in FIG. 2 (the vacuum channel 3 is not occluded with a substantially patent and open cross-section). As the compressor-actuator 40 is actuated, the compressor-actuator 40 travels along the vertical extent 35. This moves the contact point 67 towards the compression floor 26. When the compressor-actuator 40 has travelled along the entirety of the vertical extent 35, as shown in FIGS. 7 and 8, the contact point 67 has moved against the vacuum tube 2 to occlude the vacuum channel 3 completely. At the stage where the bosses 42 are at this transition point from the vertical extent 35 to the angled extent 36, the contact point 67 is as far towards the compression floor 26 as it can move in that direction - because the thickness of the vacuum tube 2 prevents further movement of the contact point 67 towards the compression floor 26.

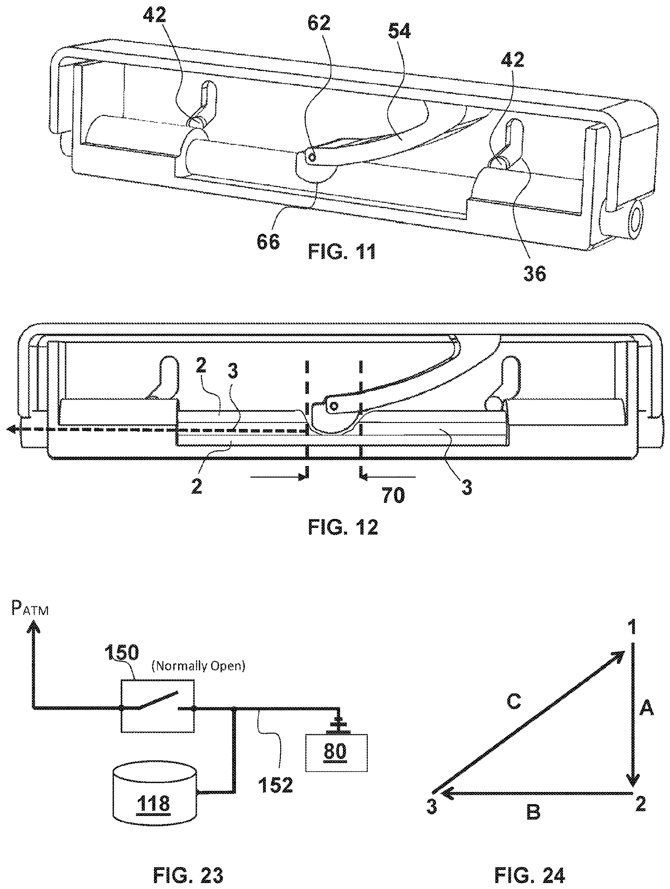

In a procedure where the vacuum tube 2 is used in a thrombectomy, the vacuum channel 3 will be filled with a fluid, i.e., blood. When the vacuum channel 3 is completely occluded, the blood that fills up the vacuum channel 3 from the contact point 67 of the compression roller 60 distally to the distal end of the vacuum channel 3 defines a column of fluid, which fluid is not compressible. The controller 10 is configured to apply the extrusion compressor 50 and the compression roller 60 to move this column of fluid a shift distance 70 in the distal direction. The volume of the shift distance can be approximately 0.001 ml to approximately 1.0 ml, in particular, approximately 0.1 ml to approximately 0.5 ml. The length of the shift distance 70 can be approximately 0.5 mm to approximately 30 mm, in particular, approximately 0.5 mm to approximately 15 mm. To effect such a movement, the compressor-actuator 40 is moved further in the direction towards the handle base 20, which means that that the bosses 42 travel along and through to the end of the angled extent 36. Because the contact point 67 is already as far towards the compression floor 26 as it can move in that direction (i.e., when the bosses 42 are at the transition point from the vertical extent 35 to the angled extent 36), the extrusion compressor 50 has no other way to move than to flex the flex arm 54 and/or to roll the compression roller 60. The contact surface 66 of the compression roller 60 is shaped to roll (counterclockwise in the views of FIGS. 2 and 8 to 12) against an upper surface of the vacuum tube 2. FIGS. 9 and 10 illustrate the rolling start of the compression roller 60 at a point where the bosses 42 are approximately halfway to the distal end of the slot 34 within the angled extent 36. (It is noted that limitation of the computer software that generates FIG. 9 to 12 do not allow for displaying a realistic view of how the vacuum tube 2 compresses as the compression roller 60 rotates. These figures, therefore, illustrate an approximation of the compression roller 60 rolling on and over the shift distance 70 of the vacuum tube 2.) The contact point 67 of the compression roller 60 is offset from the rotation axis 64 towards the contact surface 66. This forms an over center, or toggle, such that the initial rolling motion of the compression roller must first force the contact point 67 over the center of the rotation axis 64. In such a configuration, not only does the compression roller 60 roll once the bosses 42 of the compression-actuator 40 start traveling in the angled extent 37, but there is also a tactile feedback transmitted to the compression-actuator 40 once the axle 62 moves slightly forward. This feedback, when felt by the user, indicates to the user that the contact surface 66 of the compression roller 60 has rolled onto a portion of the vacuum tube 2 and, as it moves along the vacuum tube 2, squeezes that portion to translate the fluid column in the distal direction of the vacuum tube 2. With complete movement of the compression-actuator 40 towards the handle base 20 as shown in FIGS. 11 and 12, the compression roller 60 has completed its defined rotation over the vacuum tube 2 and, in doing so, has squeezed a segment of the vacuum channel 3 from proximal to distal over the length to shift the fluid column distally to a length equal to the shift distance 70.

To return the controller 10 to the initial, unactuated state shown in FIGS. 1 and 2, for example, a bias device 12 is interposed between any surface of the interior hollow of the compression-actuator 40 and any surface of interior hollow of the handle base 20. In FIGS. 2 and 8, the bias device 12 can be disposed between the surface of the ceiling within the interior hollow of the compression-actuator 40 and an upper surface of the proximal tube anchor 24. This configuration for the bias device 12 is merely one example and any return spring or similar mechanical device can be placed and used. When the user releases pressure on the compression-actuator 40, the flex arm 54 and/or the bias device 12 causes the compression-actuator 40 to return to the initial, unactuated state. This action rolls the compression roller 60 in the opposite direction (i.e., the progression from FIG. 11 to FIG. 9 to FIG. 8). As the distal end of the vacuum channel 3 experiences positive pressure from the patient and also from the increase in volume as the crushed tube rebounds, the fluid column retreats proximally back into the vacuum channel 3 and, when the compression roller 60 releases from the vacuum tube 2 to cease occluding the vacuum channel 3, vacuum being placed in the vacuum channel 3 from a vacuum pump 80 proximal to the controller 10 automatically reestablishes and draws the fluid column through the segment of the vacuum tube 2 within the controller 10.

As set forth herein, the vacuum tube 2 is sized to lay against the compression floor 26 on one side and to have the point of the compression roller 60 touch the outer surface of the vacuum tube 2 just slightly enough to grip the vacuum tube 2 but substantially not reduce the cross-sectional area of vacuum channel 3. In an embodiment where the vacuum tube 2 is not fixed within the handle base 20, the compression roller 60 is provided with a non-illustrated bias device that biases the compression roller 60 rotationally into a position shown in FIG. 2. This bias compensates in a situation where the vacuum tube 2 is not touching the compression roller in the unactuated position of the compressor-actuator 40.

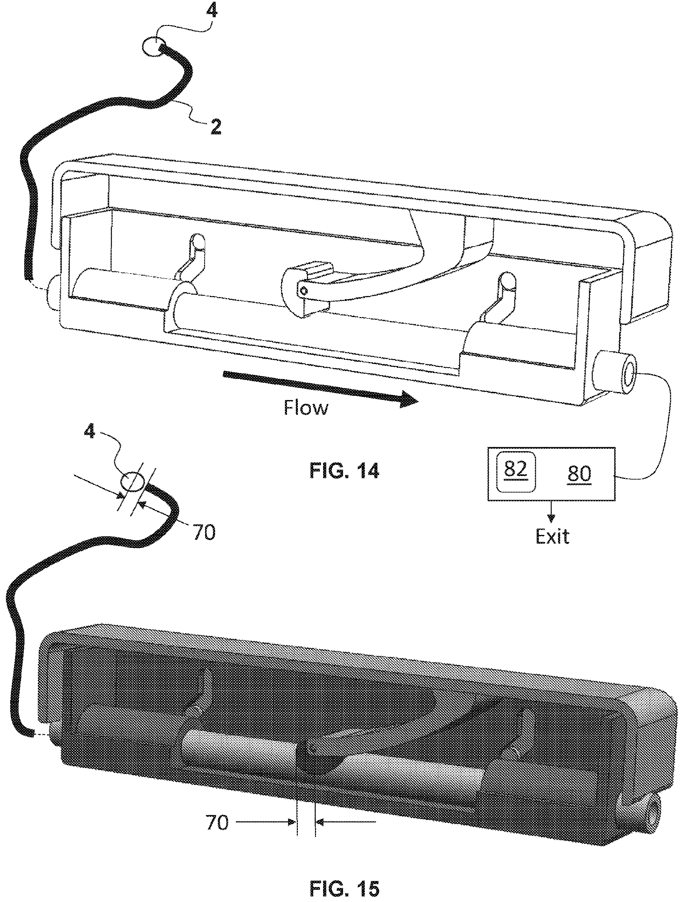

With a configuration as described, the controller 10 is to be used with a vacuum tube 2 that is or is part of a thrombectomy aspiration catheter. Such use is described with regard to FIGS. 14 and 15, in which the vacuum lumen 3 is shown as being an aspiration controller that, distal to the controller 10, is threaded through vasculature and up to a thrombus 4, which in the form of a blood clot, that has corked within or at the distal opening of the vacuum channel 3. On the proximal side of the controller 10, the vacuum channel 3 is fluidically connected to the vacuum pump 80. As indicated above, thrombi typically are trapped at the end of an aspiration catheter and removing the entire catheter from the patient when that occurs is not desirable. The inventors have discovered that removal of the catheter can be prevented using the controller 10. More particular, when the distal end of the vacuum tube 2 is clogged by a thrombus, the controller 10 is actuated to occlude all flow through the vacuum channel 3. This occurs by the first movement of the compressor-actuator 40 towards the handle base 20. The controller 10 is actuated to cause the fluid column to shift distally to the shift distance 70. This imparts a controlled reversal of flow to the fluid column within the vacuum channel 3 that slightly translates the thrombus to a prescribed shift distance 70 distally relative to the distal opening of the vacuum channel 3. During a third and final phase, the user releases actuation of the controller 10 to reset the fluid column within the vacuum channel 3 and, once again, allows the fluid to flow freely. The inventors have discovered that such movement causes either a repositioning of the thrombus or a deformation of the thrombus or both and that this movement allows the thrombus to pass entirely into and through the vacuum channel 3 where such passage was not possible before.

Operation of the controller 10 is explained with regard to the system cycle diagram of FIG. 24.
- **State 1:** Normal aspiration is occurring. The vacuum channel 3 is not occluded. The controller 100 is in a rest state where the vacuum pump 80 is connected to the vacuum channel 3.
- **Transition A** - **Occlusion:** Thrombus 4 occludes distal end of vacuum channel 3. Unclogging controller 10 actuates to occlude vacuum channel 3 and stop vacuum flow distal of the controller 10.
- **State 2:** Flow through the vacuum channel 3 has stopped.
- **Transition B** - **Unclogging:** Controller 10 continues actuation to cause reverse flow in vacuum channel 3 for a metered volumetric column shift.
- **State 3:** Flow reversal stops.
- **Transition C** - **Return Column Shift:** Controller 10 is reversed to return column and accelerate thrombus 4 into catheter tip by reconnecting the vacuum pump 80 to the vacuum channel 3.
- **Return to State 1 and Repeat:** Normal aspiration occurs.

The inventors further discovered that greater accelerations of the thrombus into the catheter provide proportionally quicker aspirations. A magnitude of the thrombus' impact velocity, and therefore its kinetic energy, when it impacts the aspiration catheter's distal tip, affects the amount of the thrombus that is deformed to fit within the diameter of the vacuum channel 3. When a catheter is extended to a thrombus that is lodged in a vessel, e.g., a vessel within the brain, the controller 10 is not needed until the thrombus 4 is stuck at the distal opening of the vacuum channel 3. Thus, the thrombus does not have any distance to move in order to accelerate towards the opening of the vacuum channel 3. Imparting the shift distance to the thrombus as described maximizes the kinetic energy of the thrombus at the point when it impacts the catheter's tip. The thrombus' acceleration (and therefore its kinetic energy) are generated by a pressure differential between intracranial pressure and the effective aspiration pressure at the catheter's tip. For the thrombus to accelerate, both it and the fluid column within the catheter system must attain a velocity. After catheters are occluded, the fluid velocity within the catheter is substantially zero. In conventional catheter architecture, the pressure that attempts to accelerate this fluid column is provided solely by an external vacuum pump. Significantly, however, this pressure is reduced by head losses in the tubing connecting the vacuum pump to the catheter's proximal end. Accordingly, conventional catheters must be fished out of the vasculature entirely because the thrombus is corked within the distal opening of the vacuum channel.

This disadvantage is removed by the controller 10. After the distal opening of the vacuum channel 3 is occluded by the thrombus, the fluid velocity within the catheter is substantially zero. The controller 10 is used to unclog the vacuum channel 3 and displace the thrombus 4 distally out from the distal opening. Then, the controller 10 reapplies vacuum. Upon re-application of vacuum, the fluid column accelerates and the thrombus 4 accelerates back into the vacuum channel 3. With such acceleration, the thrombus is deformed to a diameter allowing it to be aspirated. With one or just a few applications to displace the thrombus by the shift distance 70 with the controller 10, the vacuum channel 3 becomes unclogged and the thrombus 4 accelerates sufficiently to be completely aspirated through and out of the vacuum tube 2. With the controller 10, the head losses in the tubing are minimized, thereby allowing the thrombus to accelerate to a much greater extent than in conventional product architectures.

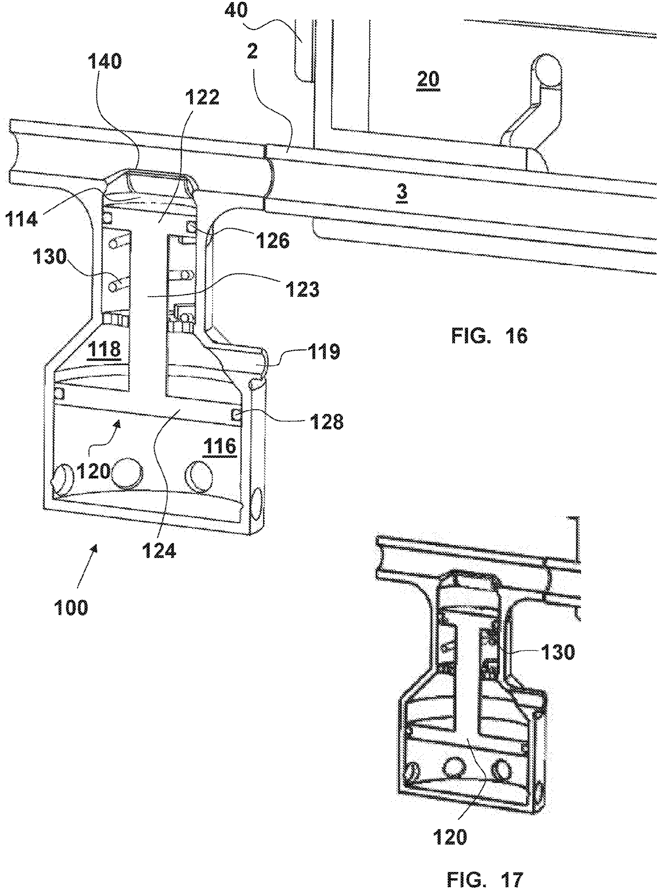

Realizing that acceleration of the thrombus proximally is a desirable trait, it becomes possible to enhance acceleration in the proximal direction when deactuation of the controller 10 occurs to re-establish vacuum. To maximize the acceleration of the thrombus and the fluid column within the vacuum channel 3 for the purpose of maximizing the thrombus' kinetic energy upon its impact with the distal tip of the vacuum tube 2, a vacuum booster 100, illustrated in FIG. 16, is fluidically connected to the vacuum channel 3 of the vacuum tube 2. In general, the vacuum booster 100 applies suction to the fluid column in a region of the aspiration catheter's proximal end to maximize acceleration of the catheter's fluid column at a user-selected time. The vacuum booster 100 can comprise a booster body 110 defining a plunger bore 112, a plunger 120 housed within the bore 112, and a bias device 130. The plunger bore 112 is shaped to define a vacuum chamber 114 and an ambient chamber 116. The vacuum chamber 114 can be cylindrical and have a first inner diameter and the ambient chamber 116 is cylindrical and has a second inner diameter larger than the first inner diameter. The vacuum chamber 114 has a volume that is smaller than a volume of the ambient chamber 116.

The plunger 120 has a vacuum piston 122 and an ambient piston 124, which is connected to the vacuum piston 122 through a rod 123. The vacuum piston 122 can have a diameter substantially equal to the first inner diameter of the vacuum chamber 114 and is able to move within the vacuum chamber 114. The ambient piston 124 has a diameter substantially equal to the second inner diameter of the ambient chamber 116 and can move within the ambient chamber 116. Between the vacuum piston 122 and the ambient piston 124 is a pressure chamber 118 in which is located the rod 123 connecting the two pistons 122, 124 together, for example, in the shape of an asymmetric dumbbell. To seal the pressure chamber 118 off from both the vacuum chamber 114 and the ambient chamber 116, a vacuum seal 126 is disposed between the vacuum piston 112 and the wall of the vacuum chamber 114 and an ambient seal 128 is disposed between the ambient piston 124 and the wall of the ambient chamber 116. The booster body 110 defines the pressure chamber 118 and a pressure port 119 that fluidically connects the pressure chamber 118 to a boost control valve or switch 150. This connection is illustrated diagrammatically in FIG. 23.

The vacuum chamber 114 operatively communicates with the vacuum channel 3 at a connection 140. The plunger 120 and the bias device 130 are disposed such that, when the bias device 130 is in a relaxed state, the vacuum piston 122 is at a given distance from the connection 140 to the vacuum channel 3; this relaxed state is illustrated in FIG. 17. In the relaxed state, the spring is at a steady state - there is no potential energy stored in the spring. With regard to pressure, in the relaxed state, both the pressure chamber 118 and the ambient chamber 116 are at ambient pressure, i.e., they are substantially equal. When the plunger 120 is moved towards the vacuum channel 3 into an energized state (which is shown in FIG. 16), the bias device 130 (e.g., in the form of a spring that is stretched) thereby stores strain energy that is directed to move the plunger 120 away from the connection 140. Such movement, when it occurs, creates suction within the vacuum chamber 114 and the vacuum channel 3 that communicates with the vacuum chamber 114.

To actuate the embodiment of the pneumatically actuated vacuum booster 100, the pressure chamber 118 is connected to the vacuum pump 80 (the vacuum source) through a relatively high impedance conduit 152. The pressure chamber 118 is also connected to the boost control valve 150, which is connected to ambient pressure but is normally open to prevent flow from the pressure chamber 118 to the environment (Patm). When the vacuum booster 100 is in a cocked state (FIG. 16), the boost control valve 150 is open (as shown) and, as such, the vacuum pump 80 is able to significantly lower pressure within the pressure chamber 118. When the boost control valve 150 is actuated (i.e., connecting the pressure chamber 118 to the ambient environment), pressure equalization occurs between the pressure chamber 118 and the ambient chamber 116. An impedance of a connection between the pressure chamber 118 and the boost control valve 150 is designed to be substantially less than the impedance between the pressure chamber 118 and the vacuum pump 80 such that, upon actuation of the boost control valve 150 (i.e., closure), rapid pressure equalization is possible.

Operation of the vacuum booster 100 is explained with regard to the system cycle diagram of FIG. 24.
- **State 1:** Normal aspiration is occurring. The vacuum channel 3 is not occluded. The controller 100 is in a rest state where the vacuum pump 80 is connected to the vacuum channel 3. The vacuum booster 100 is in the cocked state. The thrombus trap 200 is operating without bleed purge.
- **Transition A** - **Occlusion:** Thrombus 4 occludes distal end of vacuum channel 3. Unclogging controller 10 actuates to occlude vacuum channel 3 and stop vacuum flow distal of the controller 10.
- **State 2:** Flow through the vacuum channel 3 has stopped.
- **Transition B** - **Unclogging:** Controller 10 continues actuation to cause reverse flow in vacuum channel 3 for a metered volumetric column shift.
- **State 3:** Flow reversal stops.
- **Transition C** - **Vacuum Boost:** Vacuum booster 100 actuated to re-initiate flow in nominal direction and accelerate thrombus 4 into catheter tip. Shortly before, at the same time, or shortly thereafter, controller 10 opens vacuum channel 3 to reinitiate vacuum of pump 80 for fluid flow and aspiration of thrombus 4 into thrombus trap 200. Simultaneously or thereafter, controlled purging or automatic purging of thrombus trap 200 occurs allowing inspection of thrombus 4.
- **Return to State 1 and Repeat:** Vacuum booster 100 and self-purging trap 200 are de-actuated. Normal aspiration occurs.

During the occlusion and column shift phases in the operation of the controller 10, the plunger 120 is held in the energized state, with the plunger 120 raised to place the vacuum piston 122 closer to the connection 140. During or immediately upon the end of the reversal phase, the plunger 120 is released, generating suction within the locally communicating lumen of the vacuum channel 3 and thereby accelerating the fluid column proximally in the vacuum direction. What fluid is begin drawn into or towards the vacuum chamber has an effect on the efficiency of the vacuum booster 100. More specifically, if the fluid arrives only from downstream of the vacuum booster 100 when actuated, then the fluid column will not accelerate proximally as desired. When the controller 10 occludes the vacuum channel 3, fluid into and towards the vacuum chamber 114 will arrive substantially from upstream of the vacuum channel 3, thereby accelerating the fluid column in the desired direction. In an intermediate stage where fluid arrives from both upstream and downstream, the downstream portion can be limited, for example, by placing a non-illustrated check valve between the thrombus trap 200 and the connection 140, in particular, between the connection 140 and the controller 10. The check valve can be external or can use the occlusive function of unclogging handle.

The following description summarizes the forces in a pneumatic embodiment of the vacuum booster 100. In an un-cocked state of the plunger 120, the pressure chamber 118 and the ambient chamber 116 are at ambient pressure and the bias device 130 is in substantially in the relaxed state, storing little or no strain energy. In a cocked state of the plunger 120, the pressure chamber 118 is caused by the boost control valve 118 to be at a significantly lower pressure than the ambient chamber 116. The geometries of the chambers 114, 116, 118 and the pistons 122, 124, and the characteristics of the bias device 130 are selected such that, in this configuration, a force created by the pressure difference across the ambient (larger) piston is significantly greater than the force required to expand the spring. As such, when the given pressures are held, the piston and spring system translates upwards into a "cocked" position. When the vacuum booster 100 is actuated, the pressure chamber 118 is allowed to rapidly equalize to ambient pressure. With no net force input from the ambient piston 124 (the larger of the two pistons), any motion of the piston and spring system are now caused by the actions of the bias device 130 and the pressure differential across the smaller, vacuum piston 122. The geometries of the chambers 114, 116, 118 and the pistons 122, 124, and the characteristics of the bias device 130 are selected such that the bias device's restoring force in the cocked configuration is much higher than an opposing force caused by the pressure difference across the smaller vacuum piston 122, which is disposed between ambient pressure and a pressure within the vacuum channel 3. As such, when the vacuum booster 100 is actuated and the pressure chamber 118 is allowed to equalize to ambient pressure, the piston and spring system energetically drives "downwards", generating a negative displacement and a dramatic pressure decrease within the vacuum chamber 114 and thereby the vacuum channel 3 of the aspiration device.

As indicated herein, current thrombus removal devices are not able to inform the surgeon that the thrombus has been removed without full withdrawal of the device from a patient's anatomy. Surgeons do not have an ability to view the reservoirs into which aspirated contents are deposited, not only because the reservoirs are located outside of the sterile field in an operating room setting, but also because the removed thrombus is present within a significant quantity of blood contained in the reservoir.

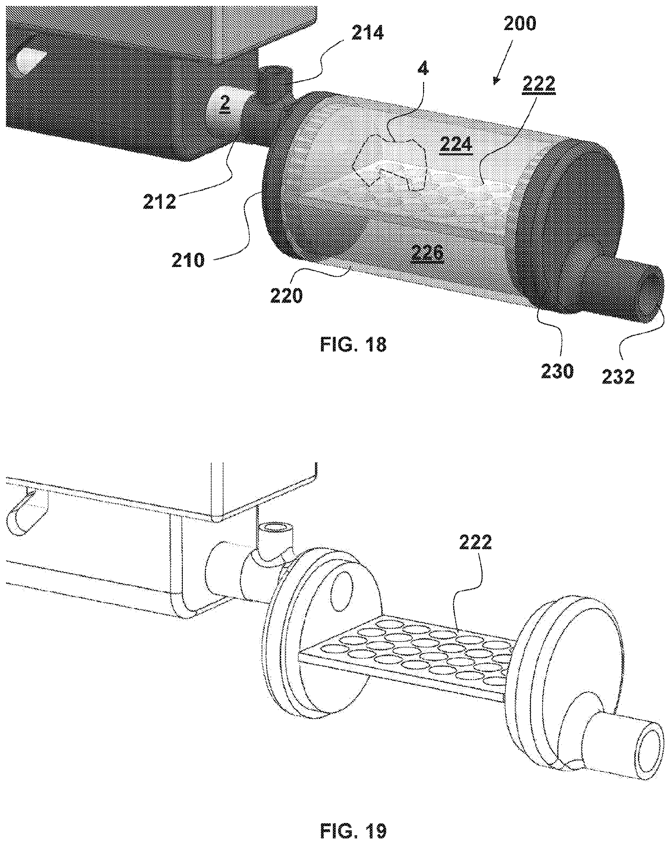

To overcome an inability to visualize the thrombus actually retrieved, a visualization-aiding thrombus trap 200 is provided and shown in FIGS. 18 to 21. The thrombus trap 200 is placed in-line with the aspiration system, in particular, the vacuum channel 3. In some embodiments, the thrombus trap 200 is within the catheter operator's immediate vicinity between the aspiration catheter and the vacuum source, in particular, between the controller 10 and the vacuum pump 80, so that the surgeon can see the thrombus trap 200 during use of the controller 10. In use, all aspirated material flows through the thrombus trap 200.

The thrombus trap 200 comprises a container having an inflow section 210 having an input orifice 212 fluidically connected to the vacuum channel 3, a transparent intermediate trap section 220 in which the thrombus is trapped, and an outflow section 230 fluidically connected to the vacuum pump 80. In operation, aspirated material and fluid travel from the vacuum channel 3 past the controller 10 through the inflow section 210 and into the trap section 220. The trap section 220 contains a trap filter 222 that is, in some embodiments, a screen or a filter through which all aspirated flow must pass. The filter 222 is configured to stop and capture thrombus material therein but allow the passage of air and fluid with minimal impedance therethrough and, thereby out of the outflow section 230 to the vacuum pump 80 and any associated vacuum pump reservoir 82. In FIGS. 18 to 22, the filter 222 can be a grating or screen having orifices sufficiently large enough for fluid and air to pass therethrough but sufficiently small enough to substantially prevent the thrombus from passing across the filter 222 from an inflow or trap chamber 224 of the trap section 220 to an outflow chamber 226 of the trap section 220. As used herein, the term "filter" includes any structure that is able to separate fluid from particulate matter by allowing the fluid to pass through the structure while preventing the particular matter from passing through. Other embodiments of the filter 222 include perforated polymer, textile, or sintered semi-permeable polymer. The outflow section 230 has an output orifice 232 that fluidically connects the outflow chamber 226 to the vacuum pump 80 for directly receiving the vacuum generated.

The container of the thrombus trap 200 is sealed when closed and in use during a surgical procedure. In some embodiments, the thrombus trap 200 can be taken apart and opened for removal of the thrombus out of the trap chamber 224 and inspection by the surgeon or pathologist, as well as for sterilization when the thrombus trap 200 is reusable.

It is noted that when a thrombus 4 is captured in the trap chamber 224, whether or not vacuum is still being applied, the trap chamber 224 is also filled with blood. Thus, the thrombus 4 cannot be visualized even if the entirety of the thrombus trap 200 is transparent for viewing inside by a user. To assist with visualization of the thrombus 4 contained within the trap chamber 224, the thrombus trap 200 is configured to temporarily purge itself of fluids that visually impede inspection of captured thrombus material. In some embodiments, therefore, the inflow section 212 is formed with an intake bleed valve 214 fluidically connected to the vacuum channel 3 and to the trap chamber 224. The bleed valve 214 is configured to operate in a closed mode, in which any flow of air and/or fluid through the bleed valve 214 and into the trap chamber 224 (or vacuum channel 3) is fully restricted, and a bleed mode, in which the bleed valve 214 intakes a fluid, in particular, ambient air. (Alternatively, if desired, in the bleed mode, the bleed valve 214 can intake a clear liquid such as saline.) During the closed mode operation, the exit of the bleed valve 214 is closed and aspirated materials are unhindered to flow through the thrombus trap 200 from the input orifice 212 and out the output orifice 232 away towards the vacuum source, leaving aspirated thrombus and other solid matter in the trap chamber 224. Accordingly, when the surgeon has captured a thrombus 4 in the trap chamber 224 during a thrombectomy procedure, the surgeon can immediately visualize that thrombus 4 by setting the bleed valve 214 into the bleed mode, which, due to a relatively larger size of the bleed valve's 214 input opening and to a decreased resistance to the vacuum by opening to ambient air, causes the vacuum pump to draw ambient air rapidly into the trap chamber 224 and thereby evacuate all fluid from the trap chamber 224. During inspection, the bleed valve 214 can be configured to occlude the fluidic connection between the trap chamber 224 and the vacuum channel 3. Actuation of the bleed valve 214 can be separate from the controller 10 or mechanically connected to the controller 10 so that, when the controller 10 is in an unactuated state where aspiration is occurring, a bleed switch on the controller can activate the bleed valve 214. The rapid inflow of air into the trap chamber 224 is directed by the descending pressure gradient between the outside environment and the relatively low pressure existing within the volume existing between the trap chamber 224 and the vacuum pump 80. As such, while the bleed 214 valve is open, airflow displaces fluids from the volume of the thrombus trap 200, leaving the volume mostly full of transparent air, instead of opaque blood. This temporary transparency allows for easier inspection of the material caught by the filter 222. The surgeon then can view the thrombus 4 unobstructed within the trap chamber 224. During this examination, the control of the bleed valve 214 (which can be a mechanical or a processor-based controller) can cause the vacuum pump 80 to reduce vacuum or to shut off completely, at least until the surgeon is ready to continue the thrombectomy procedure if continuation is desired. When the bleed valve 214 is set back to the closed mode and re-connection of the trap chamber 224 to the vacuum channel 3 occurs, normal aspiration resumes. Alternatively, the bleed valve can be connected to a fluid flush line such as a saline drip bag.

Inspection of the thrombus 4 may be enhanced by providing the thrombus trap 200 with optical filters optimized for visual contrast, transparent trap enclosures as described, built-in magnification or visualization systems, lighting, and/or sensor-based thrombus-detection methods.

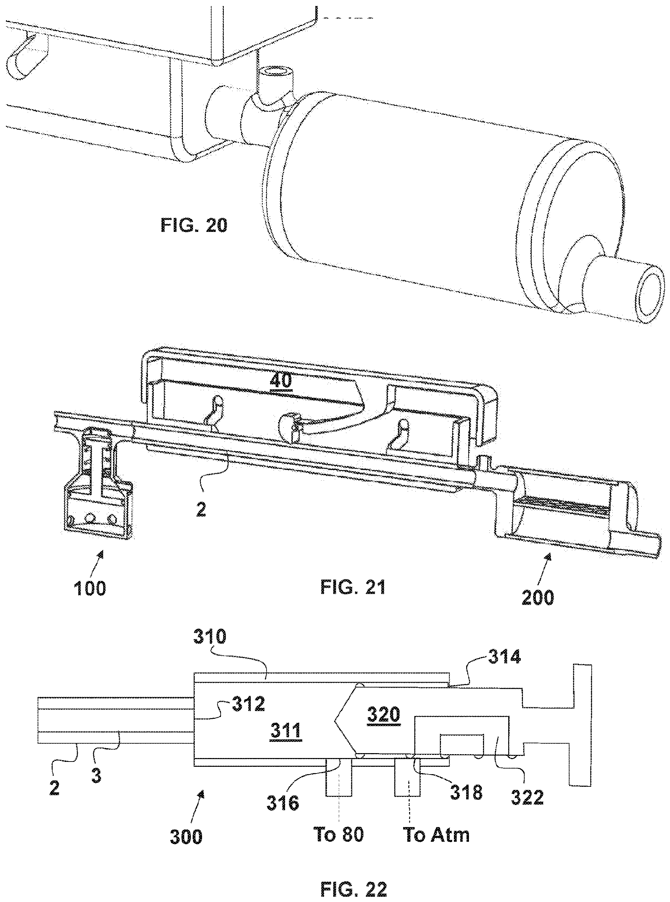

The vacuum booster 100 can be disposed upstream of the thrombus trap 200 and is on a side of controller 10 opposite the thrombus trap 200 as shown in FIG. 21. Accordingly, to maintain efficacy of the thrombus trap 200 as a terminus for all aspirated thrombi 4, vacuum booster configurations that might entrap or significantly damage or macerate the thrombus are less desirable. One embodiment of a gentler vacuum booster 200, instead of the piston design of FIGS. 16 and 17, couples a section of the tubing of the vacuum tube 2 having a deformable interior volume with a mechanical actuation mechanism. This mechanism is able to collapse and expand the interior cross-section of a length of the vacuum channel 3 to provide an increase or a decrease in pressure along that length. Another mechanical embodiment for the vacuum booster having no pneumatic actuation takes energy for vacuum boost from energy imparted by actuation of the controller 10 or from a separate energy input. For example, as user depresses a lever in the controller 10 that occludes flow and temporarily causes the column shift, the lever's motion also cocks and releases a spring-loaded piston that creates the vacuum boost. Another embodiment of the vacuum booster places a screen between the vacuum chamber 114 of the vacuum booster 100 and the vacuum channel 3 of the aspiration system. This screen allows fluid communication between the two interior volumes but occludes particulate matter from entering the piston bore defined by the vacuum chamber 114. A further embodiment that guards against clogging/accidental maceration of the thrombus alters the piston configuration of FIGS. 16 and 17 by having the connection 140 be a flexible diaphragm mechanically disposed between the surface of the vacuum piston 122 and the opening into the vacuum channel 3. The diaphragm can be contained in and cross the actual opening of the vacuum channel 3, for example. Such a membrane transmits volumetric displacement while excluding all flow. The membrane can be separate from the vacuum piston 122, fluidically coupled thereto, or attached. In each of these configurations, the volume through which the fluid column flows is unhindered to prevent entrapping or damaging the thrombus 4 when traveling thereby, whether the vacuum booster 100 is in an energized state or a resting state.

Both the vacuum booster and the blood-purging clot trap rely on the timely and controlled application of either vacuum or ambient pressures to specific parts of the device, namely the bleed valve 214 of the thrombus trap 200 or the plunger 120 of the vacuum booster 100. The self-unclogging thrombectomy aspiration catheter described and shown herein can be provided with additional features actuated by the same user input as the self-unclogging function, e.g., at or by the controller 10, but which serve to either open or occlude additional conduits for vacuum or atmospheric pressure air that control device features such as the self-purging thrombus trap 200 and/or the vacuum booster 100.

The vacuum channel 3 of the vacuum tube 2 (and any other tubing within the catheter) can be coated with a hydrophobic coating, such as carnauba wax, for example, to decrease head loss during aspiration.

With an appropriate pressure sensor (for example, a piezoelectric diaphragm transducer, an electromagnetic diaphragm transducer, a strain-gage diaphragm transducer, or a MEMS pressure integrated circuit transducer), the controller 10 can determine when the vacuum channel 3 is clogged by a thrombus and automatically perform the unclogging procedures described herein. In some embodiments, a computer connected to the sensor can detect a pressure drop and lack of flow associated with a thrombus clog in or at the vacuum channel 3. When the clog is detected, the sensor triggers the sequence that halts application of vacuum in the vacuum channel 3 and carries out the column shift sequence. With respect to visualization of the thrombus 4 in the device, another embodiment of a sensor includes an optical sensor that detects the presence of the thrombus in either or both of the distal opening of the vacuum channel 3 and the thrombus trap 200. In the latter configuration, the optical sensor associated with the trap section 220 detects when the thrombus 4 is present and cause purging of fluid by opening the bleed valve 214.

As set forth herein, the vacuum tube 2 can be made from various materials. Some materials for the vacuum tube 2 have a relatively lower compression strength, such as latex, silicone, and other synthetic rubbers. Other materials for the vacuum tube 2 have a relatively higher compression strength, such as Pebax^{®}, polyurethane, and polyvinyl chloride. Because the vacuum tube 2 within the controller 10 is subject to expansion when positively pressured in the vacuum channel 3 and is subject to contraction when negative pressured, this flexible attribute of the material from which the vacuum tube 2 is made could possibly contribute to a less effective column shift. In order to reduce these effects of pressure (both positive and negative) on the vacuum tube 2, the vacuum tube 2 can be reinforced with a braid or coil or other mechanical structure to support the portion of the vacuum tube 2 within the controller 10 against pressure changes. Where the vacuum tube 2 is made from a material with a relatively lower compression strength, the section of the vacuum tube 2 that resides within the controller 10 is made as short as possible to minimize the expansion/contraction effects.

An alternative embodiment to the controller 10 of FIG. 1, which indirectly operates on the vacuum channel 3 through the compression roller 60, is shown in FIG. 22. In the embodiment of FIG. 22, the extrusion compressor is replaced with a volume changing controller 300 that is directly fluidically connected to the vacuum channel 3 of the vacuum tube 3. The volume changing controller 300 has a barrel body 310 with an interior 311 defining an input orifice 312 fluidically connected to the vacuum channel 3. The barrel body 310 also defines a plunger orifice 314, a pump orifice 316, and a purge orifice 318. A plunger 320 sealably connects to the interior 311 of the barrel body 310 movably towards and away from the input orifice 314. When in the position shown in FIG. 22, vacuum applied by the vacuum pump 80 is connected to the distal opening of the vacuum channel 3 for aspiration of material. When a thrombus becomes clogged at the distal opening, the surgeon presses the plunger 320 inwards. In a first portion of the inwards motion, a surface of the plunger 320 seals off the pump orifice 316 to stop the application of vacuum to the vacuum channel 3. In a second portion of the inwards motion, the plunger 320 moves all fluid contained within the interior 311 and the vacuum channel 3 distally to cause the column shift. Reversal of the plunger reverses the column shift and reapplies vacuum to the vacuum channel 3.

The plunger 320 can also be used to control purging of the thrombus trap 200. The plunger is provided with a purge conduit 322. When the plunger 320 is placed in a purge position, the plunger 320 closes off the vacuum channel 3 from the vacuum pump 80 and fluidically connects the pump orifice 316 to the purge orifice 318 through the purge conduit 322. In this position, a fluid connected to the purge orifice, e.g., ambient air, is drawn through the purge conduit 322, through the purge orifice 318, and into the thrombus trap 200.

Operation of the volume changing controller 300 is explained with regard to the system cycle diagram of FIG. 24.
- **State 1:** Normal aspiration is occurring. The vacuum channel 3 is not occluded. The volume changing controller 300 is in a rest state where the vacuum pump 80 is connected to the vacuum channel 3.
- **Transition A** - **Occlusion:** Thrombus 4 occludes distal end of vacuum channel 3. Controller 300 actuates (plunges) to occlude vacuum channel 3 and stop vacuum flow distal of the controller 300.
- **State** 2: Flow through the vacuum channel 3 has stopped.
- **Transition B** - **Unclogging:** Controller 300 continues to plunge to cause reverse flow in vacuum channel 3 for a metered volumetric column shift.
- **State 3:** Flow reversal stops.
- **Transition C** - **Return Column Shift:** Controller 300 is reversed to return column and accelerate thrombus 4 into catheter tip by reconnecting the vacuum pump 80 to the vacuum channel 3.
- **Return to State 1 and Repeat:** Normal aspiration occurs.

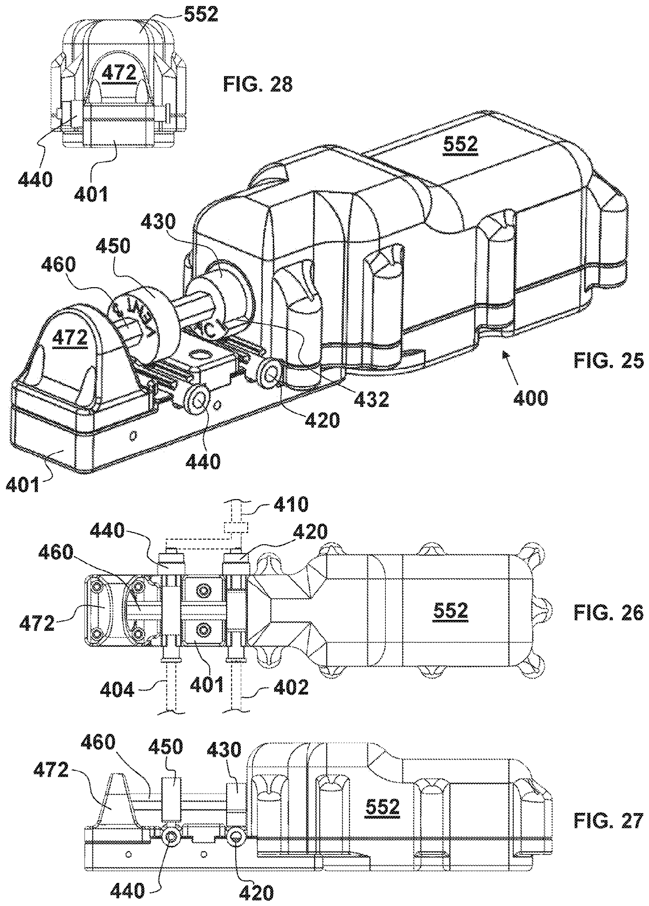

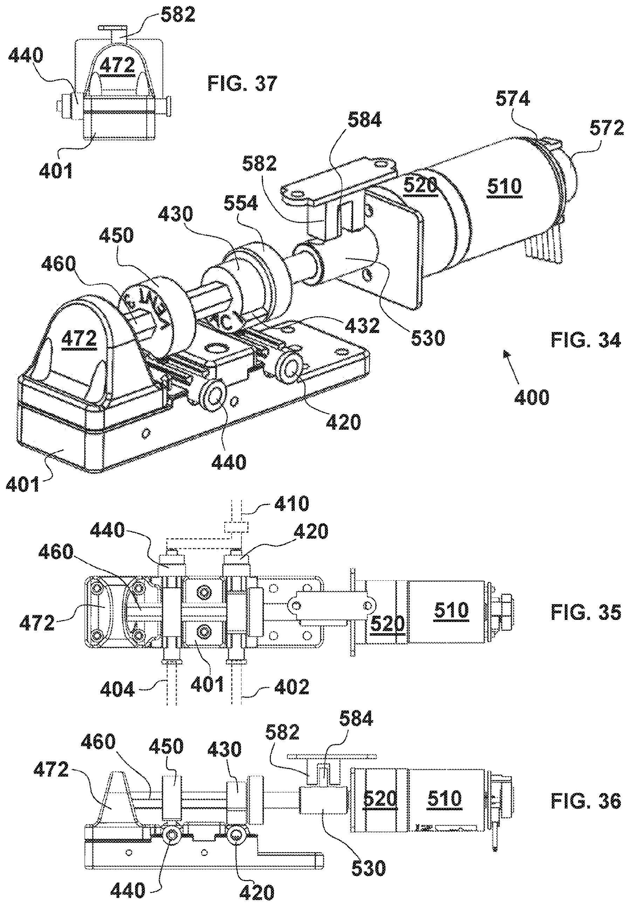

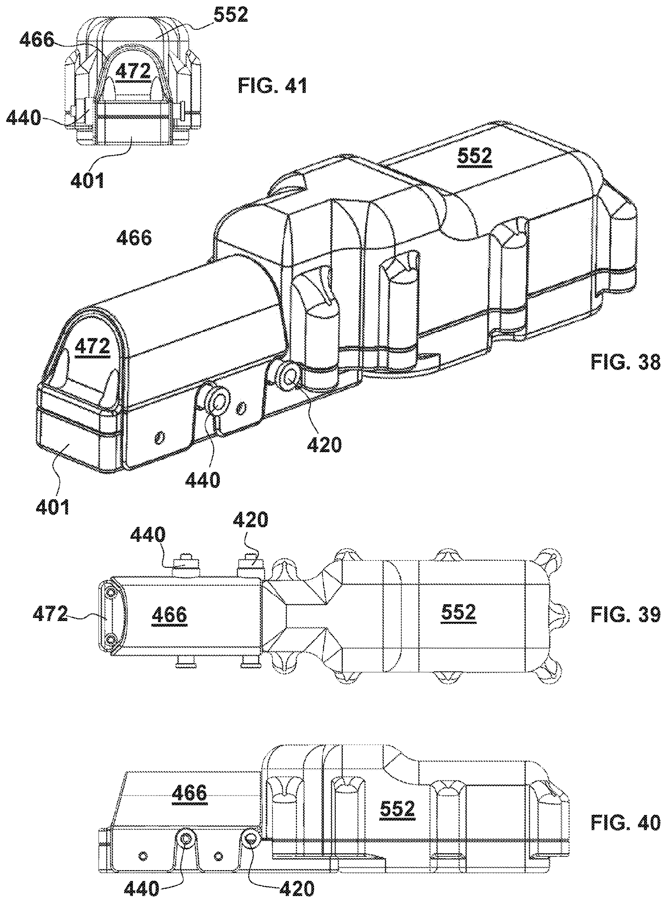

FIGS. 25 to 41 illustrate some embodiments of an aspiration thrombectomy system 400 operating with an automatic, rapid, and repeated onset of pressure change. An aspiration catheter 410 is diagrammatically indicated in FIG. 26 leading from distal orifices of a pair of valves 420, 440, which in this embodiment are pinch valves 420, 440. The pinch valve 420 can control vacuum flow and is connected between the aspiration catheter 410 and the aspiration pump (e.g., vacuum pump 80), and the pinch valve 440 can control vent flow and is connected to a supply of vent liquid. In some embodiments, the vent liquid can be any of albumin, d5W water, normal saline, half-normal saline, and lactated Ringer's solution, to name a few. The vent liquid can also be any other biocompatible fluid such as contrast media or tissue plasminogen activator (tPa). With such fluids, the catheter 410 can perform different functions. For example, switching the vent liquid to a contrast media after aspirating a clot allows the surgeon to confirm whether the clot was fully removed without having to first withdraw the catheter from the patient. This is significant because when a clot becomes lodged in the distal end of a standard aspiration catheter, the entire catheter needs to be removed from the patient and reintroduced through the vasculature to inject contrast medias for visualization. The vent liquid can be at atmospheric pressure or at a higher or lower than atmospheric pressure.

In some configurations, the valves 420, 440 are mounted to a base 401A vacuum cam 430 is associated with valve 420 and a vent cam 450 is associated with valve 440. The vacuum and vent cams 430, 450 can be connected to a common cam shaft 460. A first shaft end 462 of the cam shaft 460 is fixedly connected to a shaft bearing 470 in a freely rotatable manner. The shaft bearing 470 has a bearing body 472 mounted to the base 401. A second shaft end 464 of the cam shaft 460 is connected to a shaft drive assembly 500. The shaft drive assembly 500 comprises a motor 510, a transmission or gear box 520, a shaft coupler 530, and a motor controller assembly 550.

The transmission 520 has an output shaft 522. To connect the transmission 520 to the cam shaft 460, a first coupler end 532 of the shaft coupler 530 is connected to the output shaft 522 and a second coupler end 534 of the shaft coupler 530 is connected to the second shaft end 464. In this manner, rotation of the motor 510 corresponds to a rotation (at the same or different speed based upon the gearing of the transmission 520) of the cam shaft 460 with a corresponding rotation of the vacuum and vent cams 430, 450.

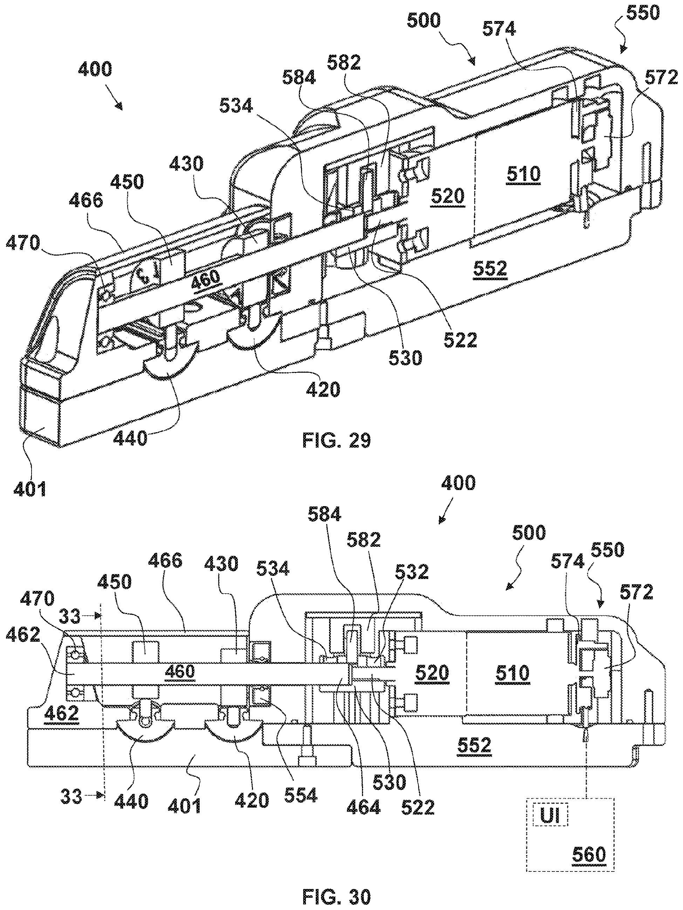

Control of the motor 510 originates from the motor controller assembly 550, which comprises a controller 560, a positional encoder 570 and a positional reset assembly 580. In some embodiments, the controller 560 is a microcontroller that has a user interface (UI) comprising user inputs that include, for example, control buttons to operate the aspiration thrombectomy system 400 in various states, examples of which are described in further detail below. The controller 560 with the UI is illustrated diagrammatically in FIG. 30. To isolate parts from fluid, the motor 510, the transmission 520, the shaft coupler 530, and the motor controller assembly 550, 560, 570, 580 can be contained in a motor assembly housing 552. The connection of the motor assembly housing 552 to the cam shaft 460 is sealed fluidically with a shaft seal 554. Similarly, the cams 430, 450, the cam shaft 460, and the shaft bearing 470 are covered with a cam housing 466. The controller 560 is indicated in FIG. 30 as separate from the motor assembly housing 552 (either wired or wireless) but it can also be integrated into or attached to the motor assembly housing 552. In a wireless configuration, the controller 560 can be an app on a computer or smartphone, for example, with all of the UI being available through a touchscreen.

The vacuum and vent cams 430, 450 are fixed rotationally to the cam shaft 460. These cams 430, 450 have various cam profiles to operate the valves 420, 440. It is desirable to know the exact rotational position of the cams 430, 450 and, therefore, cam shaft 460, so that the controller 560 can set the valves 420, 440 in whatever state that is desired. Because the motor 510 rotates freely and can end its rotation at any rotational position, it is desirable to know the exact rotational position of the cam shaft 560 at all given times. Accordingly, the motor controller assembly 550 includes the positional encoder 570 associated with the motor 510. With this association, the controller is provided with information on the exact rotational state of the cam shaft 460 and, therefore, the cams 430, 450. The positional encoder 570 comprises an encoder disk 572 and an encoder circuit 574. The encoder 570 can detect and report out to the controller 560 the current relative rotational position of the motor 510 at any point in time.

Those of skill in the art know that the motor 510 and/or the positional encoder 570 can drift in use. To account for and correct any drift, the motor controller assembly 550 comprises the positional reset assembly 580. This positional reset assembly 580 assigns a single rotational position of the cam shaft 460 as a reset point and every time that position crosses a zero-line the positional encoder resets the position of the motor 510 to zero, which in turn allows the system to know the absolute position of the cam shaft 460. In some embodiments, the positional reset assembly 580 comprises a photodiode 582 and a flag or interrupter 584. As shown in FIG. 30, the flag 584 is fixed to the shaft coupler 530. The photodiode 582 is placed at the path of the flag 584 so that the flag 584 interrupts the photodiode 582 once for each rotation of the cam shaft 460. This embodiment allows for immediate correction of any skipped steps of the encoder 570.

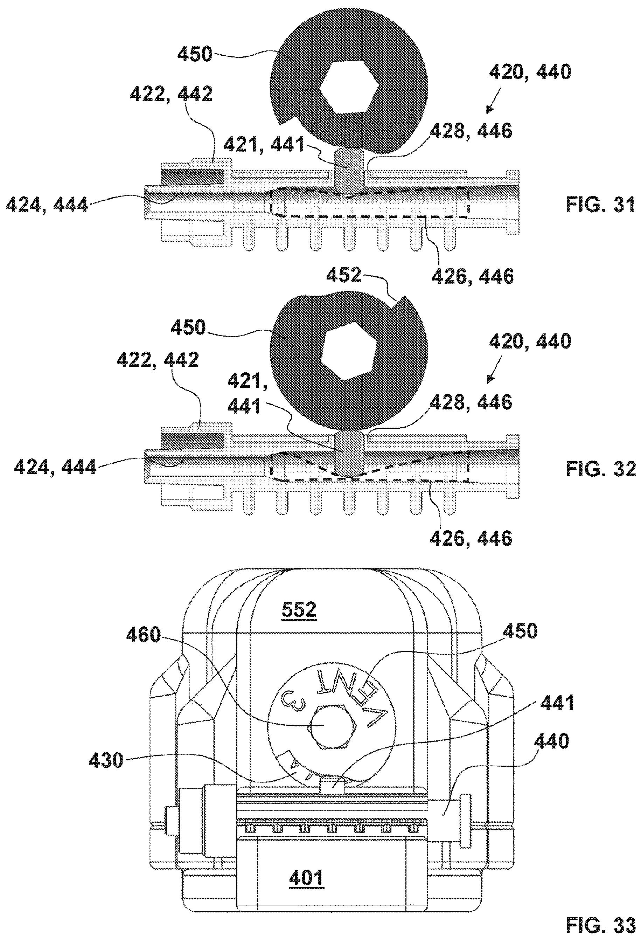

Embodiments of the pinch valves 420, 440 are explained with regard to FIGS. 31 to 33 using the vent pinch valve 440. Each valve 420, 440 comprises a valve body 422, 442 defining a vacuum or vent lumen 424, 444. An elastomeric tube 426, 446 is secured within the lumen 424, 444 at each end of the tube 426, 446. The connection can include fusing, compression sealing, fixation with an adhesive, or other suitable means. Accordingly, the tube 426, 446 spans an extent of the lumen 424, 444 with an intermediate portion of the tube 426, 446 unattached to the lumen 424, 444. A lumen of the tube 426, 446 fluidically connects a distal end of the lumen 424, 444 (to the left of FIGS. 31 and 32) to the proximal end of the lumen 424, 444 (to the right of FIGS. 31 and 32). The intermediate section of the valve body 422, 442 defines a follower connection in which is movably secured a cam follower 421. A first end of the cam follower 421 is biased against the outer surface of the cam 430, 450 with a non-illustrated bias device or is simply trapped in place. The opposing second end of the cam follower 421 rests against the intermediate portion of the tube 426, 446. Accordingly, when moved by the cam 430, 450 towards the tube 426, 446, as shown in FIG. 32, the cam follower 421 fluidically seals off the lumen of the tube 426, 446 and, when allowed to return away from the tube 426, 446, as shown in FIG. 31, the cam follower 421 opens the lumen of the tube 426, 446. The cam follower 421 can be pill-shaped or other suitable shapes that provide the function of closing off the tube 426, 446.

Both a vacuum line 402 and a vent line 404 are connected through the selectively openable valves 420, 440 to a proximal end of the aspiration catheter 410. In operation, the vacuum cam 430 and the vent cam 450 push down on the respective cam followers 421, which pinch down the short sections of tubing 426, 446, each respectively fluidically connected to the vacuum line 402 and the vent line 404. When the vacuum line 402 is open and the vent line 404 is closed, vacuum is drawn on the aspiration catheter 410. When the distal end of the catheter 410 is clogged with a clot, the closure raises a vacuum level within the catheter 410 to full (the greatest current vacuum generated by the vacuum pump). This closure creates a delta in pressure between the internal lumen of the catheter 410 and the environment external to the catheter 410, which change squeezes down the body of the catheter 410 both radially and longitudinally (e.g., the diameter and length become incrementally smaller). This change also draws out a small volume of liquid from within the lumen of the catheter 410. In some embodiments, the volume is approximately 0.2 ml. The end effect is the creation of a spring-like force within the catheter 410 that wants to expand the catheter 410 back to its steady state, but when the vacuum line 402 is closed off, that cannot happen. Thus, the vacuum is stored as potential energy until the vent line 404 is opened (as can be seen in FIG. 26, for example, the vacuum and vent lines 402, 404 are connected together distal of the valves 420, 440). When the vent line 404 is opened, there is an in-rush of fluid because of the pressure delta. This rush of fluid balances the radial force of the catheter 410 and draws in fluid to create a distally directed momentum in the column of fluid residing in the catheter 410 distal of the valves 420, 440. The momentum causes a small amount of fluid to move through a distal portion of the catheter 410 and create a small distal movement of the clot that is stuck in the distal opening at the end of the catheter 410. Once the clot is no longer stuck at the distal opening, it can be moved proximally into and through the catheter 410 with subsequent vacuum imparted to the catheter 410. Repeated selective actuation of vacuum and venting macerates the clot at the distal opening, thereby reforming it into a state where it can be completely drawn into the lumen of the catheter 410 and out of the vasculature.

The system 400 can be operated in various modes to remove clots in the vasculature. Rotation of the cams are measured in degrees, a full rotation being 360° of movement. In a first embodiment, the vacuum cam 430 is configured to establish vacuum in the catheter 410 through approximately 220° of rotation. The vent cam 450 is configured to have venting on through approximately 80° of rotation. The configuration of the cams 430, 450 stop both venting and vacuum between each respective application of vacuum and venting, for example, with a 30° rotation. This configuration, therefore results in operation states according to Table 1 below.

**Table 1**

| State | Vacuum | Venting | Cam Angle |
|---|---|---|---|
| Off | 0 | 0 | 0 to +30 |
| Vac | 1 | 0 | +30 to +250 |
| Off | 0 | 0 | +250 to +280 |
| Vent | 0 | 1 | +280 to 0 |

As soon as the vent is opened, there is an in-rush of fluid to balance out the vacuum pressure, then the vent line 404 is closed and the vacuum line 402 is opened, suddenly causing a rapid decrease in pressure that serves to forcefully pull the clot to the catheter. It is desirable, therefore, to close both vacuum and vent lines before resuming vacuum.

In another embodiment, the vacuum cam 430 is configured to establish vacuum in the catheter 410 through approximately 220° of rotation. The vent cam 450 is configured to have venting on through approximately 80° of rotation. Thus, there is created, in a desirable second configuration, a pause between vacuum draw in the catheter and venting of the catheter and another pause between venting of the catheter and resuming vacuum draw in the catheter. In this configuration, the pause can be through approx. 30° of rotation. To create a purge state, where vacuum and venting occur simultaneously, the vent cam 450 has a small inwards depression in a position of the vent cam 450 that occurs during a long vacuum-on stage (e.g., between +30° to +250°). The extent of the venting is configured to not provide a significant change in pressure or change in the vacuum energy but, instead, is configured to create a single rotation position of the cams 430, 450 where the motor control assembly 550 can stop rotation of the cam shaft 460 in that orientation where both the vacuum line 402 and the vent line 404 are connected to the catheter 410, which allows the user to purge out any air that might be present in the system (e.g., in the vacuum line 402, the vent line 404, and/or the catheter 410). The extent of the depression can be such that it only partially opens the vent to reduce the amount of vent liquid that is drawn in during this purge state. This purging can be a known position of the cam rotation and is placed in that position to ensure that all lines in the system 400 are cleared of air. Such a configuration results in operation states according to Table 2 below.

**Table 2**

| State | Vacuum | Venting | Cam Angle |
|---|---|---|---|
| Off | 0 | 0 | 0 to +30 |
| Vac | 1 | 0 | +30 to +120 |
| Purge | 1 | 1 | +120 to +140 |
| Vac | 1 | 0 | +140 to +250 |
| Off | 0 | 0 | +250 to +280 |
| Vent | 0 | 1 | +280 to 0 |

A third alternative configuration for operation of the system 400 can include a full-time vacuum with a pulsed venting including the operating states according to Table 3 below.

**Table 3**

| State | Vacuum | Venting | Cam Angle |
|---|---|---|---|
| Vac | 1 | 0 | 0 to +120 |
| Purge | 1 | 1 | +120 to +150 |
| Vac | 1 | 0 | +150 to 0 |

An opposite configuration to the states of Table 3 can including a full-time venting with a vacuum overlap.

A fourth alternative configuration for operation of the system 400 can include a vacuum during venting, which configuration includes the operating states according to Table 4 below.

**Table 4**

| State | Vacuum | Venting | Cam Angle |
|---|---|---|---|
| Purge | 1 | 1 | 0 to +30 |
| Vac | 1 | 0 | +30 to +250 |
| Off | 0 | 0 | +250 to +280 |
| Vent | 0 | 1 | +280 to 0 |

An opposite configuration to the states of Table 3 can include a full-time venting with a vacuum overlap.

A fifth alternative configuration for operation of the system 400 can include a venting during vacuum, which configuration includes the operating states according to Table 5 below.

**Table 5**

| State | Vacuum | Venting | Cam Angle |
|---|---|---|---|
| Off | 0 | 0 | 0 to +30 |
| Vac | 1 | 0 | +30 to +250 |
| Purge | 1 | 1 | +250 to +280 |
| Vent | 0 | 1 | +280 to 0 |

In further alternative configurations, there can be a variation overlapping of venting and vacuum, which would delete one or more of the OFF states in any of the state tables above.

The cam-driven valves 420, 440 allow the positional encoder driven motor to create positions for vacuum, venting, off, and purge. The motor controller assembly 550 allows the cams 420, 440 to be controlled by any frequency, e.g., they can be set to move through the various states at any given speed, for example, at 4 Hz. The frequency at which the motor runs may be more appropriate to run at lower frequencies such as 0.5 Hz, 1 Hz, or 2 Hz. Alternatively, it may be more effective to run at higher frequencies such as 8 Hz, 12 Hz, or 16 Hz. The motor control assembly 550 can also dynamically change the rate of cam shaft 460 rotation to sweep the frequency of rotation. In some embodiments, the step in speed is in a range from 1 Hz to approximately 4 Hz, the change in increment is between approximately 0.25 seconds to approximately 5 seconds, and the range of rotation is between approximately 2 Hz to approximately 12 Hz. One example for the step, increment, and range is 2 Hz and 1 second increments in the following progression 2 Hz/4/6/8/10/12/10/8/6/4/2/... Another example is 4 Hz with 0.5 sec increments in the following progression 4 Hz/8/12/8/4/... In some embodiments, the system uses the higher frequencies in the 8 Hz to 12 Hz range, which has been observed to have less movement of the proximal end of a clot stuck at the distal end of the catheter 410. Alternatively, further increments can be used to sweep the frequency through complex forms, such as sine, sawtooth, stepped, and pulsing variations.

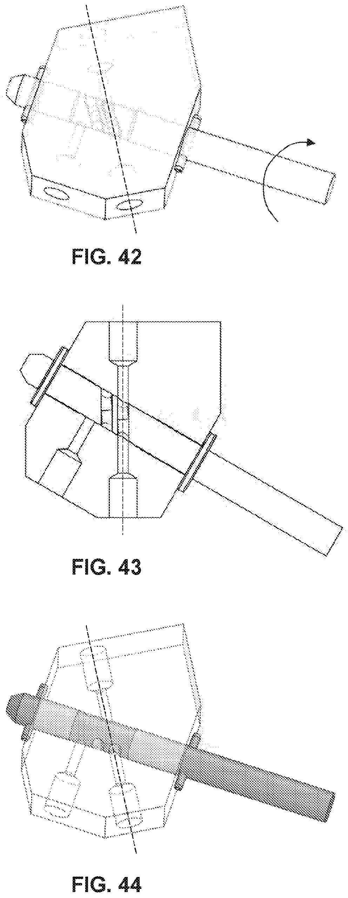

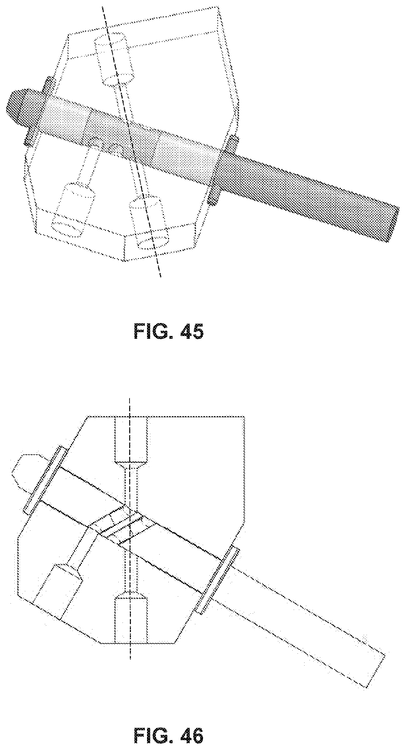

In an alternative to the pinch valves 420, 440, the valves can be solenoid-driven pinch valves or voice coil actuators. In another alternative, a rotational pintle valve can be used, as shown in FIGS. 42 to 46. The first valve state shown in FIGS. 42 to 44 can, for example, be a vacuum-on/vent-off state and the second valve state shown in FIGS. 45 and 46 can be a vacuum-off/vent-on state.

It has been determined that the most rapid onset of vacuum and venting is desirable. To create this rapid onset, the cams 430, 450 start vacuum and venting, respectively, with a cliff 452 in the shape of the cam 430, 450. Sudden creation of vacuum creates a rapid decrease of pressure inside the catheter 410, which draws the clot aggressively against the distal end of the catheter 410. Venting, as described above, creates a distal momentum that unsticks the clot and repetition of the vacuum and venting causes mechanical maceration of the clot at the distal opening until the clot completely enters the lumen of the catheter 410 and is removed from the vasculature. Therefore, the instant system 400 can be described as one embodiment of a Rapid Onset Aspiration Repeater (ROAR) system.

The control carried out by the motor controller assembly 550 has a selection of user-actuated buttons. In some embodiments, one button causes both vacuum and venting to be shut off, i.e., off operation. One button causes vacuum to occur in a continuous manner, i.e., manual control. One button causes venting to occur in a continuous manner, i.e., manual control. One button causes the cam shaft 460 to rotate the cams 430, 450 to the position in which the vacuum and vent lines 402, 404 can be purged, i.e., the purge function. One button causes the system to run or pulse repeatedly according to a desired set of states (e.g., according to any of Tables 1 to 5) along with a selection of any number of sets for step, increment, and range. As such, if the surgeon desires to use the system 400 as a simple thrombectomy device, the surgeon can just use the vacuum button. In this condition, the encoder 570 assists to have the cam shaft 460 to rotate to a position in which vacuum is open. The vacuum pump runs with a fully open vacuum until the surgeon releases the button. If the surgeon wants to purge or inject contrast, for example, then the surgeon can use the vent button to have the encoder 570 assist to rotate the cam shaft 460 to a position in which the vent is open. Likewise, the off button rotates the cam shaft 460 to a position where the cams 430, 450 close both the vacuum and vent lines 402, 404. The purge button causes rotation of the cam shaft 460 to a position where the cams 430, 450 allow simultaneous vacuum and venting.

In some embodiments of the run or ROAR mode, rotation of the cam shaft 460 is between approximately 0.5 Hz and approximately 25 Hz, further, approximately 6 Hz and approximately 16 Hz, in particular, between approximately 8 Hz and approximately 12 Hz. In some embodiments, the cam shaft 460 is rotated for between approximately 10 seconds and approximately 30 seconds and, during that time, the motor controller assembly 550 causes the motor 410 to sweep through frequencies between approximately 2 Hz and approximately 12 Hz.

As set forth above, the elastomeric tube 426, 446 is attached to distal and proximal locations of the valve lumen 424, 444. Compliance in the system 400 distal of the vacuum valve (described above as including reduction of the diameter and/or length of the catheter 410 as well as compliance of the tube 426, 446) when vacuum is applied to the catheter 410 and a clot is stuck at the distal end determines how much fluid is drawn out when the system 400 is under full vacuum and, conversely, how much fluid rushes back into the system 400 when that state is released. In other words, with a greater amount of compliance distal of the valves 420, 440, momentum imparted to the stuck clot by the column of fluid increases. It is desirable to have a minimal amount of momentum transfer from the fluid column to the stuck clot to unstick the clot sufficiently so that the next vacuum cycle macerates the clot against the distal end of the catheter 410 and causes it to enter the lumen of the catheter 410 and be removed from the vessel. To minimize this compliance (which is fixed for a given catheter 410), this tube 426, 446 is made as short as possible to still allow valve operation by the cam follower. Compliance as used herein refers to mechanical compliance of the catheter 410 and the tube 426, 446; it does not refer to any air that might be in the system 400, which air is purged before use as set forth herein. This desire for a reduction in compliance is one reason the valving system is connected directly to the proximal end of the catheter 410. This close connection minimizes overall compliance. In some embodiments, the valving system can be located away from the catheter 410 and, in such a case, substantially non-compliant tubing is desired. This configuration may experience lower performance due to the excess compliance.

To determine the status of a clot at the distal end of the catheter 410, the system 400 is put in the ROAR mode. If there are no sensors associated with the system 400, a surgeon cannot distinguish the situation when a clot is corked during ROAR or not. The surgeon has to turn off ROAR and visualize whether the catheter is corked (in which nothing is being drawn in by the catheter 410) or is not corked (in which blood is being drawn into the catheter 410). With the different situations of aborting pulse based on flow and pulsing until not corked, it is hard to know when a clot is corked.

The vent line 404 is connected to a vent liquid reservoir (not illustrated), which can contain for example, any of albumin, d5 water, normal saline, half-normal saline, and lactated ringers. When a fluid is used to vent the system 400, as described above, all air can be purged out of the system 400. Additionally, knowing that a given amount of vent liquid is used at various stages of clot removal can allow the user to correlate removal of a clot into the catheter after being stuck at the distal end to a rate of vent liquid use. In other words, the amount of vent liquid is different when the clot is corked compared to when it is not corked. Thus, a user or a sensor can look at or measure vent liquid use to determine whether to turn off the system. If the catheter 410 is aspirating without obstruction (uncorked), then a significant flow of blood will exit the system 400. If the catheter 410 is aspirating while corked, then no blood will appear at the vacuum exit. During a ROAR operation and the catheter 410 is uncorked, the user/sensor will detect some blood at the vacuum exit. Finally, during the ROAR operation when the catheter 410 is corked, the vacuum exit will receive some fluid that is a combination of both blood and vent liquid and, in this state, the flow rate of the vent liquid can indicate if the catheter is corked or uncorked.

If a surgeon visualizes free flow during vacuum and sees a captured clot (for example, in the thrombus trap 200), then the surgeon can perform a contrast injection with the catheter 410 to confirm revascularization without moving or removing the aspiration catheter 410. This contrasts with current state-of-the-art aspiration catheters where the catheter removes the clot by holding the clot corked on the end and the surgeon retracts the entire catheter to drag out the corked clot. The increased ability of a smaller diameter catheter to be able to fully ingest or secure a better grip on the clot by drawing a greater amount of it into the catheter is a significant benefit. Many clots are deep enough into the anatomy that it is difficult to get large catheters to the site of the clot. If a smaller diameter catheter can have increased effectiveness through ROAR than a greater number of clots can be accessed and retrieved.

It is noted that one desirable goal to achieve with the system 400 is to fully ingest a clot and bring it back through a standard aspiration canister (in a typical thrombectomy end reservoir) or into the thrombus trap 200. When using a standard aspiration canister and the thrombus trap 200, the system 400 can use the vent liquid to flush the thrombus trap 200 through the intake bleed valve 214 instead of using air. This allows retention of vacuum pressure in the aspiration canister.

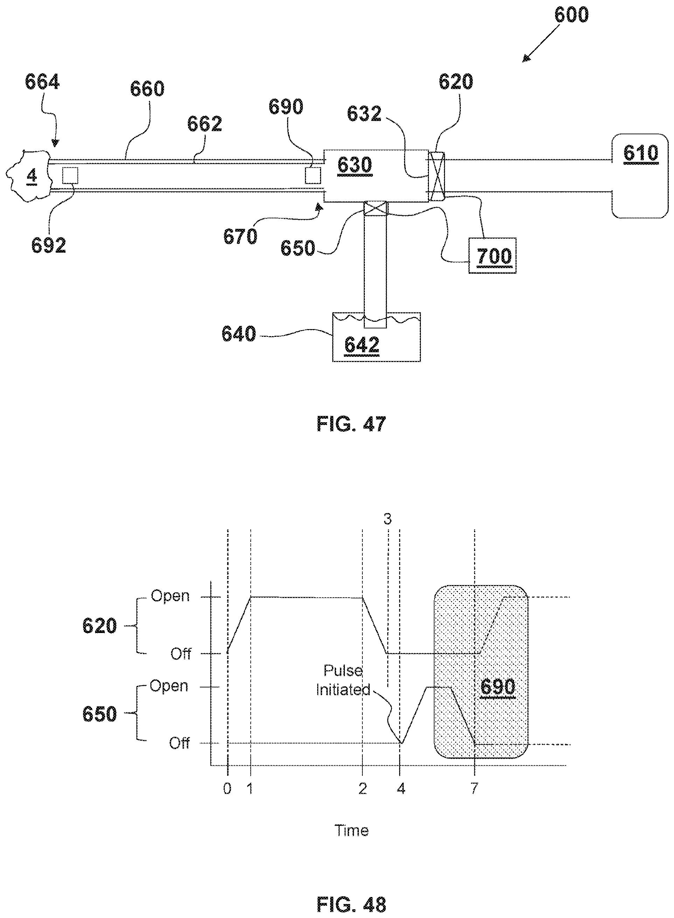

FIG. 47 illustrates diagrammatically some embodiments of an aspiration thrombectomy system 600 that operates in a ROAR mode. The system 600 comprises a vacuum source 610 fluidically connected to an input of a controllable vacuum valve 620. (Parts of the vacuum source 610, such as the collection canister, are not illustrated in FIG. 47 for reasons of clarity but are detailed below.) The vacuum valve 620 is fluidically connected to a vacuum input 632 of a manifold 630. The connection can be direct or through a conduit, such as silicone tubing. A vent fluid source or reservoir 640 containing a vent liquid 642 is fluidically connected to a controllable vent valve 650. The vent liquid 642 can be, for example, any of albumin, d5 water, normal saline, half-normal saline, and lactated ringers, to name a few. As used herein, "controllable" means that the device is able to be selected between various states, the selection including analog and/or digital switching. One embodiment is a digital switching between an open position and a closed with a single command (e.g., a change of bit 1/0). The entire working channel of the aspiration thrombectomy system 600 is to be free from air or other gaseous bubbles during use.

The vent fluid source 640 has a sufficient amount of vent liquid in the reservoir that will not end during a given surgical procedure and this prevents any possibility of air entering the system. If the vent fluid source 640 is flexible, such as with fluid supplied by a parenteral fluid containment bag or an intravenous therapy bag, the gas-free container will shrink as the vent liquid 642 is used. If the vent fluid source 640 is inflexible and has an air or gas pocket, as in a replaceable/removable and sterilizable container, the conduit that transfers the vent liquid 642 from the vent fluid source 640 to the vent valve 650 is at a level within the reservoir to keep the input of that conduit submerged within the vent liquid 642 throughout a given procedure.

A ROAR catheter 660 defines a working lumen 662 fluidically connecting a distal end 664 thereof to a proximal manifold connector assembly 670 at a proximal end of the ROAR catheter 660, which assembly 670 is described in greater detail below. The ROAR catheter 660 is configured to operate in relatively small vessels. Thus, in some embodiments, the lumen has an internal diameter of between approximately 0.038" and approximately 0.106" and, in particular, an internal diameter of between approximately 0.068" and approximately 0.088". The proximal manifold connector assembly 670 fluidically connects the lumen 662 to the interior of the manifold 630 and, thereby, the manifold 630 fluidically connects the lumen 662 to the vacuum source 610 through the vacuum valve 620 and to the vent fluid source 640 through the vent valve 650. This assembly, in some embodiments, comprises a removable cassette assembly 710.

In use within a vessel, the lumen 662 is filled with a liquid column having a proximal portion and a distal portion. Depending on the context used with respect to the catheter 660, the proximal and distal portions of the liquid column can be a given amount (e.g., less than 20 microliters or less than 5 microliters), can be a given length (e.g., a few mm or cm), or it can be an instance of the column that is approximated by using statistical flow analyses. For example, when discussing whether a distal portion of the fluid column exits the distal end of the lumen 662, that distal portion is a measurable distance at the distal end of the liquid column equal to an instance of liquid present at the plane of the lumen distal exit. In the realm of statistical analysis in this example, the distal portion is a last distal finite element in a finite element analysis (FEA) of the liquid column. The system 600 can be configured such that the distal shift of the fluid column during the vent phase is controlled such that a limited volume of liquid exits the distal end of the catheter to provide a minimal amount of momentum transfer to the clot material without ejecting the clot material from the distal end of the catheter whereby the reapplication of vacuum recaptures the clot material. As noted above, specific examples of embodiments of this are when the distal portion of the fluid column that exits the distal end is less than approximately 20 microliters, or less than approximately 5 microliters. Depending on parameters of the catheter system and the vent fluid (e.g., compliance, length, size of the vent injection inlet, vent fluid pressure, etc.), one specific example is when no greater than 6 microliters of fluid exits the distal end (approximately 2.35 mm of catheter length of I.D. 0.071"; approximately 3.29 mm of catheter length of I.D. 0.060"; approximately 1.53 mm of catheter length of I.D. 0.088"); less than these amounts may also be useful. For example, no greater than 2 microliters or, in a particularly beneficial embodiment, approximately zero microliters.

Operation of the aspiration thrombectomy system 600 occurs through a controller 700, which can be an analog controller or a digital controller. Examples of the analog controller are shown in FIGS. 25 to 46. An example of a digital controller is described in further detail below. One configuration for a digital controller is a microcontroller manufactured by Microchip Technology, Inc. The controller 700 is operatively connected to each of the vacuum valve 620 and the vent valve 650 (and to a vacuum motor as described below). The controller 700 selectively opens and closes the vacuum and vent valves 620, 650 such that, when the vacuum valve 620 is opened, the vacuum source 610 is fluidically connected to the liquid column in the lumen 662 and, when the vent valve 650 is opened, vent liquid 642 is fluidically connected to the liquid column in the lumen 662. The timing of these valves is significant so that the controller 700 can change a level of vacuum at the distal end 664 and prevent the distal portion of the liquid column in the lumen 662 from exiting the distal end 664. There are two significant actions that contribute to uncontrolled ejection of clot material when operating the valves 620, 650: compliance of the catheter system and the water hammer effect. Each will be discussed in turn. Configurations of the vacuum and vent valves are shown in FIGS. 25 to 36 and FIGS. 42 to 46. Configurations for the valves include spool valves, pinch valves, rotary valves, and rotary valve having a pintle design.

To explain timing of the valves to avoid uncontrolled ejection of clot material, reference is first made to the system depicted in the diagram of FIG. 47. It is noted that the ROAR catheter 660 is a flexible body and, therefore, it has compliance both in the radial direction and in the longitudinal direction. When the distal end 664 is corked with a thrombus (as shown in FIG. 47), vacuum is being applied to the lumen 662. Compliance of the catheter 660, therefore, causes reduction in the diameter of the catheter and reduction in the length of the catheter. When the catheter 660 corked, no flow occurs in the lumen. By having pressure lower than atmosphere within the lumen 662, the catheter 660 shrinks and reduces (shortens radially and longitudinally). This shrinkage acts like a spring squeezing down on the lumen in the catheter - in other words, it is a storage of potential energy. If the vacuum source is then cut off (e.g., the vacuum valve 620 is closed) and the vent valve 650 is opened to the vent fluid source 640, then the catheter 660 elongates and acts as a piston pulling against the vent liquid 642. Further, the vent liquid 642 is at a higher pressure (e.g., atmospheric pressure or slightly elevated by having a higher physical position than the patient) than the fluid in the lumen 662. Consequently, an amount of the vent liquid 642 enters through the vent valve 640 into the manifold 630 and then into the lumen 662 through the proximal manifold connector assembly 670. As the vent liquid 642 flows in and the catheter 660 expands to its normal or free steady state, momentum is created in the fluid column directed towards the distal end 664, referred to herein as a pressure pulse or pressure wave. In other words, a "pressure pulse" or "pressure wave" is momentum within a column of fluid that can act to move a distal portion of the fluid column in the catheter lumen distally out from a distal end of the catheter. This term relates to a given cycle of the vacuum and vent valves 620, 650 and is not limited to a single pressure transmission with that cycle. A pressure pulse, therefore, can include multiple pressure differentials with a given cycle of the vacuum and vent valves 620, 650. Thus, by adjusting a timing of the vacuum and vent valves to match a compliance and length of a particular catheter system (which can include the catheter and also the manifold and valves and other lumens in line with the catheter), a ROAR effect can be achieved for that catheter. In particular, by regulating a timing of the vent valve 650 and the subsequent reapplication of vacuum, the ROAR effect can quell the distal shift of the fluid column such that an exit flow out from the distal end of the catheter during each cycle is in a range from at least approximately zero to a limited predetermined volume of liquid so that the reapplied vacuum recaptures the thrombus before the thrombus is uncontrollably ejected from the distal end of the catheter.

Prior art aspiration thrombectomy systems periodically open and close a vacuum valve. Fluid rushes into the distal end of the catheter while the vacuum valve is open and vacuum is being applied to the fluid column. When the vacuum valve is closed, liquid rushing proximally through the lumen stops by hitting the closed vacuum valve. This causes pressure to build at the vacuum valve and create a bounce-back wave that carries momentum distally towards the distal end and ejects an amount of fluid distally from the distal end of the catheter. This action is referred to as a water hammer effect. The prior art repetitively opens and closes that vacuum valve. Thus, an amount of liquid ejects in a periodic manner out of the distal opening in those devices. This phenomena is undesirable in the area of thrombus removal because, when liquid is allowed to eject from the distal end and the physician is causing the distal end to approach the thrombus, the liquid could or will move the thrombus further distally, or it could break the thrombus up to allow arterial pressure to push the broken pieces further downstream, e.g., into smaller brain arterial vessels. In some embodiments, it would be desirable to entirely prevent any distally directed pressure pulse reaching the distal end of the aspiration catheter in a thrombus aspiration removal system. As described herein, the system 600 has a response to the water hammer effect that is tuned to safely achieve a maximum water hammer effect, which response achieves the most effective engagement and disruption of the thrombus.

Proximal and distal pressure measurement devices 690, 692 are illustrated diagrammatically in FIG. 47. In this embodiment, the proximal pressure measurement device 690 is adjacent or within the proximal manifold connector assembly 670 and/or within the proximal portion of the fluid column, and the distal pressure measurement device 692 is adjacent or within the distal end 664 or within the distal portion of the fluid column. Some embodiments for measurement devices 690, 692 include a pressure transducer manufactured by TransducersDirect.com.

Measurement in the fluid column of the catheter 660 at or adjacent the manifold 630 and adjacent the distal end 664 reveals a time delay in travel of the pressure pulse - the pressure rises at the manifold 630 first and then pressure rises at the distal end 664 later. By knowing the time delay and the distance between the sensors, the speed of the wave can be calculated. By knowing the distance from the most distal sensor to the tip of the catheter 660, the time it will take for the wave to travel to the distal tip can be calculated. This information can be used by the controller to time the valves properly to stop the pressure pulse. If the pressure pulse is allowed to travel all the way to the distal end 664, then a distal portion of the fluid column in the lumen 662 will exit the distal end 664. If, during this time, the distal end 664 is corked with a thrombus 4, that pressure pulse could or will uncontrollably eject the thrombus 4 distally if it is too large. Alternatively, if the distal end 664 is approaching a thrombus 4, any pressure pulse exiting the distal end 664 could or will move the thrombus 4 further distally. Movement of the thrombus 4 in a distal direction before or after it has been captured and corked at the distal end 664 of the catheter 660 is to be avoided. Therefore, the pressure pulse needs to be reversed or stopped before that pressure pulse reaches a point where it could move the thrombus 4 either further downstream or off of the distal end 664 in the distal direction. Such reversal is referred to herein as "quelling" the pressure pulse.

Operation of the aspiration thrombectomy system 600 with the ROAR effect does not produce the same results as prior art catheters. When operated with the distal end 664 unobstructed, the vacuum valve 620 and the vent valve 650 are periodically opened and closed. Fluid rushes into the distal end 664 of the catheter 660 and into the canister of the vacuum source 610 while the vacuum valve 620 is open and vacuum is being applied to the fluid column. When the vacuum valve 620 is closed, the sudden stop of flow creates the pressure wave generated as described above due to the water hammer effect from the closing of the vacuum valve 620. The controller 700 is timed to control the vacuum and vent valves 620, 650 to create the ROAR effect even when the distal end 664 is open to vasculature and, therefore, distally directed pressure pulses in the aspiration thrombectomy system 600 are quelled so that during a thrombus retrieval procedure. Through experimentation, net flow of liquid at the distal end 664 remains positive in the proximal direction - in other words, while operating with the ROAR effect in the corked or un-corked state, liquid either moves through the distal end 664 towards the vacuum source (when un-corked) or does not flow at all (when corked). In both circumstances, substantially no liquid exits the distal tip 610.

To accomplish the ROAR effect, the change in the level of vacuum at the distal end is at least approximately 15 inHg, further, at least approximately 20 inHg, and, in particular, at least approximately 25 inHg. A time for the change in the level of vacuum from low to high or from high to low at the distal end is no greater than approximately 50 ms, further, no greater than approximately 30 ms, and, in particular, no greater than approximately 20 ms. This change can be referred to as a maximum pressure delta. Various combinations of these variables include a change in the level of vacuum of approximately 15 inHg and a time for that of no greater than 50 ms, or the change in the level of vacuum of approximately 20 inHg and a time for that change of no greater than 30 ms, or the change in the level of vacuum of approximately 25 inHg and a time for that change of no greater than 20 ms.

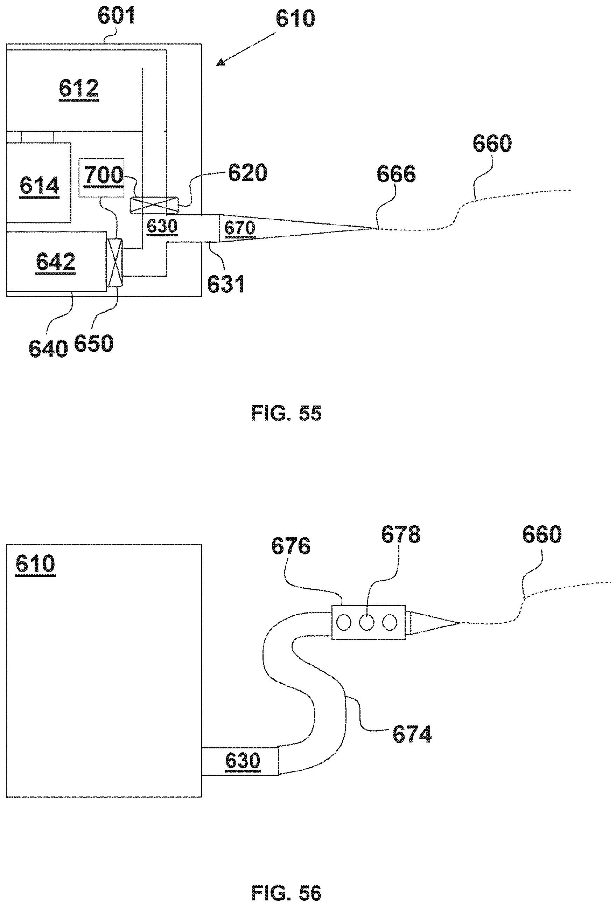

The ROAR catheter 660 is operated to quell all pressure pulses in some embodiments according to the graph of FIG. 48. The state of the vacuum valve 620 is shown in the waveform at the top of the graph and the state of the vent valve 650 is shown in the waveform at the bottom of the graph. The repetitive cycle starts at time 0 with the valve starting to open in this embodiment. At time 1, the vacuum valve 620 is fully open and the vent valve 650 is closed. Vacuum continues until time 2, when the vacuum valve 620 starts to close. Closing of the vacuum valve 620 is not instantaneous and, therefore, the vacuum valve waveform decreases at a sharp angle and is fully closed at time 3. After the vacuum valve 620 is closed, at time 4, the vent valve 650 starts to open. This closing of the vacuum valve 620 initiates a water hammer and the closing of the vacuum valve 620 and subsequent opening of the vent valve 650 causes vent liquid 642 to enter the manifold 630 (and possibly the proximal end of the lumen 662). The potential energy stored in the compliant catheter 660 is also allowed to release due to the change in pressure from the negative pressure generated by the vacuum source 610 to the relatively larger pressure (e.g., arterial) existing in the vent fluid source 640. This combination of events initiates a pressure pulse at time 2 that travels distally through the lumen 662 towards the distal end. If there was no further change in the valves 620, 650, then liquid in the column may uncontrollably eject out from the distal end 664. However, as shown in FIG. 48, after a relatively short vent-open time compared to the vacuum-on time, the vent valve 650 is closed (at time 7) and, shortly thereafter, the vacuum valve 620 is opened. This means that, while the pressure wave is travelling distally along the length of the lumen 662 of the catheter 660, when the vent valve 650 is closed to turn the vent liquid 642 off and the vacuum valve 620 is opened to turn vacuum back on (time 0 of the repeating waveform), switching of these valves 620, 650 causes vent liquid 642 to cease entering the manifold 630 and to move the fluid in the manifold 630 and in the lumen 662 proximally into the collection canister 612 of the vacuum source 610 (see, e.g., FIG. 55). Thus, a reverse momentum is imparted within the fluid column.

This reverse momentum can be sufficiently large enough to prevent the pressure pulse from ever reaching a point where the distal portion of fluid in the lumen 662 exits the distal end 664 - thereby fully quelling the pressure pulse before it reaches the distal end. In other applications, the reverse momentum is timed such that there is only limited exit flow from the distal end 664 that is not more than a predetermined volume of the distal portion of the fluid column in the lumen 662 so that reapplication of vacuum recaptures the clot material - thereby effectively quelling the pressure pulse to enable the reapplied vacuum to draw the clot material back quickly and forcibly into the catheter. In some embodiments the ROAR effect retains a level of pressure at the distal end at less than, equal to, or greater than physiological pressure. The area 690 of the two waveforms shown in FIG. 48 includes a time at which the pressure pulse has been quelled. In some embodiments, waveforms repeat in a periodic manner to continue the distal-then-proximal momentum pulse without allowing the distal portion to exit the distal end 664 of the catheter 660, while in other embodiments a limited volume of the distal portion can exit the distal end 664. The rapid change in pressure at the catheter tip from near full vacuum to nearly zero vacuum pressure or slightly above mean arterial pressure is the ROAR effect. In some embodiments of the ROAR effect, pressure at the distal end 664 can rise to just short of being arterial pressure and reversal of that rise is, then, started due to the reestablishment of vacuum. This allows the aspiration thrombectomy system 600 to approach a thrombus 4 without imparting distal movement to the thrombus 4 and to retain the corked thrombus 4 at the distal end 664 without any distal movement of the thrombus 4 caused by a change in pressure within the fluid column.

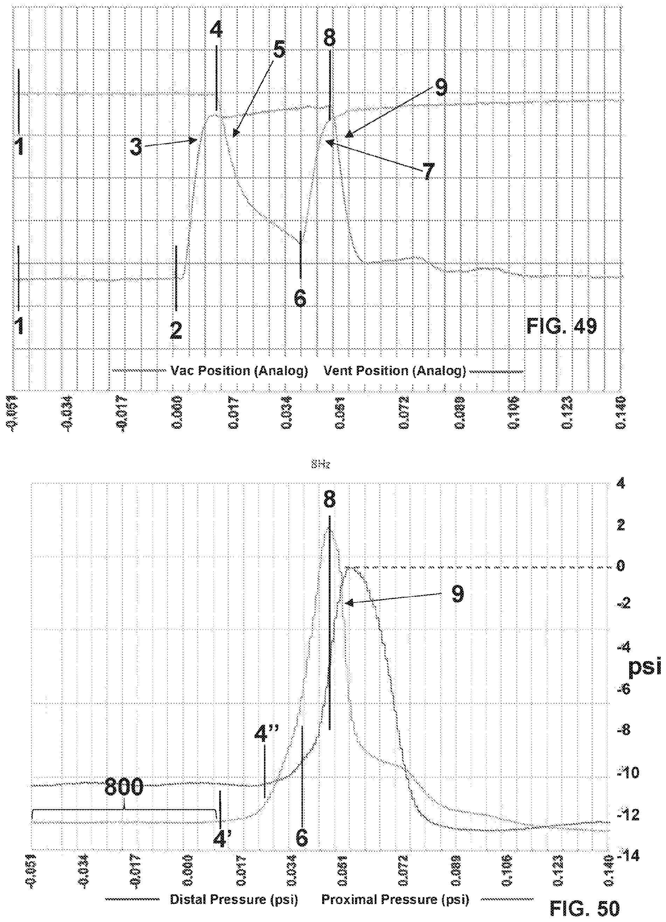

Another embodiment for creating the ROAR effect with the catheter 660 utilizing the vacuum valve 620 and the vent valve 650 is shown in the waveforms of FIG. 49, which are repeated at a rate of between approximately 1 Hz and approximately 250 Hz, further, between approximately 2 Hz and approximately 20 Hz, still further, between approximately 4 Hz and approximately 12 Hz, in particular, between approximately 6 Hz and approximately 8 Hz. At time 1, the vacuum valve 620 is in the open/on position and the vent valve 650 is in the closed/off position. At approximately time 2, the vacuum valve 620 starts transitioning to the closed/off position. At approximately time 3, the vacuum valve 620 is closed/off. At time 4, the vent valve 650 starts to open and at approximately time 5, the vent valve 650 is fully open. The vent valve 650 remains open while the vacuum valve 620 is closed until time 6, at which the vent valve 650 starts to close. The vent valve 650 is closed at approximately time 7. The vacuum valve 620 starts to open at time 8 and is partially open at approximately time 9. The vacuum valve 620 is full open when near the bottom extent of the curve in the graph. This process is repeated periodically, which in this example is at 10 Hz.

In what is referred to herein as static aspiration, the distal end 664 of the catheter 660 is pushed against a thrombus 4 while suction is applied to the catheter 660. The lower pressure in the catheter 660 creates a force on the clot 4 equal to a pressure differential across the clot 4 multiplied by the area of the inner diameter of the catheter 660. It is this force that "sticks" the clot 4 to the distal end 664 of the catheter 660 in an attempt to retrieve the clot 4 entirely.

In the ROAR cycle, suction is applied to the clot by rapidly opening a valve, causing a rapid rise in vacuum pressure. The source of suction is then turned off and a vent fluid source is rapidly opened. This relieves the vacuum present in the catheter 660, which again rapidly changes the pressure applied to the clot. The vent valve 650 is then rapidly closed and the vacuum valve 620 is rapidly opened. This cycle is repeated multiple times per second. For example, the period for repetition is between approximately 2 Hz and approximately 16 Hz, in particular, between approximately 8 Hz and approximately 12 Hz. The rapid drop of pressure across the clot 4 when the vacuum valve 620 is opened causes the clot 4 to accelerate into the lumen 662 of the catheter 660. The release of vacuum pressure when the vent valve 650 is opened causes the clot 4 to rebound back from the catheter 660. When the vacuum is applied again, the clot 4 once again accelerates towards the catheter 660. These accelerations and rebounds cause the distributed mass of the clot 4 to oscillate. The large internal accelerations of the distributed mass from the oscillation creates internal forces in the clot 4 that are high enough to exceed the tensile strength of the clot 4 and cause it to fail. The torn pieces of clot 4 are then aspirated all the way through the catheter 660 and into the vacuum collection canister 612. To maximize forces in the clot, the pressure differential across the clot and the rate at which this differential pressure is applied is maximized. The higher the rate at which this force is applied to the clot, the higher the internal acceleration of the distributed mass of the clot, and thus the higher the internal forces within the clot, and thus the higher the likelihood of the clot to tear. The times for the pressure change in both the up and down directions are about 20 ms either way. At 8 Hz, for example, each cycle is 125 ms and at 12 Hz each cycle is 83ms. The greater the frequency of the cycle, the greater the number of these impacts that the catheter can have to interact with the clot. Using higher frequencies is, therefore, better, but only up to a point where there is not enough time to cause an effective enough pressure delta within each cycle.

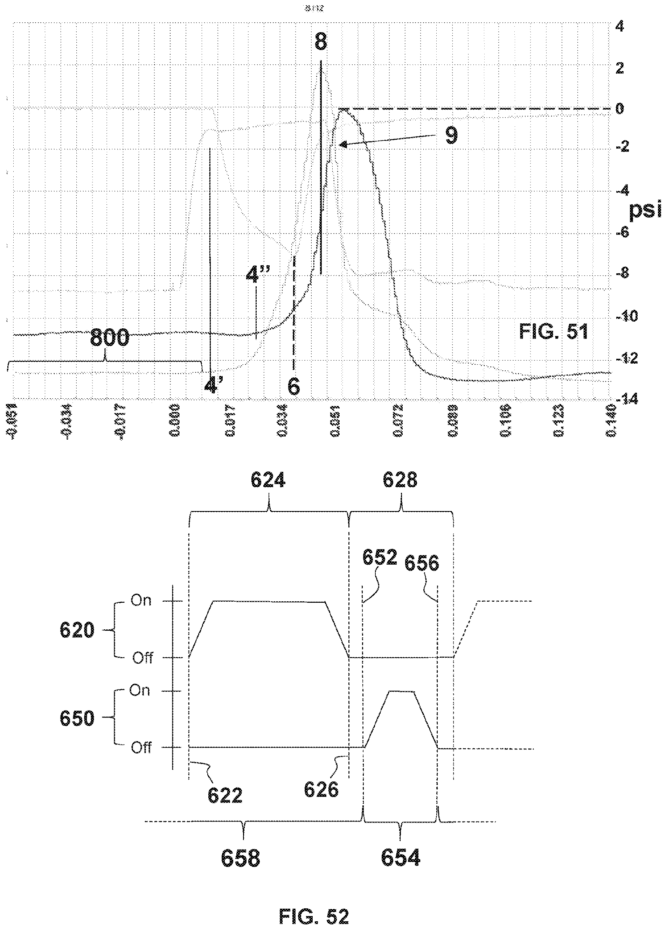

During operation of the catheter 660 with the ROAR effect, measurement of the pressure pulse can be undertaken with the proximal and distal pressure measurement devices 690, 692. The graph of FIG. 50 illustrates pressure sensed by these devices 690, 692 during the 10 Hz pulse present in FIG. 49 (FIG. 51 illustrates the graphs of FIGS. 49 and 50 superimposed on one another). The pressure sensed by the proximal pressure measurement device 690 starts at the lower pressure value (approximately at -13 psi) and the pressure sensed by the distal pressure measurement device 692 starts at a higher pressure value (approximately at -11 psi). This represents a partially corked system where an incomplete seal of the thrombus simulant to the catheter allows some flow by creating a slightly lower pressure at the distal measurement. At time 4, vacuum is off and the vent valve 650 starts to open. Accordingly, vacuum in the lumen 662 starts to be relieved. This means that pressure at the proximal pressure measurement device 690 starts to increase, which is evidenced by the upwards curve starting at approximately 4'. A short while later, as the change in pressure propagates distally down the catheter 660, pressure at the distal pressure measurement device 692 starts to increase, which is evidenced by the upwards curve starting at approximately 4". At time 6, the vent valve 650 starts to close and, therefore, no more vent liquid 642 is entering the manifold 630 to add to or augment the fluid column in the lumen 662. Pressure at the proximal pressure measurement device 690, nonetheless, continues to rise as the vacuum (negative pressure) is entirely removed or is compensated by the pressure of the vent liquid 642. Pressure at the proximal pressure measurement device 690 peaks and, as shown in FIG. 50, the pressure at the peak is at a positive pressure of approximately 2 psi - this occurs even though the aspiration thrombectomy system 600 does not actively apply any positive pressure to the fluid or the lumen 662. Rather, this level of pressure being > 0.0 psi is due to the momentum of the fluid traveling within the lumen 662. Thus, a positive pressure within the lumen 662 is acceptable but it needs to be suppressed before arriving at distal end. At time 8, the vent valve 650 has already closed and the vacuum valve 620 starts to open at approximately the time of peak pressure at the proximal pressure measurement device 690. Opening of the vacuum valve 620 drops pressure within the lumen 662 and stops pressure at the proximal pressure measurement device 690 from going any higher (if not stopped at this level, then it is possible that pressure at the distal pressure measurement device 692 would be > 0.0 psi, which means distal exit flow will occur out from the distal end). Quelling of the pressure pulse is proven by review of the pressure track of the distal pressure measurement device 692. As can be seen in the graph of FIG. 50, the pressure increase at the distal pressure measurement device 692 follows the pressure increase at the proximal pressure measurement device 690. At time 8, pressure recorded at the distal pressure measurement device 692 is still negative (approximately at -5 psi) but is rising. With continued operation of vacuum to and past time 9 (when the vacuum valve 620 is fully open), the peak pressure measured at the distal end 664 by the distal pressure measurement device 692 is less than 0.0 psi (horizontal dashed line in FIG. 50), which means that the pressure pulse is quelled and that liquid from the distal portion does not exit the distal end 664. The ROAR effect allows the clot simulant to remain sealed to the catheter and the system 600 is able to achieve the full vacuum of -13 psi at both measurements. Because the distal pressure is relieved almost to zero but then shortly arrives at the full -13 psi vacuum, the pressure delta illustrated is approximately 13 psi.

As described herein, it is possible to force flow from the distal end 664 of the catheter 660 while cycling between vacuum and vent. The rapid switching between vacuum and vent creates pressure pulses in the fluid column that, if not managed, will force the fluid column out of the distal end 664 of the catheter 660. The waves move through the fluid column at a very high speed through the medium. The speed is primarily a function of the density of the fluid, the compliance of the system (the bulk modulus), and the length of the fluid column. To prevent these waves from forcing the fluid column out of the catheter, the system is considered as a whole and the parameters of the valve switching cycle are set such that the forces that cause the fluid column to flow are controlled. To ensure that the fluid column does not exit the distal end 664 of the catheter 660, it is important that the catheter 660, any extension tube that connects to the catheter 660, the controller 700, and the valving sequence be tuned as a system.

The goal of the tuning process is at least two-fold: prevent the pressure waves generated during ROAR operation from uncontrollably ejecting the clot material distally within the vessel and optimize the ROAR effect. The length of the fluid column is critical to the tuning of the system. The pressure wave moves rapidly within the fluid column. The time for the wave to reach the distal tip of the catheter is a function of this speed and the length of the fluid column. The speed is a function of the density of the fluid in the column and the bulk modulus of the catheter and extension. The bulk modulus refers to the compliance of the system: both the radial and the longitudinal flexibility of the catheter and the extension tube. The density of the fluid column is not as significant a variable as the bulk modulus unless it changes greatly, such as is the case if the fluid column has gaseous (air) bubbles in it. It is thus, important, to have all air purged from the system prior to implementing ROAR operation. For a given catheter and extension tube configuration, the bulk modulus and the length of the system is fixed. Compliance can be added to the system to change the speed and thus tune the timing of the pressure wave. A flexible length of tubing could be added in-line with the relatively stiff catheter and extension, for example. This flexible length of tubing expands as the pressure pulses occur during ROAR operation. This decreases the bulk modulus of the system and reduces the speed, thus slowing down the pressure wave and increasing its transit time to the distal tip of the catheter. Compliance can be added in other ways as well, such as by including a piston backed by a spring in a bore that communicates with the catheter lumen such that the pressure wave displaces the piston, which increases compliance of the system. By manipulating the compliance and the valve timing, the system can be tuned for many different combinations of catheters and extension lines. Careful tuning results in achieving a resonant condition. If the suction and release pulses in the catheter are tuned to match a natural frequency of the clot, enhanced ROAR effect can be achieved.

Tuning can happen statically or dynamically. A statically tuned system is tuned so that the catheter 660 (and any extension tube that connects to the catheter 660) is mated to the controller 700 with a fixed valving sequence (such as the configuration shown in FIGS. 29 and 30). The controller 700 senses the presence of the catheter 660 when it is attached and verifies that it is the correct one for the tuned sequence of that controller 700. If the correct catheter is not sensed, the tuned sequence does not initiate. A dynamically tuned system is, in comparison, tuned during operation. Prior to operation, a valve sequence is initiated that creates a series of pressure pulses in the catheter 660. Sensors, such as strain gauges, on the catheter 660 and/or an extension tube detect these pulses and are used to adjust parameters of the controller 700 for operation to create the ROAR effect. The catheter 660 contains and transmits critical data, such as its length, to the controller 700. Using this tuning, any catheter, within limits, could be used without causing the fluid column to flow from the distal end 664 during ROAR operation. Alternatively, the catheter 660 and the extension can be tuned at the manufacturer and the specifics of the valve timing can be transmitted to the controller 700 by the catheter 660.

In some embodiments, the valve sequence is as follows:
the vacuum valve 620 is closed;
a time later the vent valve 650 is opened;
a time later the vent valve 650 is closed;
a time later the vacuum valve 620 is opened; and
a time later the sequence is repeated.
When the vent valve 650 is opened, a bit of vent liquid 642 enters the system and creates a pressure pulse. If the vent valve 650 is not closed, and the vacuum valve 620 is not opened before the pulse reaches the distal end 664 of the catheter 660, the fluid column can exit the distal end 664 of the catheter 660. To prevent the fluid column from exiting the catheter 660 or to prevent too much fluid from exiting the catheter before vacuum can act against the clot material, it is this time -- the time that it takes for the pressure pulse to traverse the catheter -- for which the system can be tuned. Additionally, the pressure pulse from closing the vacuum valve 620 in a flow condition will cause a pressure increase that can be quelled by opening the vent valve 650.

Tuning is accomplished by selecting appropriate times for the vacuum cycle and the vent cycle. In this regard, the vacuum cycle includes Vac-ON time 622, Vac-ON duration 624, Vac-OFF time 626, and Vac-OFF duration 628. Similarly, the vent cycle includes Ven-ON time 652, Ven-ON duration 654, Ven-OFF time 656, and Ven-OFF duration 658. Accordingly, tuning is explained with reference to FIG. 52. A cycle time is the duration of the repetition of the entire cycle. At 8 Hz, the cycle time is 125 ms and at 12 Hz, the cycle time is 83.33 ms. The cycle time is determined by adding the Vac-ON duration 624, the Ven-ON duration 654 and the first and second times in the cycle that both the vacuum and vent valves 620, 650 are off, referred to as the "double-off" or "double-closed" times or states. The cycle time is optimized by the dynamics or the resonance of a particular catheter 660. The Vac-ON duration 624 must be long enough for the system to pump down to full vacuum and the longer the vacuum is on during a particular cycle, the better the aspiration of the thrombus 4. In this embodiment, opening only the vacuum valve is referred to as the "vacuum-only" state. The first double-off time, which is the time after the vacuum is turned off (vacuum valve 620 closed) up until the time that venting begins (vent valve 650 opens), has an effect on the extent to which exit flow occurs. As this is a short time, such exit flow is referred to as flow burping. Through experimentation, some embodiments of an 0.071" inner diameter catheter 660 experiences flow burping when the first double-off time is greater than approximately 30 ms; the longer the double-off time, the greater flow burping. During ROAR operation, the first double-off time is about 10 ms; therefore, this is significantly less than the flow burping threshold, which means that substantially all exit flow is quelled. The Ven-ON duration 654 is determined by a maximum time that occurs before a corked clot 4 is dropped from exit flow. A ratio between the Ven-ON duration 654 and the second double-off time is a compromise for the longest Ven-ON time 654 and a minimum of the first double-off time. In this embodiment, opening only the vent valve is referred to as the "vent-only" state. Finally, with respect to the second double-off time, the vent valve 650 is off (fully closed) before the vacuum valve 620 is opened and vacuum recommences.

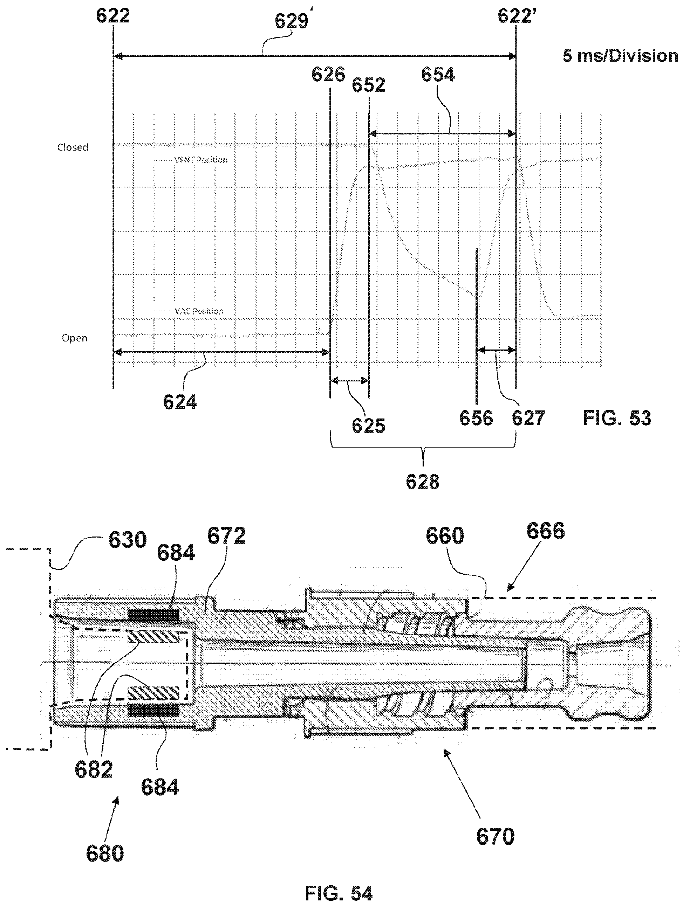

The calculation is explained further with regard to the valve position graphs in FIG. 53. Starting from the left of the graph at Vac-ON time 622, the vent valve 650 is closed and the vacuum valve 620 is open. The Vac-ON duration 624 is long enough for the system 600 to pump down to full vacuum (between approximately 10 ms and approximately 50 ms, in particular, approximately 30 ms). The longer the Vac-ON duration 624, the better the catheter 660 performs because of increased flow rate in the proximal direction. There is a compromise based on achieving a higher frequency for more hits/sec on the clot 4. In some embodiments, the Vac-ON duration 624, calculated from the Vac-ON time 622 to the Vac-OFF time 626, is between approximately 40% and approximately 60% of the cycle time 629. As set forth above, the cycle time 629 is a minimum determined by summation of Vac-ON duration 624 plus the first and second double-off times 625, 627 plus the Ven-ON duration 654. The Ven-ON duration 654 is short enough to fill the lumen with vent liquid without causing undo exit flow (between approximately 10 ms and approximately 50 ms, in particular, approximately 30 ms). The first double-off time 625 is set based upon when open flow burping occurs. The maximum value for the first double-off time 625 applies to either of the periods from the Vac-OFF time 626 to the Ven-ON time 652 or the Vac-OFF time 626 to the Vac-ON time 622' whichever is shorter. Each of these values are optimized by the dynamics/resonance/compliance/length of the particular catheter 660 and extension set.

From this, some observations can be made. When the vent is opened, a pressure pulse is generated. In some embodiments, it is important to stop the pressure pulse before it reaches the distal end to prevent exit flow that uncontrollably ejects the clot material from the distal end such that the subsequent reapplication of vacuum is not able to recapture the clot material (e.g., draw the clot material against the catheter). The way to stop the pressure pulse is to either close the vent and/or turn the vacuum back on if the vacuum was off prior to venting. If the vacuum remains on, then there is a need to turn the vent off. Or, if the vacuum does not remain on, the vent is turned off and the vacuum is turned back on before the pressure pulse makes it to the distal end 664. In other words, in such embodiments the vent needs to be closed before the pressure pulse makes it to the distal end 664 and the vacuum has to be turned on. So, the condition of merely opening the vacuum valve 620 when the vent valve 650 is opened may not be enough to quell the pressure pulse because of the low resistance between the vent and the vacuum; the vent will overwhelm the vacuum so the vacuum cannot have an effect over the length of the catheter. The time that it takes for the pressure pulse to propagate to the tip of the catheter 660 and then cause exit flow is used to define the time that the vent valve 650 is left open. The time that the vent is left open is selected to be shorter than the time it takes for the pressure pulse to propagate to the distal end 664.

In some embodiments illustrated diagrammatically in FIG. 55, all portions of the aspiration thrombectomy system 600 except for the ROAR catheter 660 are incorporated into the body 601 of the vacuum source 610. In particular, the vacuum source 610 comprises the body 601, a collection canister 612, and a vacuum motor 614. The vacuum motor 614 is fluidically connected to an outlet of the collection canister 612 and the input of the collection canister 612 is fluidically connected to a vacuum side of the manifold 630. Accordingly, vacuum generated by the vacuum motor 614 imparts vacuum within the collection canister 612 to draw fluid into the collection canister 612 from the manifold 630 but not into the vacuum motor 614. The vacuum valve 620 present at the manifold 630 prevents input fluid received at the manifold 630 from entering the collection canister 612 and closes off the manifold 630 from vacuum generated by the vacuum motor 614. The vent fluid reservoir 640 containing the vent liquid 642 is fluidically connected to a vent side of the manifold 630. The vent valve 650 present at the manifold 630 closes off the manifold 630 from the vent liquid 642 and prevents liquid within the manifold 630 from entering the vent fluid reservoir 640 (as pressure in the manifold 630 is typically lower than pressure within the reservoir 640, liquid from the manifold 630 will not typically enter the reservoir 640). In summary, a catheter input port 631 of the manifold 630 is fluidically connected to the collection canister 612 through the vacuum valve 620 and is fluidically connected to the vent liquid 642 in the reservoir 640 through the vent valve 650. The catheter input port 631 is fluidically connected to the downstream end of the proximal manifold connector assembly 670. The upstream end of the proximal manifold connector assembly 670 is fluidically connected to the proximal end of the catheter 660.

Direct connection of the catheter 660 to the aspiration thrombectomy system 600 is explained with regard to FIGS. 54 and 55. The proximal manifold connector assembly 670 connects the proximal end 666 of the ROAR catheter 660 to the manifold 630. In some embodiments shown in FIG. 54, the proximal manifold connector assembly 670 comprises a male luer lock fitting 672 connected to the manifold 630 (shown in dashed lines), either removably or integrally. The assembly 670 includes a ROAR identification (ID) sub-assembly 680. The embodiment of the ID sub-assembly 680 shown in FIG. 54 comprises an inductive sensing device or sensor 682 connected to the manifold 630. The inductive sensor 682 detects the presence of an inductive sensed part 684 that is present in or integral with the proximal manifold connector assembly 670. In some embodiments where the manifold 630 can be used with various different ROAR catheters 660, each of the types of ROAR catheters has a unique inductive sensed part 684 and the inductive sensor 682 of the manifold 630 is able to determine which type of ROAR catheter 660 is attached. Accordingly, with an appropriate communication of the ROAR catheter 660 type to the controller 700, the controller 700 is able to operate the ROAR catheter 660 according to its own unique configuration to produce the ROAR effect for every one of the different ROAR catheters 660 that are used. When the sensor 682 does not detect a sensed part 684 and the aspiration thrombectomy system 600 is, nonetheless, operated, the controller 700 will automatically prevent ROAR operation of aspiration thrombectomy system 600 and that connected catheter will only be operated as a standard vacuum catheter.

Indirect connection of the catheter 660 to the aspiration thrombectomy system 600 is explained with regard to FIGS. 56. The proximal manifold connector assembly 670 can simply be a fitting 672 as shown in FIG. 54 or it can be or include a separate extension line 674 between the catheter input port 631 and whatever catheter 660 (standard or ROAR) that is to be used along with the aspiration thrombectomy system 600. In some embodiments of the extension line 674, not only does the extension line 674 comprise a lumen extension for aspiration through the catheter 600, the extension line 674 also comprises system controls for operating the aspiration thrombectomy system 600. These controls include, for example, turning on and off the vacuum motor 614 and turning on the ROAR operation (e.g., one button for each of these or an on/off switch for vacuum and a push-to-start button for ROAR). As shown in the diagram of FIG. 56, the extension line 674 has a distal end that is able to connect to both standard catheters and ROAR catheters 600 -- as both can be used with the aspiration thrombectomy system 600. When the standard catheter is connected to the extension line 674, the aspiration thrombectomy system 660 only acts as a standard vacuum pump and ROAR is disabled. When a ROAR catheter 660 is connected to the extension line 674, identification sub-assemblies in the ROAR catheter 660 and the extension line 674 inform the system 600 which ROAR catheter 660 and which extension line 674 are connected.

In some embodiments with digital control of the vacuum and vent valves 620, 650, a processor and memory in the controller 700 stores the identification data and, upon identification of a particular ROAR catheter (e.g., different lengths, different outer diameters, different materials), the controller 700 loads the valve sequence and operates the vacuum and vent valves 620, 650 according to the characteristics of the particular catheter connected to the vacuum source 610. In one embodiment, the identification data can be preprogrammed at the manufacturer for all ROAR catheters 660 that currently exist. Thus, with direct connection of the ROAR catheter 660 to the system 600, the controller 700 can operate without receiving any information other than the identity of the catheter 660. If a ROAR extension line 674 is used between the ROAR catheter 660 and the controller 700 (in other words, an extension line that is ROAR compatible and is able to inform the system 600 of its augmentary characteristics to those of the ROAR catheter 660 to which it is connected), the controller 700 can operate without receiving any information other than the identity of the catheter 660 and the identity of the intermediate extension line 674 because connection with the ROAR extension line 674 allows the system 600 to detect which particular one of the different ROAR catheters 660 has been connected to the distal end of the ROAR extension line 674 and then to operate ROAR in a predefined manner appropriate for that particular ROAR catheter 660 with the ROAR extension line 674. In the case of RFID or near field communication (NFC), the chip embedded in the ROAR catheter 660 is programmed with the specific valve timings required by that catheter 660. The controller 700 reads these values and functions properly for that catheter 660. This ensures future compatibility with new generation catheters that require different tuning, which tuning would not be known at the time the controller 700 is programmed at the manufacturer. If the catheter to be used is not a ROAR catheter 660, then ROAR should not be used with that catheter because of the high probability of exit flow at the distal end that could uncontrollably eject the clot material. Accordingly, the system 600 automatically prevents use of the ROAR effect when a non-ROAR catheter is connected to the distal end of the ROAR extension line 674 or is connected directly to the system 600 or is connected to the distal end of a non-ROAR extension line 674.

The identification sub-assemblies include various measures present at least at the proximal end of the ROAR catheter 660 (e.g., the inductive sensing system 682, 684 or a 1-wire detection system, such as a DALLAS Semiconductor encryption chip, RFID, Bluetooth low energy (BLE), metallic touch pads, a simple passive design based upon resistors (in series for catheter and extension, to name a few). In the sub-assemblies, there can be two or more electrical contacts. For example, there can be three contacts including power, ground, and a signal using a Hall sensor. In a two-contact configuration, there can be a 2-wire configuration using resistors and mechanical switches. Resistance can be measured between two contacts and, depending on the resistance, a state of the switch can be detected. Power and signal can be combined on one line (plus an additional ground line) to create a "one-wire" connection, for example, using a DALLAS chip mentioned above. An identification sub-assembly also can be present at the distal connection (e.g., a Luer fitting) of the extension line 674 (to contact with the identification sub-assembly at the proximal end of the ROAR catheter 660) and extend back through the extension line 674 to a communication connection with the vacuum source 610, e.g., the proximal manifold connector assembly 670. Therefore, the aspiration thrombectomy system 600 has an ability to sense/detect when a ROAR catheter 660 is connected as differentiated from a standard catheter (i.e., not ROAR). Connection of the ROAR catheter 660 enables use of the ROAR function; connection of a non-ROAR catheter (or, e.g., to a side port of a rotating hemostasis valve (RHV)) disables use of the ROAR function and only normal aspiration is available. Where the identification sub-assembly includes electrical contacts in the ROAR catheter 660, the conductive connection to the vacuum motor 614 can utilize one or more coils of the ROAR catheter 660 as one of these conductors. Alternatively, two or more conductors can be wrapped within the ROAR catheter 660. Alternatively, or additionally, conductors can be bonded on the outside of the ROAR catheter 660.

In addition to the some embodiments where the system 600 already stores the operating parameters for performing aspiration and automatically uses those parameters when the ROAR catheter 660 and/or the extension line 674 is connected or where the ROAR catheter 660 or the extension line 674 provides the operating parameters for performing aspiration, the user can be provided with selectable programs in the controller. These selectable programs can be, in one embodiment, programmed where the controller 700 is manufactured. The user has an instruction manual associating the particular ROAR catheter 660 and/or the extension line 674 being used with a code that loads in the operating parameters, such as pressures, delays, timing. Instead of an instruction manual, these operating parameters can be manually entered by the user instead of through the selectable program(s), for example, by reading the information the instructions for use (IFU) or the packaging of the system 600, or ROAR catheter 660, or extension line 674. In addition, if the user has a desired method of operation (for example, to increase a particular timing), the user can enter the parameter(s) directly through a user interface on the system 600. In other embodiments, a code supplier (e.g., a QR code, a barcode, or an RFID chip) could be on the packaging of one or more of the components and the user presents that code supplier to the controller for reading. In this regard, the system 600 comprises a barcode reader and/or a QR code reader and/or an RFID communication device. With a display on the system 600, in another embodiment, the screen presents parameters to the user and those parameters could be fixed or alterable by the user. In other words, the user could accept or alter the parameters shown. In a particularly inexpensive embodiment, the parameters can be "stored" on a punch card that is supplied with the ROAR catheter 660 or the extension line 674 and the system 600 has a punch card reader. In this embodiment, the user inserts the inexpensive card (e.g., provided with a covering that protects it from liquids present in the operating room) into the card reader and the controller 700 utilizes the parameters on the card or utilizes a code on the card, which code is associated with a set of stored parameters.

All of these embodiments could present the operator with a choice of alternative programs or parameters, or the system 600 could list the parameters that are about to be utilized separately on a display screen and then allow the operator to select those parameters or alter the provided parameters. Similarly, operators are able to store parameters/programs into empty memories within the controller 700. The stored information provided by the catheter, the extension line, the card, the code, etc., could be either ROAR parameters or, alternatively, the information can be characteristics of the catheter and the extension line so that the controller 700 could make compensations to provide a predefined ROAR waveform at catheter tip. In other words, rather than offer up stored ROAR programs, the catheter and the extension line could simply give information to the controller 700 so that the timing and pressures could be modified for each catheter/extension line combination to achieve the predefined ROAR pressure/time profile. By storing either compensation parameters or actual time/pressure parameters, the controller 700 is able to allow future catheters and extensions not yet available. Further, chips, resistors, RFIDs, or BLE could be used to prevent use of the system 600 with catheters not provided by the manufacturer of the system 600, and/or to present a warning or alarm condition to the operator so that they know that the catheter and/or extension is not supported by the system 600.

The proximal manifold connector assembly 670 can comprise the extension line 674 having a system control board 676 with remote controls 678 illustrated in FIG. 56. Some embodiments of the remote control 678 is a mechanical slide switch that turns vacuum on or off based upon a longitudinal position. This can be a two-position switch with a button for ROAR operation. Alternatively, a three-position switch can be provided. In a forward position, the vacuum is off, in a middle or intermediate position the vacuum is on, and in a rear position ROAR operation takes place. When the remote switch is connected, any control buttons on pump are disengaged but the pump can have an "emergency off" switch on the pump that allows the user to turn off the pump if desired regardless of the operation of the remote controls. LEDs can be provided on the remote controls 678 and/or on the body 601. These LEDs can, for example, be: Red = off, Green = Vacuum on, Blinking Green = ROAR, Blinking Red = Error, Blue = vent/purge. In some embodiments, a mechanical redundant pinch valve is present against catheter that, when actuated, pinches closed the lumen of the catheter. In some embodiments, the distal end of the proximal manifold connector assembly 670 that is connected to the ROAR catheter 660 comprises a luer lock part that connects to another luer lock part on the ROAR catheter 660. In various embodiments, the switch is passive (e.g., a simple mechanical switch) or it is an active switch (e.g., capacitive, pressure, magnetic). In such a case, the switch is powered by wires through the extension line 674. In an alternative embodiment, the switch is a separate module that attaches to the extension line 674 and is, for example, battery powered.

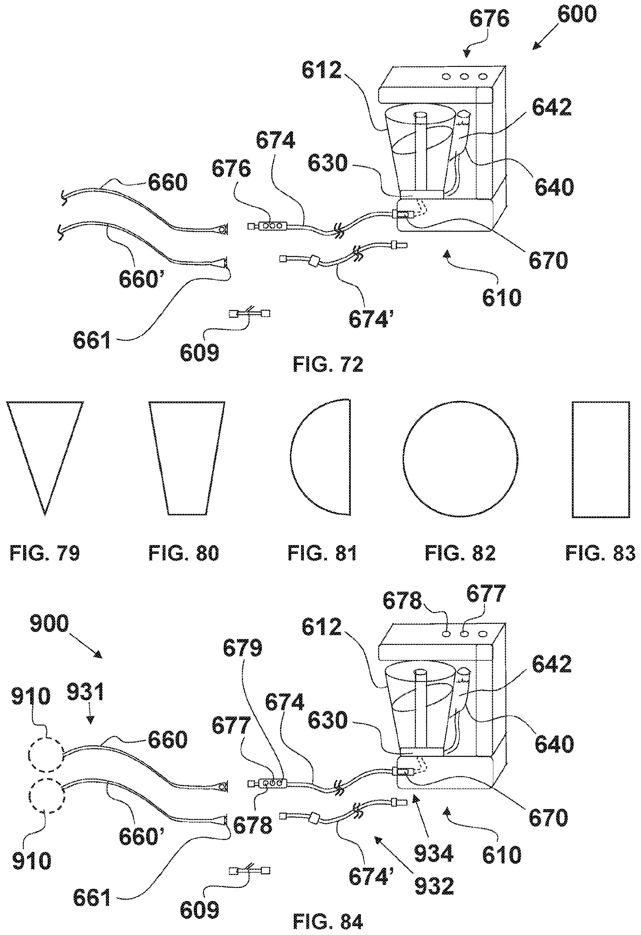

A first benefit of the aspiration thrombectomy system 600 is that, with such a configuration, the same vacuum source 610 can be used with all catheters that previously could be connected to any surgical aspiration devices/vacuum pumps. A second advantage relates to security for enacting the ROAR effect. In such a configuration, users are persuaded to connect the proximal end of the ROAR catheter 660 to the distal end of the proprietary extension line 674. This is beneficial for various reasons. First, ROAR will not work unless the two unique ROAR parts are directly connected and a positive ROAR ID is established. Second, if a standard rotating hemostasis valve is connected between the ROAR catheter 660 and the extension line 674 (for whatever reason that the surgeon/nurse may have), then identification will be negative and ROAR will be disabled. There is a risk that fluid contained within lumens of such rotating hemostasis valves will enter into the ROAR catheter's fluid column and, thereby, introduce air bubbles, which need to be purged entirely from the system for use. An RHV 609 increases the probability of air remaining in the lumens or entering the fluid system. See FIG. 72. Thus, a particularly desirable configuration for the ROAR catheter 660 is a direct connection between the proximal end of the ROAR catheter 660 and a distal end of the proprietary extension line 674. There is also an issue of safety to ensure that the ROAR effect is utilized only with ROAR catheters 660. As described above, each ROAR catheter 660 has a particular set of characteristics related to compliance and, therefore, operation of the vacuum and vent valves 620, 650 is set for that characteristic set. The system 600 is set to react with a particular ROAR configuration based upon the physical characteristics of the ROAR catheter 600 connected, such as length and lumen size. Therefore, the system 660 is tuned/programmed to store a given ROAR setting for each ROAR catheter 660.

However, it is possible that new ROAR catheters 660 and new ROAR extension lines 674 are created after the system 600 or the controller 700 are put into the field. Providing the identity of the ROAR catheter 660 or the ROAR extension line 674 would, therefore, not be sufficient to permit operation of those components properly. Thus, in an additional or alternative embodiment, each of the ROAR catheters 660 and the ROAR extension lines 674 are provided with a memory device (e.g., a DS28E07 EEPROM memory chip) that, when connected to the system 600, provides the controller 700 with the variables necessary for that ROAR catheter 660 and/or that ROAR extension line 674 to operate with the ROAR effect. Example variables that are stored in the memory of each of the ROAR catheters 660 and the ROAR extension lines 674 include, but are not limited to, the frequency of the waveform cycle, a time in the cycle at which the vacuum turns on (Vac-ON time 622), a duration of the vacuum (Vac-ON duration 624), a time in the cycle at which the vacuum turns off (Vac-OFF time 626), a duration that the vacuum is off (Vac-OFF duration 628), a time in the cycle at which the vent turns on (Ven-ON time 652), a duration of the vent (Ven-ON duration 654), a time in the cycle at which the vent turns off (Ven-OFF time 656), and/or a duration that the vent is off (Ven-OFF duration 658). By being able to provide such information to the controller 700, the system 600 can utilize any future ROAR catheter 660 and/or ROAR extension line 674 that might be created for use with the system 600.

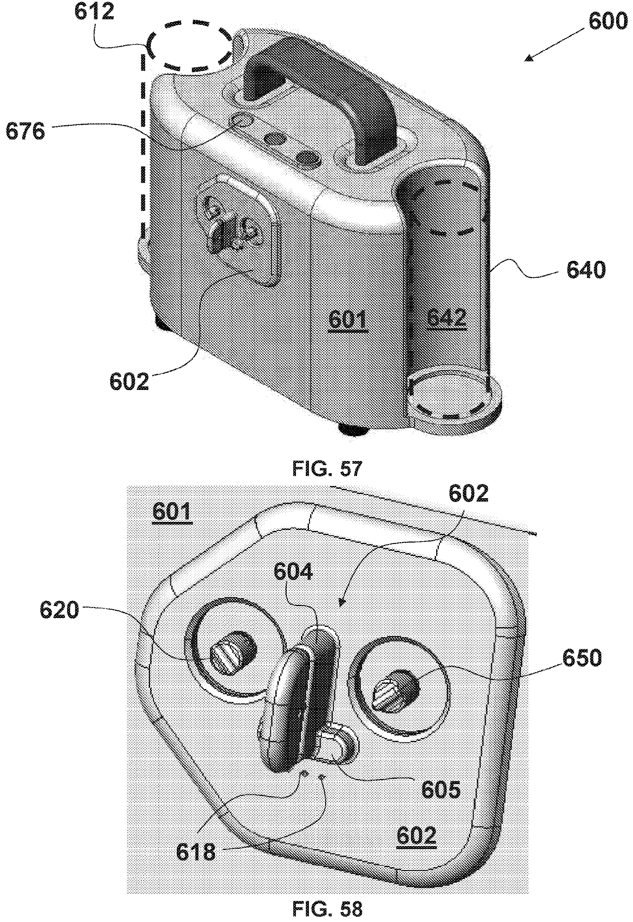

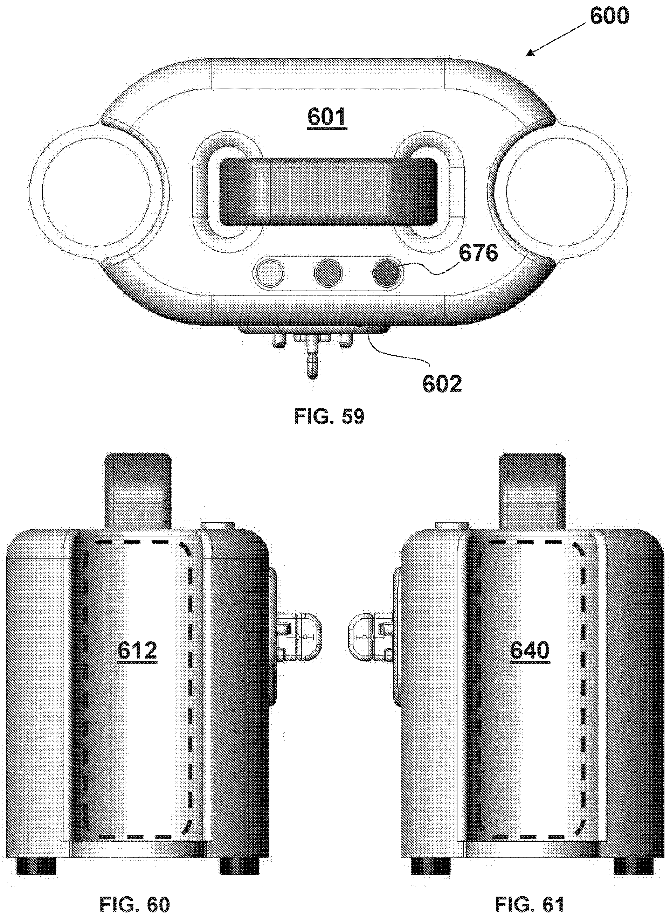

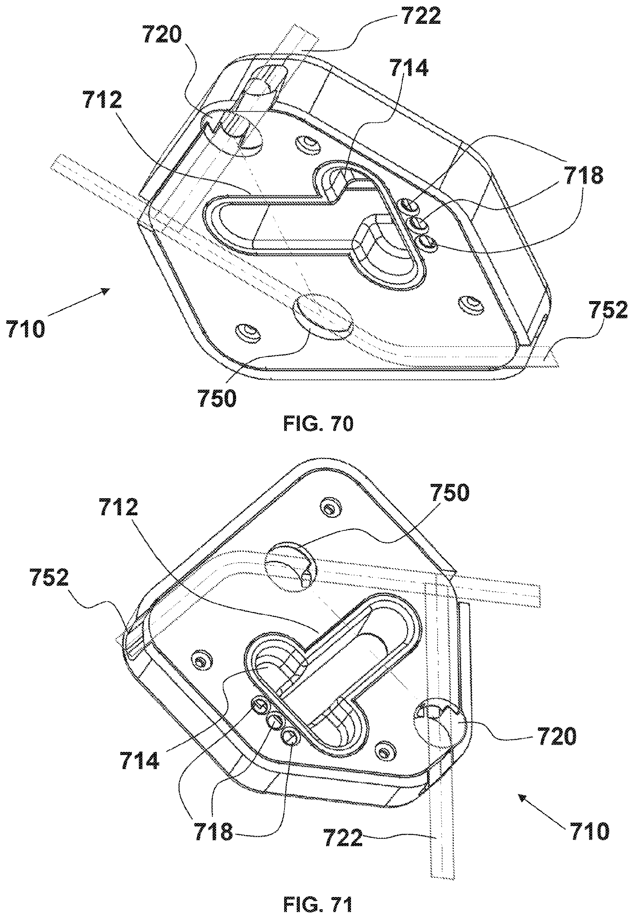

Some embodiments of a self-contained aspiration thrombectomy system 600 is shown in FIGS. 57 to 71. The system 600 has an exterior body 601 containing therein a vacuum motor 614, the controller 700, and controls for the vacuum and vent valves 620, 650 (embodiments of the controls for the valves are shown in FIG. 29 and FIGS. 42 to 46). The vacuum motor 614 is fluidically connected to a collection canister 612 (shown diagrammatically with dashed lines). The body 601 houses a set of system controls 676 (in an alternative embodiment, the controls 676 can be located on/also located on the extension line 674). In this embodiment, there are three buttons: off, purge, and ROAR/Vac. (The purge function will be described in further detail below.) On a side opposite the collection canister 612 is a vent fluid reservoir 640 (shown diagrammatically with dashed lines) containing therein vent liquid 642. As mentioned above, the fluid path of the catheter 660 is to be free from bubbles/air at all times during a surgical procedure.

The body 601 has cassette connection assembly 602 on a front face thereof. The cassette connection assembly 602 protrudes from the front face and has an exterior shape substantially the same as a cassette assembly 710 that will be attached thereto. The vacuum and vent valves 620, 650 protrude from the front face 608 of the cassette connection assembly 602 and, in some embodiments, are centered within respective depressions of the cassette connection assembly 602. In this embodiment, the vacuum and vent valves 620, 650 are pistons that have at a distal-most end thereof a pinching structure. In this embodiment, the pinching structure is substantially in the shape of a standard slot screwdriver. As the vacuum and vent pistons extend out from the depression a given distance (e.g., 8 mm), the slot presses against tubing (in the cassette assembly 710) to close off the lumen within the respective vacuum or vent hose. Closing off the hose acts as a shut-off of the respective valve and releasing away from the hose acts to open the vacuum or vent, respectively. Thus, if the hoses for each of the vacuum and vent lines are placed directly in front of the pistons, the valves 620, 650 will control vacuum and vent according to the ROAR process described herein. (As described below, the cassette assembly 710 positions those hoses in this manner.) In between the valves 620, 650 is a boss 604 protruding from the front face 608 of the cassette connection assembly 602. The boss 604 has an exterior surface with a given shape, e.g., a raceway, and orientation wings 605. At the end of the boss 604 is a rotating lock 606 in the shape of half circle or half oval. The rotating lock 606 has a central pivot to allow it to rotate 90 degrees from the position shown in FIGS. 57 to 67. In the rotated orientation, therefore, the rotating lock 606 defines lower surfaces (opposite the front face 608 of the cassette connection assembly 602) that are perpendicular to the protruding extent of the boss 604. These lower surfaces are set at a distance to define a gap between the lower surface of the rotating lock 606 and the front face 608 of the cassette connection assembly 602. Also present on the front face 608 of the cassette connection assembly 602 is/are conductive connectors 618. The conductive connectors 618 are used to detect when a cassette assembly 710 is present and locked on the cassette connection assembly 602. Detection of the cassette assembly 710 can be made by mechanical measures (such as a pogo pin) or a combination of mechanical and optical and electrical measures.

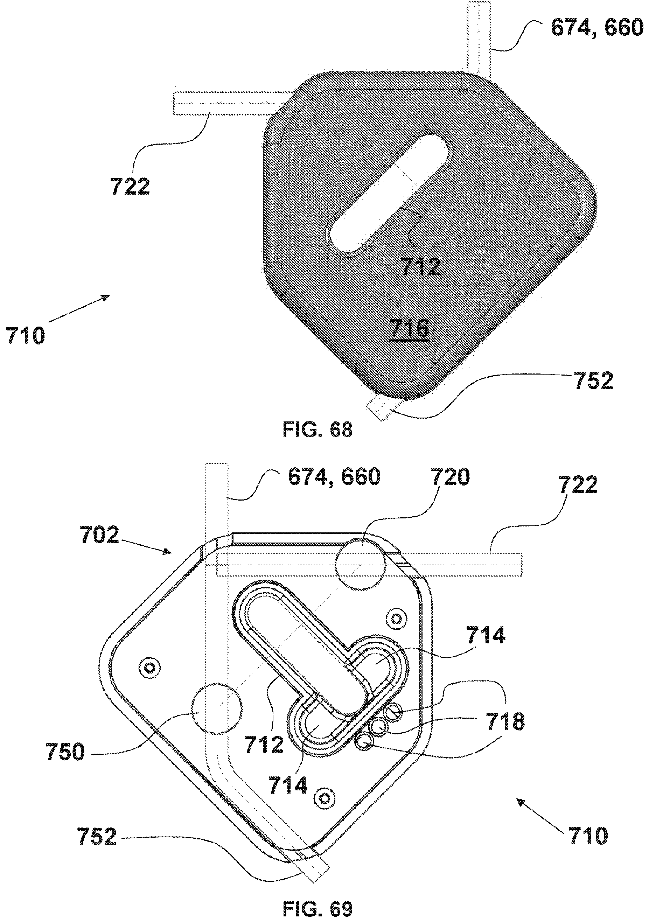

A cassette assembly 710 is removably connected to the cassette connection assembly 602 and some embodiments of this cassette assembly 710 is illustrated in FIGS. 68 to 71. As shown in FIG. 68, the cassette assembly 710 has an interior orifice 712 with a shape corresponding to the given shape of the boss 604. The boss 604 and interior orifice are matched in shape so that the cassette assembly 710 can fit onto the boss 604 and slide down thereon until the rear face of the cassette assembly 710 aligns with and/or touches the front face 608 of the cassette connection assembly 602. The rear face of the cassette assembly 710 is depicted in FIG. 69. In the view of FIG. 69, pockets 714 corresponding in shape to the orientation wings 605 are present in the interior surface of the cassette assembly 710 at the interior orifice 712. In this regard, when the cassette assembly 710 is slid down the boss 604, there is only one orientation in which the cassette assembly 710 can approach the front face 608 in a lower-most position e.g., as in a key within a keyhole. This placement insures that the distal end effectors of the vacuum and vent valves 620, 650 are always aligned with vacuum valve area 720 and the vent valve area 750 within the cassette assembly 710.

When the "T" shape of the boss 604 and wings 605 are matched with the interior T-shape of the orifice 712, three connections are made possible. First, as set forth above, the distal end effectors of the vacuum and vent valves 620, 650 are aligned with vacuum and vent valve areas 720, 750 within the cassette assembly 710. Second, conductive connectors 718 on a rear face of the cassette assembly 710 are aligned with and make contact with respective conductive connectors 618 adjacent the boss 604. These connectors 718, 618 can be, for example, respective pads and pogo pins to insure positive electrical connection when the rotating lock 606 is used to lock the cassette 610 onto the body 611. With appropriate electrical connections, these connectors 718, 618 can inform the controller 700 that the cassette assembly 710 is installed and ready for use and which kind of cassette assembly 710 is installed if it is associated with a particular ROAR catheter 660 and needs identification. Finally, the rotating lock 606 is located above the front face 608 of the cassette connection assembly 602 and the bottom surfaces of the rotating lock 606 are above the outer front face 716 of the cassette assembly 710. A protrusion distance of the boss 604 is configured to place bottom surfaces of the rotating lock 606 (those surfaces facing the front face 608) at a distance approximately equal to the thickness of the cassette assembly 710 such that, with rotation of the lock 606, the bottom surfaces of the lock 606 engage the outer front face 716 of the cassette assembly 710, thereby pressing the cassette assembly 710 firmly in place against the front face 608 to touch the connectors 718, 618 together and locking the cassette assembly 710 to the body 601. The quarter-turn rotating lock 606 secures the cassette assembly 710 on the body 601 and also provides a cam force that holds the cassette assembly 710 thereon, in particular, while the valves 620, 650 actuate against vacuum and vent tubing present within the cassette assembly 710. In some embodiments, a non-illustrated switch is integrated in the rotating lock 606, the switch detecting the quarter-turn and, with the electrical connectors 718, 618, verifying that the system 600 is armed and ready for use.

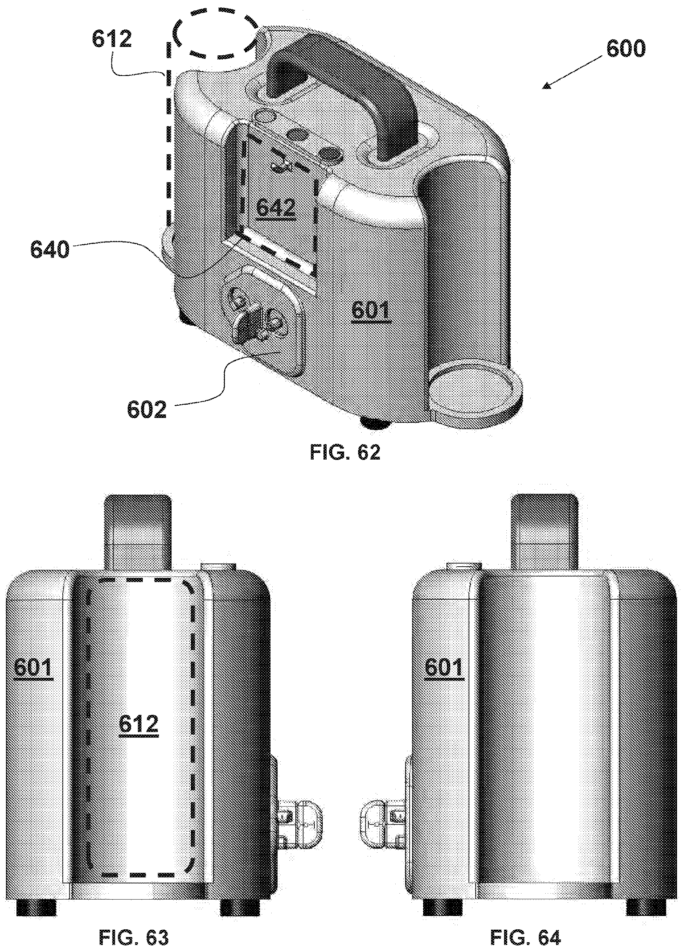

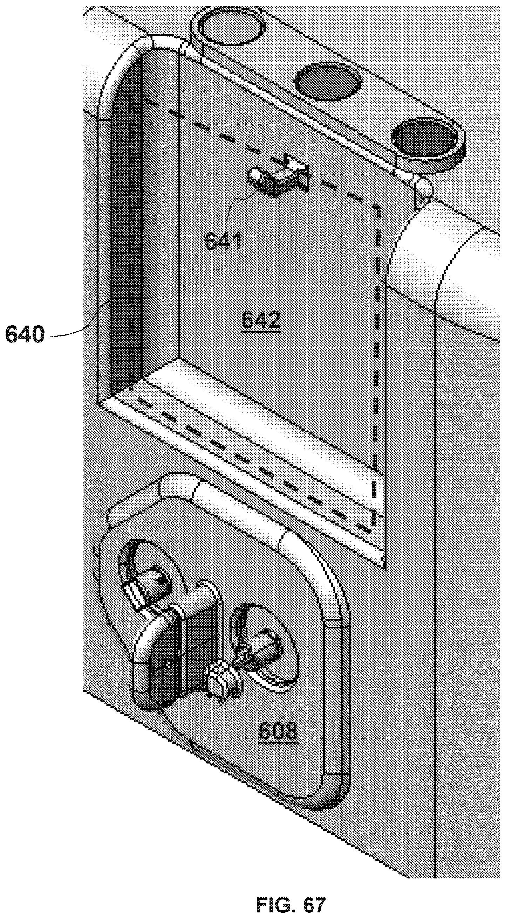

In a particularly efficient configuration, the cassette assembly 710 can be removable, replaceable, and disposable as an entire set including the junction box shown in FIGS. 68 to 71 and a tubing set. The cassette assembly 710 has as set of relatively short whips of tubing including a first tubing whip 722 fluidically connected to the collection canister 612 of the vacuum source 610 and a second tubing whip 752 fluidically connected to an intake of the vent valve 650, and an extension whip that can be the extension line 674 or it can be a short tubing to be connected directly to the catheter 660. Once connected, this efficient configuration allows the system of lumens to be automatically cleared of air/bubbles. By locating the vent liquid above all of the lumens (such as with the bag 640 in FIG. 67), the catheter 660, and the collection canister, opening the output of the vent fluid reservoir 640 will fill all interior lumens and clear the system of any air/bubbles before use. If desired, a cam lock can be mechanically connected to the vacuum motor 614 (either temporarily or fixed) and the motor 614 can be operated to actively draw all air into the collection canister and thereby purge the system 600. As an alternative to the front-loaded configuration of the cassette assembly 710, the cassette assembly 710 can be connected or molded as a part of a bottom of the disposable collection canister 612. In this configuration, two disposable parts can be provided together in a sterile packaging and disposed of in one piece. With a vent fluid reservoir that is either a hard container (FIGS. 57 to 61) or a bag (FIGS. 62 to 67), the vent liquid 642 can be part of the cassette 700 with all lumens pre-filled with saline and part of a single disposable package. The vent fluid reservoir 640 and the collection canister 612 can either or both be part of a disposable cassette 700 system. All of the disposable parts used in a catheter procedure can be integrated together in one disposable package.

As explained previously, it is important for the system to be purged of air to achieve the ROAR effect. Purging can be achieved in several ways. The two main methods used to purge the system are forced purged and dribble purge. The forced purge method involves submerging the tip of the extension line 674 into sterile fluid such as saline and while submerged activating the "purge" function. The controller 700 will then alternatively open one or both of the control valves for a predetermined time and sequence to pull the sterile fluid through the extension line 674 and valves and displace all the air that may have been in the system. Once this purge process is complete, both valves will close and the extension line 674 with a full fluid column can be connected to the catheter, which has also been de-aired and ROAR applied. In comparison, the dribble method relies on a small positive pressure (created by gravity, squeezing the fluid bag, pressurizing the vent fluid tank, or a peristaltic pump or any similar measures) to allow the vent fluid to dribble through the lumens and, thus, flood them. In this embodiment of the dribble purge system, the vent fluid source 640 is higher than the exit of the extension line 674 and the vent liquid path does not contain any air traps.

For the dribble method, the purge cycle is initiated by pressing the purge button and, in some embodiments, is performed by the controller 700. With the vacuum valve 620 closed, the vacuum source 610 is turned on and the vacuum vessel is pumped down to a desired vacuum level. The vent valve 650 is opened and vent liquid 642 is allowed to flood the vent line and the extension line 674. To ensure that all air is removed from the manifold 630, the vacuum valve 620 is opened momentarily while the vent valve 650 is also open. This allows the vent liquid 642 to be drawn from the vent fluid source 640 and from the extension line 674, and through the vacuum manifold passageways thus purging them of air. The vent valve 650 is left open for a period of time after the vacuum valve 620 closes to ensure that the quantity of fluid that the vacuum cycle removed from the extension line 674 is replenished. This cycle of vent liquid flow and momentary vacuum can be repeated several times to ensure complete purging. The purge pump can be a peristaltic pump, a pressurized cuff around a flexible vent liquid container (such as an IV bag), and/or a vent fluid canister pressurized by using the exhaust from the vacuum source 610.

The presence of bubbles in the fluid system adversely affects the water-hammer effect. Accordingly, the system 600 facilitates or automatically purges air from the fluid lumens. In some embodiments, bubble sensors (either optical, ultrasonic, or fluid-pressure-profile based) are incorporated into the system 600 to facilitate this purging or to automatically engage a purging function (e.g., under operator control to prevent purging when the catheter 660 is present in the bloodstream). There are various measures for bubble detection. For example, an optical sensor could be placed in the cassette assembly 710 to sense the presence of bubbles. With a sensor coupled to the vacuum source 610, a slow rise in pressure can be sensed to prevent using the incorrect catheter or extension. The specific compliance of a catheter 660 or an extension line 674 is among the parameters used to program or compensate the system 600. A user-feedback indication informs the user when the system has been sufficiently purged. In some embodiments, the compliance of the catheter 660 and the extension line 674 are controlled to be below some optimum range. Also, pressure-rise information is used to modify the ROAR settings, for example, to detect corking and provide an optimum pressure profile for that condition.

The configurations of the aspiration thrombectomy system 600 described and shown provide various significant benefits. Before describing these additional benefits, reference is made to FIG. 72, which illustrates one embodiment of the aspiration thrombectomy system 600 with extension lines 674, and catheters 660. The aspiration thrombectomy system 600 comprises the vacuum source 610 with the collection canister 612, the vent liquid reservoir 640 with the vent liquid 642, the manifold 630, and the proximal manifold connector assembly 670. Removably connected to the proximal manifold connector assembly 670 is a ROAR extension line 674. Next to the ROAR extension line 674 is an off-the-shelf extension line 674' usable both with the system 600 by connecting through the proximal manifold connector assembly 670 and with conventional surgical vacuum sources. The ROAR extension line 674 comprises the system controls 676, which are also shown on a top surface of a frame of the system 600. Also shown is a ROAR catheter 660 and an off-the-shelf aspiration catheter 660'. With proximal Luer connectors, both catheters 660, 660' can be used with either extension line 674, 674'.

There are several topologies for the disposable, reusable, and limited-reuse components of the aspiration thrombectomy system 600 as described and shown herein. The vacuum source 610 can be a limited-reuse component that plugs into a reusable electronics/power-supply system, such as the frame in FIG. 72. The valve-element cassette assembly 710 includes pinch-tubes and, therefore, it is a single-use only component. Alternatively, the valving components can be reusable, for example, where the valve actuator is separate, either in a separate semi-reusable module, or part of the pump/control system. The different kinds of valves (e.g., rotary, trumpet) that have different ways to separate the disposable/reusable portions of the system 600. The valve actuators can be part of a reusable portion or part of a limited-reuse portion (e.g., along with a pump module). Alternatively, the valve-actuators can be a second limited-reuse module. The cassette assembly 710 with the valve elements can include a diaphragm or piston that is actuated by a mechanical actuator in the reusable part of the system 600. With such modularity, the architecture of the system 600 becomes adaptable to use with any vacuum source, even a household vacuum system (which could include a vacuum pressure regulator to ensure uniformity of the system 600. The power source for the system 600 may be a rechargeable battery or a replaceable module attached to the system 600, in which case the latter does not require sterility. Alternatively, the power source is a primary battery included as part of the disposable components, which could include disposable pumping elements.

The various configurations permit multiple product topologies specifically targeted at different use cases. For example, one topology is a minimum-recurring-cost system with only the tubing set being disposable. Alternatively, another topology is a system requiring minimum capital cost and incorporating modules whose costs are easily amortized for each surgical case.

Various additional safety measures can be added to the system 600. For example, a liquid level detector can be provided at or with the vent fluid reservoir 640 to confirm that vent liquid 642 is within the tank or pouch, to indicate a warning to the user when the level of vent liquid is low, and to prevent operation of the system 600 if the vent liquid about to run out or is empty. In the configurations with the cassette assembly 710, the system 600 will not start unless the cassette assembly 710 is in place and is correctly installed. The system 600 can have a purging function that is used to fill the various lumens of the catheter 660, the extension line 674, and any tubing connecting the vent fluid reservoir 640 and the collection canister 612 before use. It is noted that the system 600 should not be operated if air is present anywhere in the lumens. Thus, the controller 700 can operate the system to draw in vent liquid 642 and fill the various lumens in a pre-use setup phase. This could include having the vacuum motor 614 operate in reverse to apply positive pressure for purging the various lumens. Alternatively, the controller 700 could actuate a peristaltic pump to purge vent fluid through the lumens. The controller 700 can be programmed, during operation of the system 600, to detect peaks of use during ROAR. If these peaks are not sharp, then a conclusion that air is present in the system can be determined. Bubble detectors (i.e., ultrasonic) can be added to the system such that they straddle the tubing in the cassette and provide feedback to the controller to ensure that the tubing has been properly purged. With such a conclusion, the controller 700 can be programmed to cease operation and start an auto-purge routine to flush the various lumens with an external liquid source or from the vent fluid reservoir 640.

It is noted that the mechanism of action to aspirate a clot is interaction between the tip of the catheter and the clot. In a prior art static aspiration catheter, this happens only once - at the time of clot corking. The rate at which vacuum is applied to have this one mechanism of action at the catheter tip in static prior art systems is governed by four characteristics:
1) how quickly one can open the manual valve;
2) how quickly one can pull the plunger on a syringe;
3) how long it takes for the vacuum pump to come up to pressure; and
4) what *fluid* is between the vacuum source and the catheter tip.
Thus, prior art systems are not efficient in terms of the mechanism of action that breaks up the clot. In contrast, the repetitive cycle of the aspiration systems, devices, and methods described herein causes interaction between the tip of the catheter and the clot to occur many times and, in particular, many times a second (e.g., between approximately 1 Hz and approximately 25 Hz). This change in pressure not only occurs over a short time in the herein-described embodiments (ΔP/ΔT), but it also occurs many times per second. Thus, ROAR aspiration has a significant advantage over the prior art because it can increase pressure changes (via double-off times 625, 627 and Ven-ON duration 654; FIG. 53) and can decrease the time of each pressure change and, significantly, can repeat it periodically (Hz).

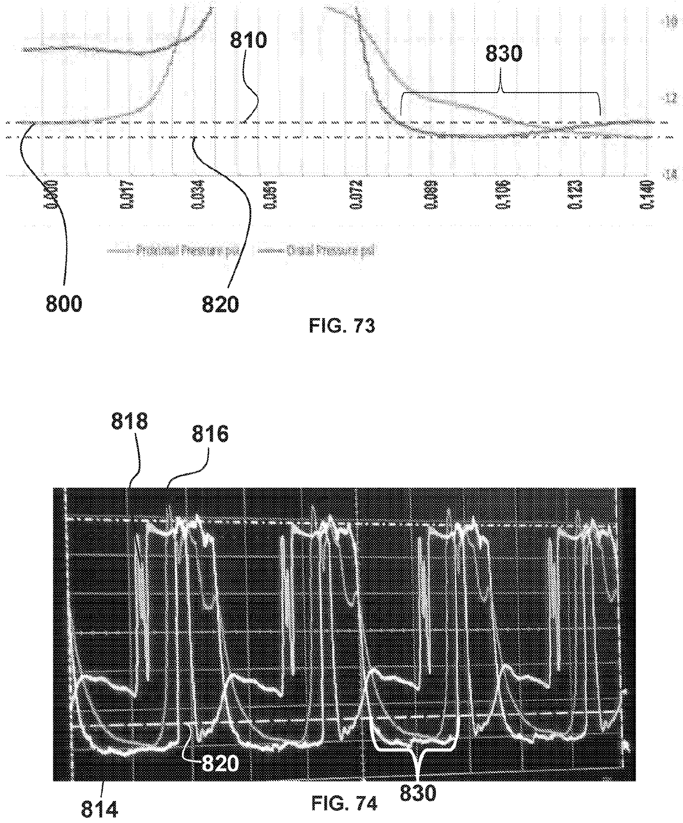

Controlling the vacuum level at the distal end of the catheter according to the exemplary embodiments described herein allows the observation of a unique aspect and behavior of the liquid column and how it can draw a clot into the lumen of the catheter. With reference to the lower left bottom of FIGS. 50 and 51, the steady state maximum level of vacuum 800 originating from the vacuum source 610 (proximal pressure) is somewhere between -12 and -13 psi. The bottom of FIG. 50 is enlarged in the view of FIG. 73 and a dashed horizontal line 810 is placed at this maximum level of vacuum 800 produced by the vacuum source 610. As can be seen in FIGS. 50 and 51, the distal pressure change of the fluid column follows the proximal pressure change by a given time (which is dependent upon a number of characteristics of the liquid in the catheter including, e.g., viscosity, density, compressibility, as well as characteristics of the catheter itself, including, e.g., bulk modulus, tensile strength, compressibility, durometer of construction materials, hoop/radial strength). What should be apparent and/or expected is that the highest level of vacuum in the lumen 662 at the distal end 664 of the catheter 660 should be no greater than the highest level of vacuum at the proximal end 666 of the catheter 660, i.e., the steady state maximum level of vacuum 800. This, however, is not the case. Instead, the maximum level of vacuum experienced in the lumen 662 at the distal end 664 of the fluid column is greater than the steady state maximum level of vacuum 800 originating from the vacuum source 610 and being applied at the proximal end 666 of the fluid column. This improved result is depicted by the dot-dashed line 820 in FIG. 73 and is approximately 0.5 psi lower than the steady state maximum level of vacuum 800. Simply put, the pulsed aspiration devices, systems, and methods described and shown herein are capable of delivering a vacuum level to the distal end 664 of the catheter 660 that is greater than the actual maximum level of vacuum that can be supplied by the vacuum source 610 to the proximal end 666 of the catheter 660, referred to herein as a "super-vacuum." The super-vacuum at the tip of the catheter 660 occurs for a given period of time 830, explained in further detail below.

The graph of FIG. 73 illustrates the relative pressures experienced at the proximal end 666 of the catheter 660 and the distal end 664 of the catheter 660 for one instance of the pulse shown in FIG. 48, for example; in this example, the vacuum valve 620 is open, then closed, and, in parallel, the vent valve 650 is closed, then opened, and then closed. The graph in FIG. 74, in comparison, depicts the system operating with a pulse that is periodically repeated at a given rate (e.g., between 1 Hz and 25 Hz, between 6 Hz and 16 Hz, between 8 Hz and 12 Hz) and shows a set of characteristic pressure traces of various places in the system when first contacting a clot at the distal end 664 of the catheter 660. There are three pressure transducers on the system, each outputting an individual pressure trace and all three are depicted in FIG. 74. The yellow line 814 is the pressure trace of the pressure transducer at the catheter tip (e.g., distal end 664), the red line 816 is the pressure trace of the pressure transducer at the catheter hub 661 (see FIG. 72), and the teal line 818 is the pressure trace of the pressure transducer at a proximal end of an extension line 674 connecting the catheter 660 to the vacuum source 610 (see, e.g., FIGS. 56 and 72). The horizontal dashed line 820 in FIG. 74 indicates the steady state maximum level of vacuum from vacuum source. The upper dot-dashed line indicates atmospheric pressure. As can be seen, the super-vacuum at the catheter tip 664 exists for a given period of time 830 during each period of the pulse. More particularly, the yellow line (tip pressure) is able to go to a level of vacuum substantially below the steady state maximum level of vacuum 812 from the vacuum source 610. The yellow trace also shows that a low vacuum level is maintained and an overall pressure curve occurs at the tip with a greater percentage of time at vacuum. Having the transitions of vacuum be as short as possible is most desirable. Knowing the various time instances between each part of the period (the vacuum on/off and the vent on/off) allows the controller to set these time instances to make the vacuum transitions as short as possible. A rapid transition from no vacuum to full vacuum allows the system to be improved over the prior art. By having vacuum applied very quickly (open suddenly, e.g., less than approximately 10 ms, in particular, less than approximately 5 ms), the column of fluid in the extension line 674 gets drawn towards the vacuum source 610 and, because the liquid column has mass and is incompressible, the column develops momentum towards the vacuum source 610. That momentum makes the liquid column behave like a plunger of a syringe, therefore creating a deeper, momentary vacuum at the distal end 664 of the catheter 660.

The super-vacuum is even able to be witnessed by a user of the system; specifically, a user is able to see a vacuum bubble form at the distal tip of the catheter. The vacuum bubble lasts as long as the period 830 (see FIG. 74) and (when the catheter is not opaque) the user can visibly see the bubble grow proximally and then retreat distally, as the lower pressure at the catheter tip 664 draws the liquid column back towards the tip as the bubble collapses.

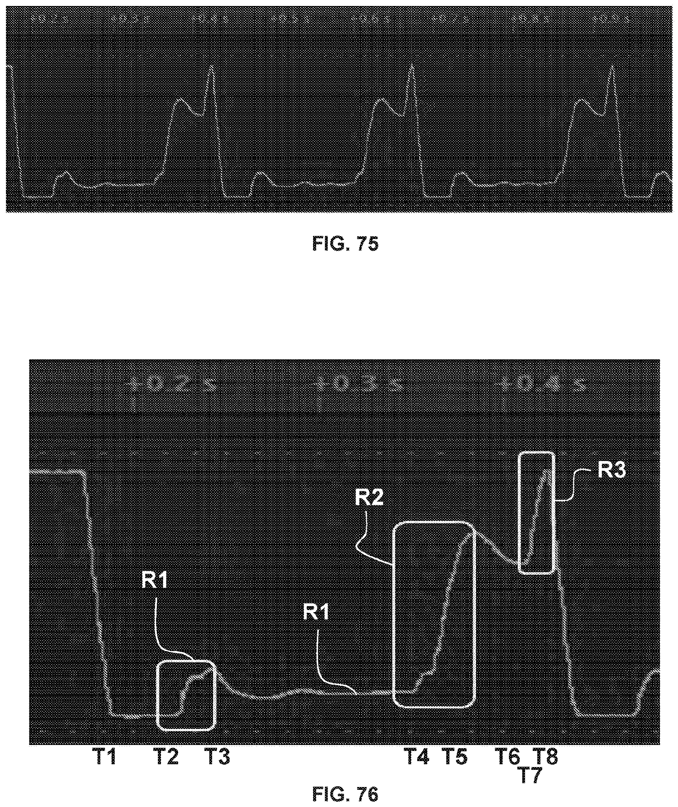

The graphs of FIGS. 75 to 78 illustrate the pressure level at the distal end 664 of the catheter 660 with different timings for various time instances/sections of the pulse (see, e.g., times 1 through 9 in FIGS. 48 and 49). One pulse of the waveform of FIG. 75 is shown enlarged in FIG. 76. As set forth above, the pulse in FIGS. 48 and 49 includes, at T1, the vacuum valve 620 in the open/on position and the vent valve 650 in the closed/off position. This position T1 is shown on FIG. 76 immediately after the vacuum valve 620 is opened (while a clot is corked at the distal end 664 of the catheter 660). At that time, the vacuum source 610 imparts a force against the liquid column, drawing the liquid column proximally and creating the super-vacuum at the distal end 664. At approximately T2, the vacuum bubble of the super-vacuum starts to collapse, changing the pressure towards the steady state maximum level of vacuum 800. The collapsing vacuum causes a rising pressure slope from T2 to approximately T3. The trace R1 in FIG. 76 shows the collapse of the super-vacuum. The rebound, in this exemplary embodiment, follows an exponentially decaying sinusoid function. Without changing any valves, the vacuum will stabilize at the steady state maximum level of vacuum 800, which occurs between T3 and T4. At T4, the vacuum valve 620 starts transitioning to the closed/off position which can be seen as the small bump at T4. At approximately T4 (after the double-off/double-closed), the vent valve 650 is opened, thereby allowing the vent fluid to enter the catheter lumen. No later than approximately T5, the vent valve 650 is fully open. Opening of the vent valve 650 causes an in-rush of fluid and an associate increase in pressure (upwards in FIG. 76). This time instance/period is shown as R2. While the vacuum valve 620 remains closed, the vent valve 650 remains open until approximately T6, at which time the vent valve 650 starts to close, and the vent valve 650 is closed at approximately T7. This time instance/period is shown in the trace R3 of FIG. 76 by a rise in pressure from T6 through T7 because closing of the vent valve 650 pushes a volume of fluid into the extension line that is connected to catheter lumen 662. At time T8, the vacuum valve 620 is reopened while the vent valve 650 remains closed.

In some embodiments, the vent valve 650 is a pinch valve (comprised of a silicone tube and an actuator moving orthogonally to pinch off the lumen of the tube). The actuator that pinches the tube can be narrowed to a short length or widened to a long length and it can have a selected width. Examples of the actuator are shown in FIG. 58 (see reference numerals 620, 650). Suitable cross-sectional shapes of the pinching part of the actuator can include geometric shapes (e.g., a wedge, a trapezoid, a hemisphere, a sphere, a rectangle) and combinations or portions of these or any other geometric shape (see, e.g., FIGS. 79 to 83) or more complex mechanisms like the mechanisms 60 and 66 of FIGS. 5, 6, and 8 to 10. The width and the shape of the actuator can be selected to displace a controlled amount of fluid dependent upon the characteristics of that shape (see, e.g., FIGS. 5, 6, 8 to 12, 15, and 79 to 83), as well as selecting the timing and duration of the actuator displacement. The displaced fluid is free to travel in both directions of the tube until the actuator causes the two opposing sides of the tube's lumen to contact (thereby closing off the respective valve 620, 650). Moving the actuator further distant, continues to displace fluid within the lumen on both sides of the valve at least until the actuator can no longer move or until the shape of the actuator no longer causes a change in shape or length of the tube's lumen. In addition to motion of the actuator, physical characteristics of the actuators themselves can affect this displacement of fluid. For example, the actuator material can be a conformable material (e.g., springy) that changes from narrow to wide based on how much force is applied; an exemplary material for this characteristic is silicone. Another way to change the shape of the actuator is to provide a multipart actuator column that is mechanically adjusted, for example, using pneumatic actuator parts, electromechanical actuator parts, and/or actuator parts made from micro-electromechanical systems (MEMS). Compliance of the tube permits this bidirectional fluid displacement. In particular, displacement of fluid on the distal side of the vent valve 650 causes the R3 rise shown in FIG. 76. The vacuum valve 620 starts to open at approximately T8, at which time the level of pressure at the distal end 664 of the catheter 660 starts to decrease. This process from T1 to T8 is repeated periodically.

FIGS. 75 and 76, therefore, show that the pressure rise associated with corked flow is the result of four cascading events, not necessarily in order. One event is the closing of the vacuum valve, during which fluid produces a small distal displacement of the liquid column (e.g., a push) towards the distal end 664 of the catheter 660. Another event is the collapse of the super-vacuum, which collapse can occur before the vacuum valve closes but this can be tuned to occur during or after the vacuum valve closes. A third event is the opening of the vent valve, in which there is a fluid in-rush into the lumen of the catheter 660. A fourth event is the closing of the vent valve, during which fluid produces a small distal displacement of the liquid column (e.g., a push) towards the distal end 664 of the catheter 660. Finally, the liquid is drawn proximally by the opening of the vacuum valve 620. Any one of these four events can change pressure at the distal end 664 of the catheter 660. The mechanisms that cause positive pressure to produce an exit flow are dependent upon timing of the valves 720, 750. When there is a fluid in-rush, the compliance of the catheter surrounding the aspiration lumen under vacuum is released to increase the volume of that lumen and draw fluid therein; when a vacuum is drawn in the aspiration lumen, the catheter is pre-loaded to refill that volume when the vent valve opens. Also, when fluid rushes into the aspiration lumen, this fluid has momentum. If that momentum is allowed to go uncontrolled, it will likely drive the fluid column out the distal end to the extent that the clot material would be ejected such that the reapplication of vacuum could not recapture the clot material. The phenomenon of water hammer can also cause exit flow in an open system. If flow suddenly stops, a pressure wave is created that carries through to the distal tip of catheter. If that wave is allowed to arrive at the distal tip, exit flow occurs.

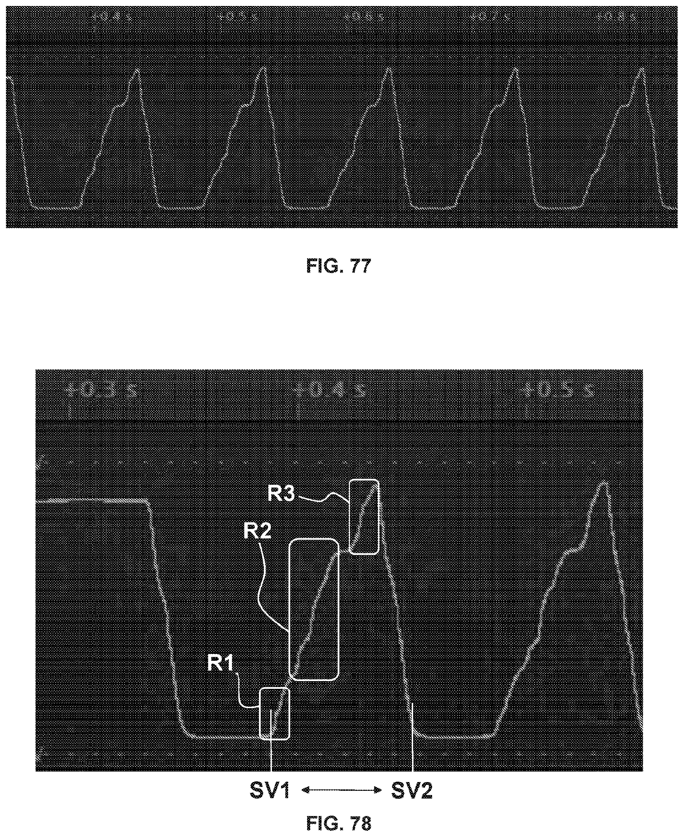

It was discovered that the above events can be timed with the collapsing localized super-vacuum at the catheter tip and that coordinating all four of these events optimizes the pressure spike at the distal end 664 of the catheter 660. Therefore, with pre-set timing (i.e., tuning), these optimizations can be added to each other and maximize the pressure spike at each repetition of the cycle and also to make the time that the system is not at vacuum as short as possible. Such optimization occurs when the rises of R1, R2, and R3 are aligned - which can be accomplished by tuning the timing of the vent and vacuum valves 650, 620 (see FIG. 52); an exemplary embodiment of such an aligned efficiency trace with tuned timing is depicted in FIGS. 77 and 78, where R1, R2, and R3 form an approximately straight line and, therefore, a time T between an endpoint of the first super-vacuum SV1 and a starting point of the next super-vacuum SV2 is minimized (T = min [SV2-SV1]); in other words, the system is at vacuum in as long a time as possible. More specifically, alignment of R1, R2, and R3 is made possible by adjusting the vent-on and vent-off times 652, 656 (which define the vent-on duration 654 and the vent-off duration 658) and how these times are aligned with the vacuum-on and vacuum-off times 622, 626 (which define the vacuum-on duration 624 and the vacuum-off duration 628). With such adjustment by the controller (e.g., 500, 700), a control pattern of the vacuum source is able to change a vacuum level at the distal end 664 of the catheter 660 to a level that is greater than the steady state maximum level of vacuum 800 from the vacuum source 610 and to relieve and reapply the vacuum in a shortest time possible.

As set forth herein, to obtain the most efficient clot-removing result, it is important to have all air purged from the system prior to implementing ROAR operation. There are a number of user errors that induce air to enter into the system. It is desirable, therefore, to minimize or entirely avoid each potential user error. There are two different paths to achieving a complete purged result with the catheter system. First, the surgeon can be taught to avoid air-inducing actions and, thereby, minimize entry of air into the system. Second, the system can check the fluid-filled lumen of the catheter and determine, before or concurrent with application of ROAR, whether or not air is present in the lumen. Each are explained in turn.

Referring to FIG. 69, the lumen of the system includes the catheter, the extension tube, the vacuum line, and the vent line. A three-way junction or fitting or a T-junction or a T-fitting is present at the cassette assembly 710 at areas 720, 750 where the vacuum and vent valves 620, 650 interact. More particularly, the first and second tubing whips 722 and 752 pass respectively along the vacuum and vent valves 620, 650 and meet at a downstream junction 702 (see, e.g., FIG. 69) at which they connect fluidically and extend into the extension line 674, which then connects to the catheter 660. Where the first and second tubing whips 722, 752 and extension line 674 are not integrated, a non-illustrated T-fitting can connect the three lines together. In such an exemplary embodiment, the cassette assembly 710 can be formed with a non-illustrated pocket to accommodate and hold therein the T-fitting, for example, in a snap-lock manner. In this embodiment, the first and second tubing whips 722, 752 are respectively connected at the proximal ends to the vent fluid reservoir 640 and to the vacuum source 610 and the distal ends of the first and second tubing whips 722, 752 are connected to respective inputs of the T-fitting. The proximal end of the extension line 674 is connected to an output of the T-fitting.

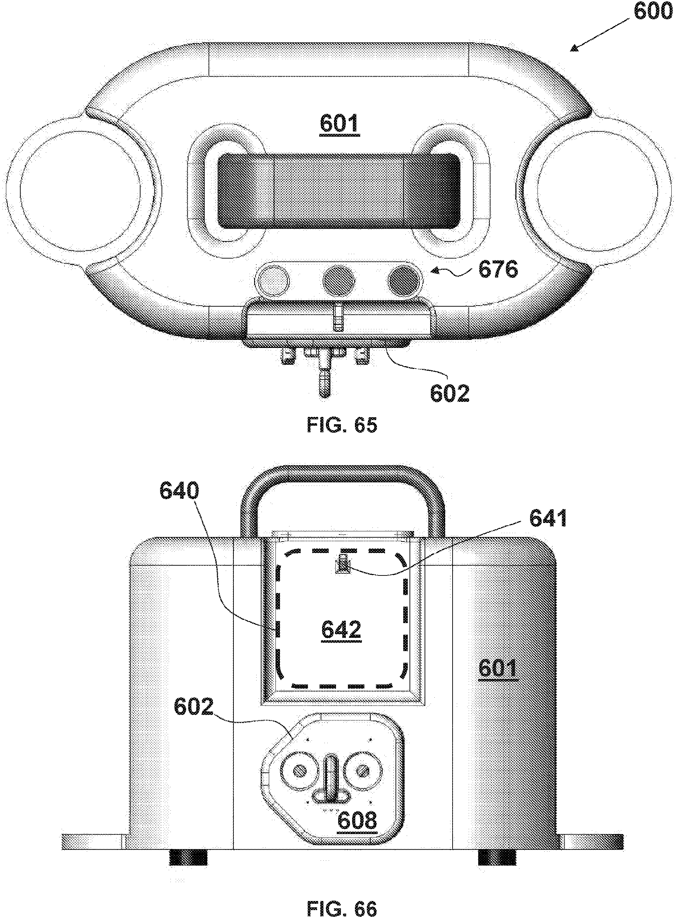

To purge these lumens, the distal end of the extension line 674 can be placed in a purge liquid and the purge action of the system is started (for example, the purge function can be carried out by pressing the blue (right) button in FIG. 65). In this function, the vacuum source 610 is turned on while one or both of the vacuum and vent valves 620, 650 are open, thereby filling the extension line 674 and the first and second tubing whips 722, 752 with the purge fluid. After the user visually sees removal of all bubbles from the lines, the user can actuate the purge control again, thereby turning off the purge function. Alternatively, knowing the size of the tubing and how long the purge action of the system must remain on to remove all bubbles from the lines, the system can be programmed to automatically shut off the purge function after a pre-set time. This sub-process fills the lines 674, 722, 752 with liquid that is free from air.

These lumens can be purged in another way in which an elevated or pressurized liquid source is fluidically connected to the vent line and the purge action of the system is started (for example, the purge function can be carried out by pressing the blue (right) button in FIG. 65). In this function, the vacuum source 610 may be turned on while the vacuum and/or vent valves 620, 650 are open, thereby filling the extension line 674 and the first and second tubing whips 722, 752 with the purge fluid. In one mode of operation, the vent valve 650 is open and the vacuum valve 620 can remain closed. In another mode of operation, the vent valve 650 is open and the vacuum valve 620 can be modulated and/or left partially open. After the user visually sees removal of all bubbles from the lines, the user can actuate the purge control again, thereby turning off the purge function. Alternatively, knowing the size of the tubing and how long the purge action of the system must remain on to remove all bubbles from the lines, the system can be programmed to automatically shut off the purge function after a pre-set time. This sub-process fills the lines 674, 722, 752 with liquid that is free from air.

Still in another embodiment, the lines 674, 722, 752 are translucent or clear so that a user can see bubbles moving through and out of the lines 674, 722, 752 into the collection canister 612. In either of the above functions, the vacuum and vent valves 620, 650 may be opened and closed variably to agitate, dislodge, and/or free bubbles from within the lumen. In yet another way to eliminate bubbles from these lumens, the tube set is packaged prefilled with liquid. The only remaining portion of these lines that is susceptible to entry of air is at the distal end of the extension line 674.

Under a procedure (either a test or surgery), a liquid flush drip is fluidically connected to a rotating hemostasis valve (RHV) fluidically connected to the proximal end of the catheter 660 and continually flushes the entire fluid column of the catheter 660 in the distal direction, visible by a slow drip out of the distal end 664 of the catheter 660. The catheter 660 may also be manually filled with liquid, for example, with a syringe. Prior to connecting the catheter to the extension line, the surgeon disconnects the RHV from the proximal end of the catheter. When the surgeon connects the proximal end of the catheter 660 to the distal end of the extension line 674, a meniscus-to-meniscus touching technique is used to prevent trapping of an air bubble therebetween. With this air-free system, a continuous liquid column is achieved within the lumen(s) of the catheter 664 and extension line 674, 674' and T-fitting, allowing for maximum ROAR performance.

Clots lodged in distal anatomy can be accessed when a guidewire and/or microcatheter is inserted within the lumen of the catheter to help guide the aspiration catheter through tortuosity. In an exemplary embodiment, an RHV is fluidically connected to the proximal end of the catheter 660. The Tuohy-Borst valve of the RHV allows the surgeon to insert the guidewire/microcatheter through the catheter 660 to allow the surgeon to navigate to the clot site with the guidewire/microcatheter and then slide the catheter 660 distally towards and over the distal end of the guidewire/microcatheter to place the distal end 664 of the catheter 660 at the clot face. At this point in the procedure, the catheter 660 is adjacent or contacting or pushed into the clot but the guidewire/microcatheter is still taking up significant volume of the catheter lumen. Even with an active flush drip fluidically connected to the RHV, if the guidewire/microcatheter is removed too rapidly, the lumen volume that was just taken up by the guidewire/microcatheter will be filled with air when the removal rate of the guidewire/microcatheter exceeds the fill rate of the flush drip line. There are three locations from which this volume can be filled, either from the flush line drip source (most desirable), from the distal end 664 of the catheter 660 adjacent the clot (less desirable), or at the site of the Touhy-Borst valve where the guidewire/microcatheter is being removed (not desirable because air is the fluid replacing the missing volume). In most instances, a surgeon prefers to keep the distal tip of the catheter 660 at the clot or pushed into the clot once that tip has been guided successfully to the treatment location. Therefore, the distal opening of the lumen can be considered to be corked when the guidewire/microcatheter is being removed. The fluid rate replacing the volume that is taken up by the guidewire/microcatheter when the guidewire/microcatheter is removed is dependent upon the speed at which the surgeon removed the guidewire/microcatheter. In other words, if the surgeon removes the guidewire/microcatheter sufficiently slowly, then the flush line drip rate will fill in the volume of the lumen previously taken up by the guidewire/microcatheter without drawing in any air to form bubbles within the fluid column of the catheter lumen. However, if the surgeon removes the guidewire/microcatheter too fast from the lumen, the purge drip source will not be able to fill the volume fast enough and, therefore, the Touhy-Borst valve surrounding the guidewire/microcatheter will be the source of fluid, i.e., air, that makes up for the volume deficiency, which leads to entry of air bubbles in the catheter lumen.

Keeping FIG. 84 in view, a bubble test configuration 900 of the aspiration thrombectomy system 600 (the bubble test configuration 900 can be a separate testbed or can be an integral part of the system 600). The bubble test configuration 900 includes the ROAR system, a vasculature model with at least one pressure transducer, and test catheter having at least one of a guidewire and a microcatheter threaded there in, the catheter having a proximal RHV with a flush line. Other pressure transducers can be located along the aspiration lumen. The bubble test configuration 900 is used to have the surgeon rehearse use of the aspiration thrombectomy system 600 to practice best bubble removal/prevention techniques. The bubble test configuration 900 allows the surgeon to obtain a purge score based upon detection or lack of detection of air bubbles before and during use of the ROAR catheter 660. In an embodiment where the extension line 674, 674' is separate from the cassette assembly 710, the surgeon first connects the extension line 674, 674' to the proximal manifold connector assembly 670 of the vacuum source 610. Alternatively, where the extension line 674, 674' is integral with the first and second whips 722, 752, this connection is not needed. It is noted that both the ROAR remotely controlled extension line 674 and the ROAR extension line 674' are shown in FIG. 84. Then, the surgeon clears the extension line 674, 674' by placing the distal end of the extension line 674, 674' into purge fluid and then by pressing the purge function activator/button 677, for example. Activation of purge causes the vacuum and vent valves 620, 650 to open, thereby drawing in fluid through the distal end of the extension line 674, 674' and into the collection canister 612. Activation of the off activator/button 679 ceases vacuum or ROAR at any point. Any air is removed from the extension line 674, 674' of the bubble test configuration 900. As this is an automatic purging, or a set time for purge that is greater in time to guarantee flow of fluid present at the distal end of the extension line 674, 674' into the collection canister 612, there is little risk of bubble entry in this step. The test catheter is set up with at least one of a guidewire and a microcatheter therein and an RHV at the proximal end through which the guidewire and/or microcatheter extend. The RHV has a flush line connected thereto and flush fluid is being administered during the test. In several embodiments, the surgeon has already connected a flush line to an RHV at the proximal end of the catheter 660 and now removes the guidewire and/or microcatheter with flush. The RHV is removed and the surgeon uses a meniscus-to-meniscus technique to connect the proximal end of the catheter 660 to the distal end of the extension line 674, 674'. Ideally, this action of connecting does not introduce any air in the two interior lumens but careless connection by a surgeon can. After navigating the distal end of the catheter through the vasculature model to the distal pressure transducer, the ROAR test can begin. A bubble evaluation score is calculated based upon looking at the pressure signal generated at the distal pressure transducer.

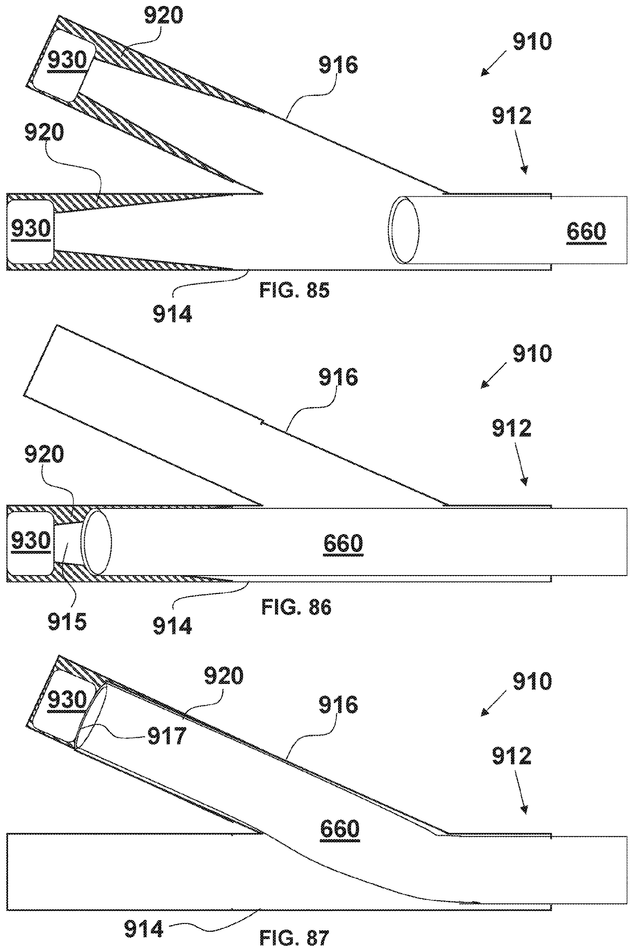

Shown in FIG. 85 is a cross-section of an embodiment of a testbed 910 for performing ROAR on a simulated clot 920, depicted in FIG. 84 with dashed lines. The testbed 910 includes an entry port 912 shaped to receive slidably therein the distal end 664 of the catheter 660 in a fluid-sealed manner (e.g., in the manner of a Touhy-Borst Valve). The interior of the testbed 910 is hollow and, in this embodiment, has two channels through which the distal end 664 of the catheter 660 can pass. The first channel 914 is in line with the central axis of the entry port 912 and the second channel 916 extends at an angle to the central axis of the entry port 912. This two-channel configuration is only exemplary and one, two, or more channels can be provided as desired for training a surgeon (this can also be part of an anatomical flow model). The first channel 914 is provided to give the surgeon the easiest way to guide the distal end 664 of the catheter 660 to a simulated clot 920 - straight ahead. The second channel 916 is provided to give the surgeon a more difficult path in which to guide the distal end 664 of the catheter 660 to a simulated clot 920 - into a branch using, for example, a guidewire/micro-catheter. In an embodiment, the simulated clot 920 is molded silicone having a proximal opening diameter approximately equal to or slightly less than the interior diameter of the respective first and second channels 914, 916. In this manner, as the surgeon advances the catheter 660 in either of the first or second channels 914, 916, the distal end 664 of the catheter 660 will embed itself in the simulated clot 920 and seal itself off from the remaining interior of the testbed 910 either at a short distance from (see FIG. 86) or at the interior end face of (see FIG. 87) the respective first or second channel 914, 916. In this state, a characteristic (e.g., pressure) at the distal end 664 of the catheter 660 can be measured and shown to a user to assess whether the action caused by ROAR on a clot is maximized. By placing a sensor 930 at the distal end(s) of the interior lumen(s) of the testbed 910 (e.g., a pressure sensor), pressure can be measured with great accuracy. Placement of the sensor 930 is shown at the distal end of the internal space of the simulated clot 920, which is tapered inwards to grasp and seal the distal end 664 of the catheter 660 when inserted therein. By having an accurate measurement of action (e.g., pressure, force, strain, flow, motion, displacement, acceleration, impulse, heat-transfer, temperature, optical, audio, visual, MEMS) against a clot caused by the catheter 660, a baseline characteristic curve or value(s) can be measured and stored, for example, in the processor and/or the memory of the controller of the testbed, which can be the controller 700 of the aspiration thrombectomy system 600. Knowing that a given test or tests have no air bubbles in the internal lumens allows the testbed 910 to define an ideal characteristic curve or value(s) (e.g., max/min) for use of the aspiration thrombectomy system 600. When a surgeon practices with the bubble test configuration 900, the characteristics measured by the sensor 930 can be compared to the ideal curve/value(s) and, thereby scored using various signal comparison or Al techniques. From this comparison, a score can be provided to the surgeon. When the surgeon has undergone practice with the testbed 910 sufficient to achieve a score that is in a defined upper percentage, then the surgeon can know that similar use of the aspiration thrombectomy system 600 within a patient should achieve similar high ROAR action results against a clot present at or adjacent to the distal end 664 of the catheter 660. If desired, a score indicator (e.g., numerical, graphical, colored, audible, haptic) can be supplied on the bubble test configuration 900 (e.g., at or near the system controls 676).

Placement of a sensor 930 is not limited to the testbed 910. For example, the sensors 930 can be placed anywhere along the interior lumen of the catheter 660, the extension line 674, 674', or in the lumen of the proximal manifold connector assembly 670 where the aspiration thrombectomy system 600 can measure action of the incompressible fluid in these lumens, thereby providing valuable information to the aspiration thrombectomy system 600 (and thereby the user) to measure efficiency of the pulsating aspiration applied to a clot within a patient. Accordingly, the catheter 660 is supplied with a sensor 931 that measures characteristics (directly or indirectly) within the lumen while ROAR occurs, which can be carried out by pressing one of the system controls 676, e.g., the ROAR function activator/button 678. Suitable sensors 931 includes, but are not limited to, pressure, force, strain, flow, motion, displacement, acceleration, impulse, heat-transfer, temperature, optical, audio, visual, and MEMS sensors. The sensor 931 can include a micro, three-axis, gyroscopic sensor that provides inertial output data to the controller 700, which data is converted into a characteristic curve/value(s) to be compared to an ideal ROAR curve/value(s).

One interesting characteristic of the ROAR aspiration performed by the system 600 is that a user can feel the pulsatile aspiration when grasping the catheter 660 or the extension line 674, 674'. An experienced user of the system 600, for example, can tell by feel when the system 600 is running well and can feel what state in which ROAR is running, e.g., corked or open flow. Alternatively, or in addition to tactile feedback, at least one sensor 931, 932, 934 that detects the motion/momentum can evaluate the movement electronically (e.g., motion, displacement, acceleration, impulse) and transmit data to the controller 700 for analysis. Thus, the controller 700 can evaluate whether the ROAR aspiration is operating in the most efficient mode or not. If characteristics are so outside pre-determined expectations, aspiration can be ceased. Alternatively, if a characteristic(s) is(are) outside ranges but can be compensated for by a change(s) of system parameters, e.g., over push, Vac-ON Time 622, Vac-ON Duration 624, Double Off Times 1 and 2 625, 627, Vac-OFF Time 626, the speed at which a valve closes (e.g., between 1 ms and 50 ms), and/or Vac-OFF Duration 628, then operation can continue with such changes implemented.

The sensor 931 can be located at the distal end 664 of the catheter or anywhere along the length thereof in any position where it can measure characteristics of the fluid within the interior lumen of the catheter 660. Additionally, or alternatively, the extension line 674, 674' is supplied with a sensor 932, as described herein, that measures characteristics within the lumen (directly or indirectly) while ROAR occurs. The sensor 932 can be located at the distal end of the extension line 674, 674' or anywhere along the length thereof in any position where it can measure characteristics of the fluid within the interior lumen of the extension line 674, 674'. Additionally, or alternatively, the proximal manifold connector assembly 670 is supplied with a sensor 934, as described herein, that measures characteristics within the assembly's lumen (directly or indirectly) while ROAR occurs. The sensor 934 can be located at the distal end of the proximal manifold connector assembly 670 where it can measure characteristics of the fluid within the interior lumen of the catheter 660 but can be distal of or within the junction 702 (of the tubing whips 722, 752) to place the valves 720, 750 proximal of the sensor 934.

Described herein are at least one sensor 930, 931, 932, 934. This phrase, however, is not used to limit the sensors to one, two, or three sensors as described. There can be any number of and any combination of sensors deployed along the aspiration lumen. Data supplied by the sensors are relative and correspond to a position along the aspiration lumen. This data can be supplied between and among the sensors in addition to being supplied to the controller 700.

A separate passive sensor can be placed at the system 600 that may or may not be connected to the controller 700, which sensor can be analog to display a flag or other visual indication when minimum ROAR performance is not achieved or when ROAR performance is achieved.

When using at least one sensor 930, 931, 932, 934 during a surgery, information is provided to the controller 700 that is used to provide data to the surgeon on attributes of the ROAR process, e.g., how effective it is/was, whether timing of the valves needed to be adjusted, whether the procedure needs to be re-applied, and/or whether bubbles were present.

Including of any of the sensors 930, 931, 932, 934 with the catheter 660 or the extension line 674, 674' of the system 600 provides the benefit of identifying the type and/or characteristics of the component(s) being used in the procedure. The sensor(s) 930, 931, 932, 934 can store an ID and/or other data associated with the component, which can be read by the controller 700. Alternatively, or additionally, the controller 700 can identify the type of sensor passively to, thereby, know what component is attached/being used. Additionally, the sensor(s) 930, 931, 932, 934 can be used to ensure/validate a fluidic connection between the component and the system.

In a severe case where an excess amount of air is present, the controller 700 can detect the air and inform the surgeon that a purge needs to be carried out manually or the system 600 can pause and purge the aspiration lumens automatically. Alternatively, if a minimum amount of air is present, the system 600 can adjust valve timing and/or ROAR duration to compensate for the possible decrease in performance. These conditions describe on-the-fly events during surgery but also a single pulse or different action at the beginning of each ROAR application can occur to check for readiness before ROAR is applied. The pulse/action can be done automatically or manually selected by the user. All data measured by the system 600 can be stored for later analysis and/or training of the Al and/or for updating system parameters, such as valve timing and/or duration.

Cycle optimization can also be based upon characteristics unrelated to bubbles. For example, a new catheter can be measured in a test mode or on the fly and the system 600 tunes itself to optimize particular characteristics of the system 600 for that new catheter. Optimization can also occur for different flow states, for example, in wide-open flow or in a complete lack of flow or in any flow state in between.

The feedback to the surgeon when optimal ROAR is not achieved can include (1) an indication to revert to static aspiration and to prolong application of static aspiration after ROAR and (2) an indication of insufficient performance that can be used to improve the surgeon's skill at bubble mitigation in future cases.

With at least one sensor(s) 930, 931, 932, 934, the system 600 can determine diminishing returns during application of ROAR aspiration and automatically convert to static aspiration. Additionally, the system 600 can change between different pre-defined ROAR valve timing profiles based on at least one sensor(s) 930, 931, 932, 934 feedback. For example, a first ROAR timing cycle can be applied and, if the controller determines insufficient or excessive sensor feedback data, the valve timing can change to increase or decrease the aggressiveness of the valve timing, which can include any one or more of over push, Vac-ON Time 622, Vac-ON Duration 624, Double Off Times 1 and 2 625, 627, Vac-OFF Time 626, the speed at which a valve closes (e.g., between 1 ms and 250 ms), and/or Vac-OFF Duration 628.

With the presence of at least one sensor(s) 930, 931, 932, 934, the controller 700 can be provided with data that indicates a location of the clot within the catheter 660 or in the tube set, in the valve(s), or in any aspiration lumen of the remainder of the tube set. For example, a strain gauge or similar sensor placed in the at least one of the whips that is part of one of the valves can detect when the clot is at least partially contained within that section of tubing at the particular valve because closure of the valve is impeded.

With the above features, the system 600 can carry out a clot hunting mode. It is understood that full vacuum with open flow in a vessel is undesirable after a given time because of blood loss concerns. The system 600 can slightly open or intermittently open the vacuum valve 720 (and keep the vent valve 750 closed) while the surgeon advances the catheter 660 through the vasculature. When the distal end 664 of the catheter 660 encounters a clot, a decrease in the flow rate or a decrease in pressure is detected by the at least one sensor(s) 930, 931, 932, 934, indicating the presence of the clot at or adjacent the distal end 664. With this detection, the controller 700 automatically performs ROAR or static aspiration for either a prescribed amount of time or until the at least one sensor(s) 930, 931, 932, 934 or the controller 700 determines that the clot no longer is present at the distal end 664, in the aspiration lumen of the catheter 660, or in the aspiration lumen of the tube set. At the end of the application, the system 600 converts back to the hunting state (vacuum valve 920 slightly open or intermittently open) for further clot removal(s).

By obtaining the maximum pressure existing at the vacuum source 610, a measurement by at least one of the sensors 930, 931, 932, 934 of a single pressure value at that sensor location can be used to determine actual flow rate of the fluid within the aspiration lumen at which the sensor is present. Alternatively, with a lookup table stored in the controller 700 of corresponding flow rates within the aspiration lumen, a single measurement by one of the sensors 930, 931, 932, 934 of a characteristic, such as pressure, within the aspiration lumen can allow the controller 700 to determine flow rate and/or to communicate it to the surgeon.

A difficulty to aspirate, due to the softness/hardness/volume of the clot, can be detected and stored in the memory of the controller 700. The effort that is required to aspirate the clot is detectable because, for example, the pressure fluctuations required to aspirate greater volumes of clot are larger than smaller volumes, which fluctuations are approximately linear.

Rarely, there is a leak in the aspiration lumens of the various components of the system 600. If a leak from the aspiration lumen to the environment is present, the sensor(s) (e.g., in the form of a pressure or flow sensor) can detect that there is a hole or a poor connection between various components along the line of the aspiration lumen.

Also rare in the operation of the system 600 are instances where, for example, the vacuum motor 614 is not running properly, a restriction in one of the whips 722, 752 exists, or a valve 720, 750 is not properly opening. Each failure of these system components can be detected by the at least one sensor(s) 930, 931, 932, 934 and reported to the controller 700 for warning to the surgeon. If an error is identified, a visual display (e.g., LED, LCD) or audio source can indicate the exact system malfunction, giving the surgeon or staff an ability to quickly correct the error.

If there is a device remaining in the aspiration lumen that was supposed to be removed before ROAR use (e.g., stentriever, microcatheter, guidewire), the at least one sensor(s) 930, 931, 932, 934 can either warn the surgeon, lock out the surgeon, or change parameters, such as valve timing, to adjust for that obstruction.

The sensor(s) can detect a corked state on a clot and inform the controller 700 of that state in order perform ROAR and/or any other optimization and to indicate this state to the user, e.g., with a green indicator. One of the sensors 930, 931, 932, 934 described herein, e.g., a pressure sensor, can perform one or all of the features described above.

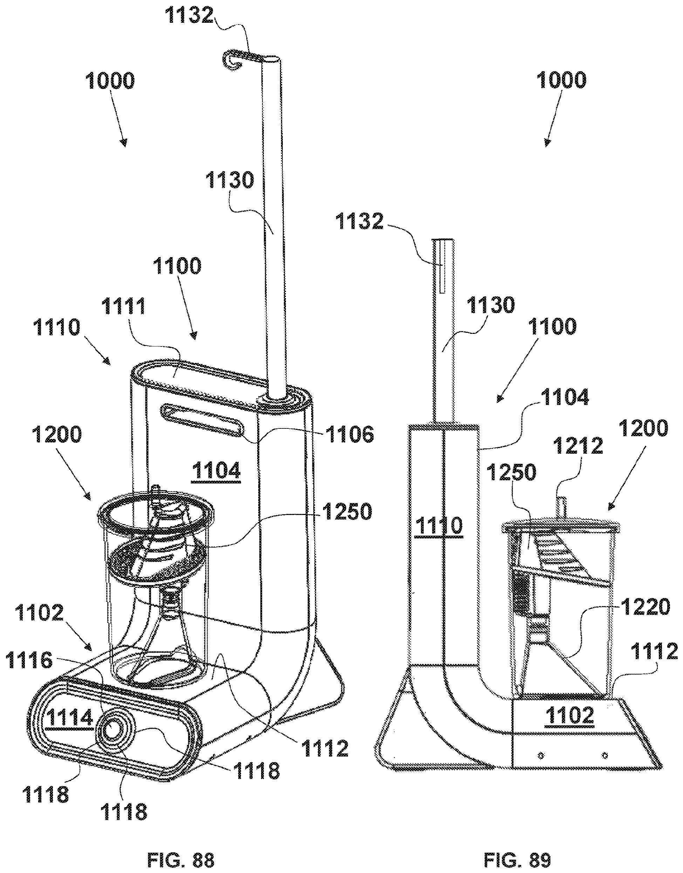

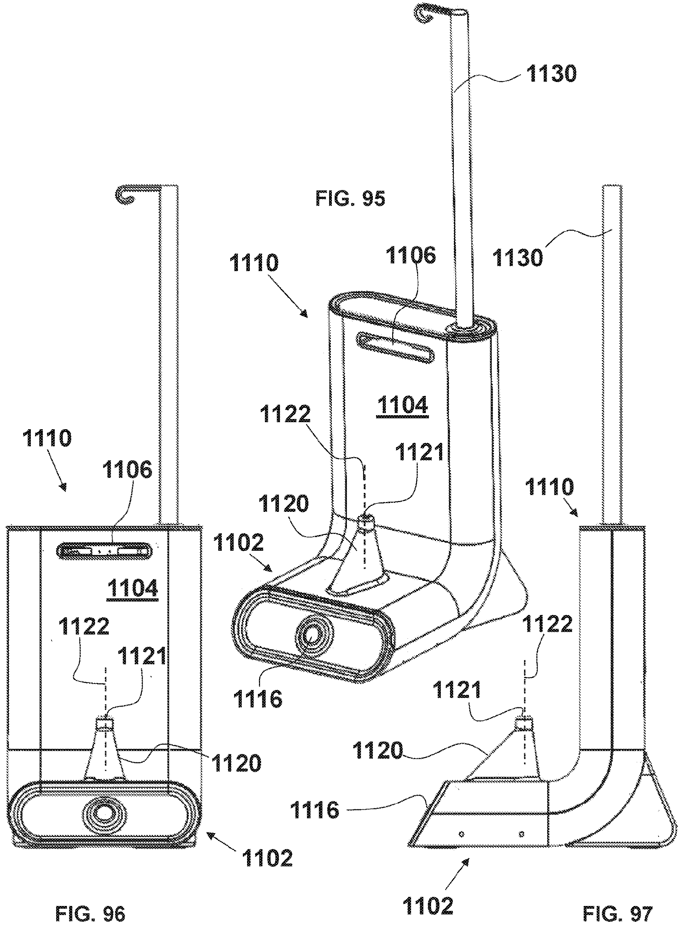

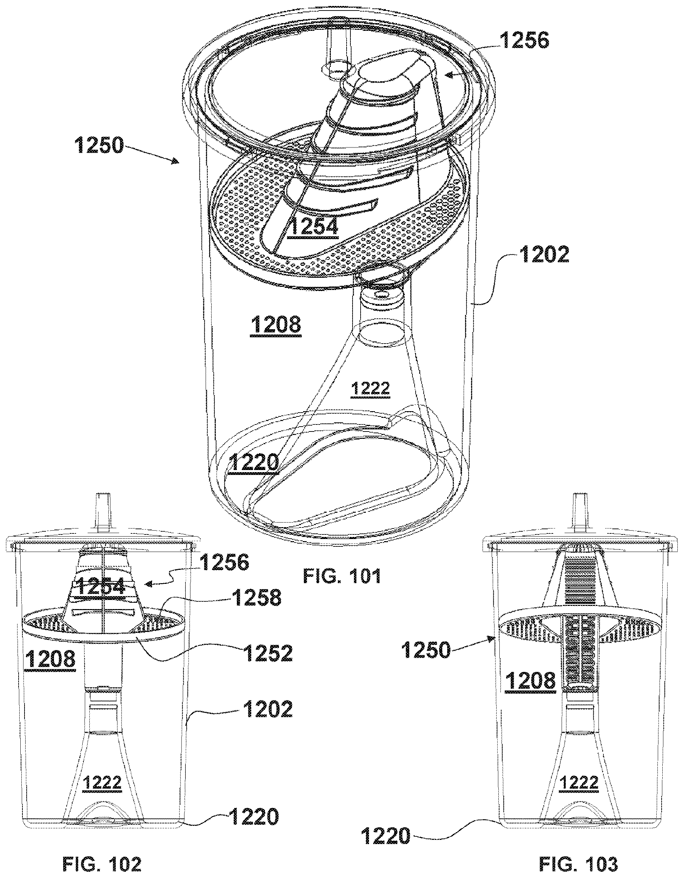

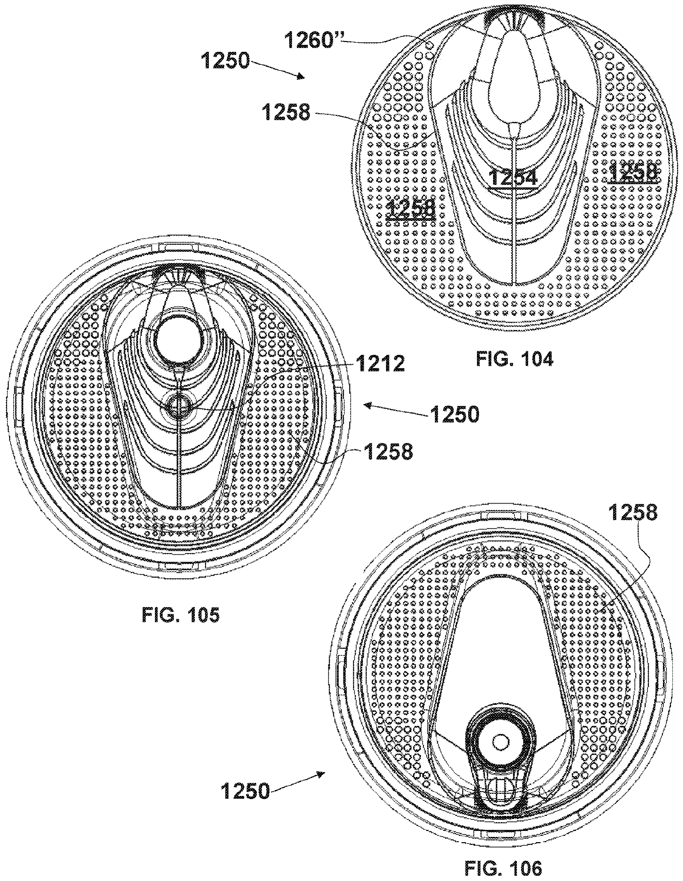

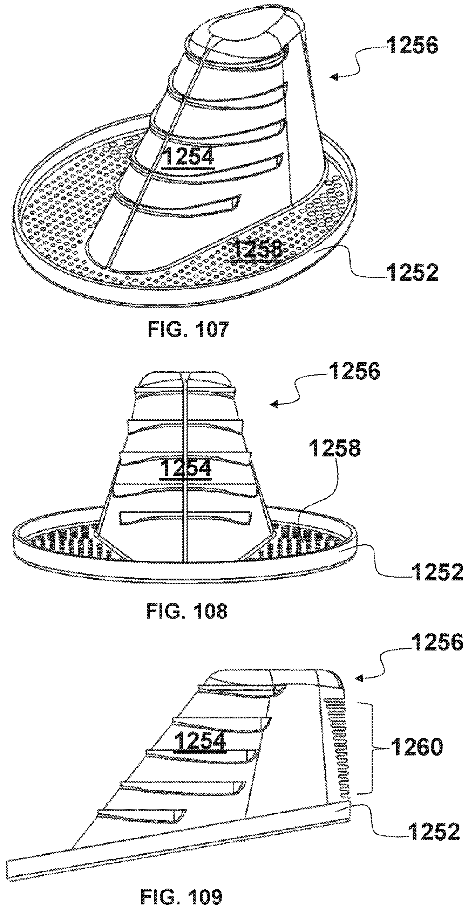

Another embodiment to the aspiration thrombectomy system 600 is the aspiration thrombectomy system 1000 of FIGS. 88 to 109, features of one can be used in the other. Accordingly, when reference numeral 600 is used anywhere herein, reference is being made to both aspiration thrombectomy systems 600 and 1000 even when numeral 1000 is not present, and vice versa.

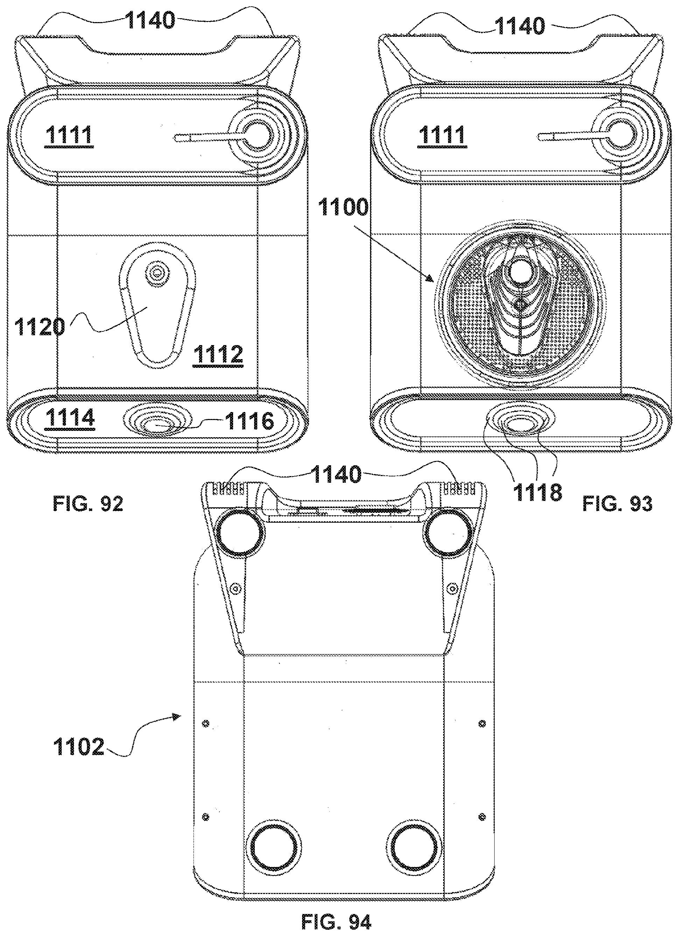

The vacuum console 1100, from a side view, is L-shaped as shown in FIG. 89, providing a lower horizontal front section 1102 and an upper vertical rear section 1104. A vacuum canister 1200 rests removably on a horizontal lower platform 1112 that forms the top of the lower horizontal front section 1102, as shown in FIGS. 92 and 95 to 97. The lower platform 1112 contains a unipositional, upwardly extending boss 1120. In an embodiment, this boss 1120 has an exterior shape that allows the vacuum canister 1200 to be placed thereon in only one orientation (out of the possible 360-degrees of rotation about a vertical axis 1122) with respect to the lower platform 1112. The lower horizontal front section 1102 has a front face 1114 that has at least one user interface 1116 and is tilted to place an upper extent thereof further rearward than a lower extent, thereby giving the user easier access when the vacuum console 1100 is placed, for example, at a standard counter height. In the embodiment shown, there is only one user interface 1116 in the form of a button. Any other configuration is also possible, for example, the three-button configuration of the system 600 in FIG. 57. The user interface 1116 can be surrounded by lighted indicators 1118 that, for example, show a state where full vacuum exists when the outer light ring is illuminated (or all rings are illuminated) and show a state where less than full vacuum exists when one (or more inner light rings) is (are) illuminated. The front face 1114, in an alternative embodiment, provides audio or visual indicator(s) of ROAR scores and/or performance in the form, for example, of an LED, a LCD, a dot-matrix, a flip-dot display, a speaker, a chime, a beep, and even use of voice-coil valve actuators as audio source, to name a few.

The upper vertical rear section 1104 has a top side 1130 from which an enlargeable pole 1130 extends. The pole 1130 can be extended from a completely stowed position to a partially extended position shown in FIG. 89 or to a fully extended position shown in FIG. 88. A fixed or pivotally connected hanger 1132 (e.g., in the form of a hook or carabineer or the like) is connected at an upper end of the pole 1130. The pole 1130 and hanger 1132 support a fluid source, such as an IV bag, for use with the system 1000 as, for example, a vent fluid. The IV bag can be used as the fluid supply for purge of the catheter 660. The pole 1130 can be a single piece or it can be a telescoping set of pole portions. To lock such telescoping pieces, latches in various forms are provided. In one exemplary embodiment, the latches are press-buttons similar to those in luggage handles. In another exemplary embodiment, the latches are rotary friction locks. In a further exemplary embodiment, the latches are ball detents. In one exemplary embodiment, the pole 1130 can be a sealed telescoping set of pole portions having a hollow interior portion connected to a check valve that controls leak of interior fluid (e.g., air) to the atmosphere of the environment and provide the pole 1130 with damped retraction/extension characteristics. The pole 1130 can be formed in the manner of tent poles with several interlocking pieces connected by a stretch cord or a few pieces with threaded ends to screw the various pieces together to provide a set of pole lengths. Alternatively, the pole 1130 can be an entirely separate component from the vacuum console 1100 or it can be removably or permanently affixed to console 1100.

Selection of the height of the upper vertical rear section 1104 can provide various changes to the pole 1130. For example, an increase of a height of the console 1100 reduces the number of segments required in a multi-part pole 1130. The L-shape of the console 1100 provides a wide and stable base to mitigate or even prevent tipping. Weight can be added to the lower front section 1104 for that purpose to lower the console's center of gravity.

In the exemplary embodiment depicted in FIGS. 88 to 90, 93, and 98 to 103, the vacuum canister 1200 is transparent. Alternatively, the vacuum canister 1200 can be translucent. As can be seen in FIG. 89, when the vacuum canister 1200 is placed on the lower platform 1112, a user has visual access to the entirety of the interior of the vacuum canister 1200 from any angle around the exterior of the vacuum canister 1200, making it easy to view a clot that may be drawn into the interior of the vacuum canister 1200, whether it is in the front adjacent the front face 1114 or it is behind the clot catcher 1250 adjacent the upper front wall 1104. More particularly, the configuration of the vacuum canister 1200 with respect to the vacuum console 1100 permits visualization over greater than 180 degrees around the canister 1200 and, in particular, greater than 270 degrees or even a full 360 degrees about the canister 1200. Visibility of a removed clot is important for many reasons, including, for example, to end a thrombectomy and remove the patient from surgery and to see the components of the clot and determine if further clots remain or if foreign matter is present.

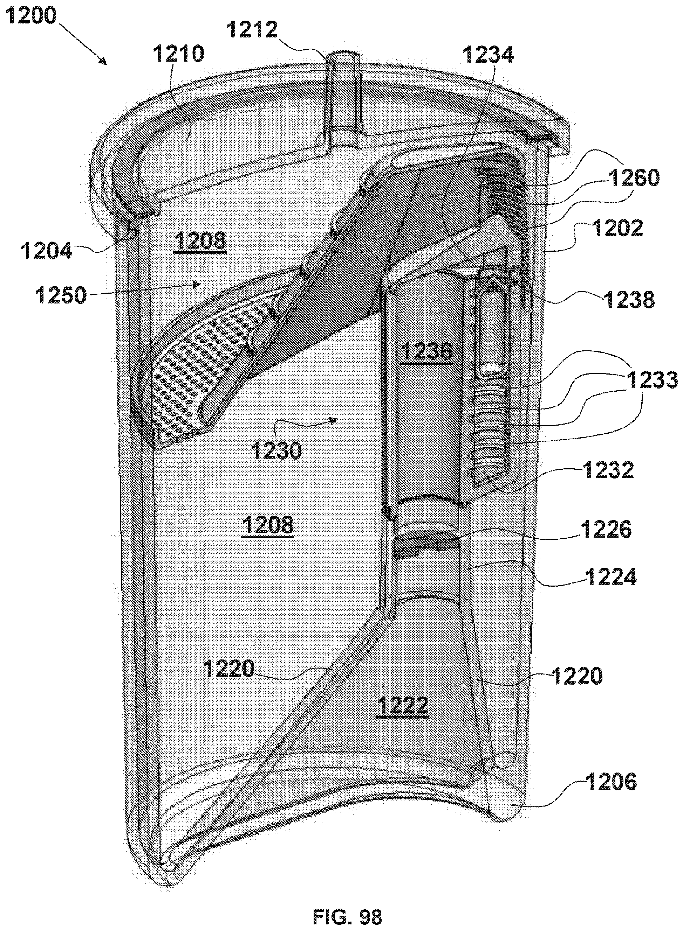

The exterior vertical shape of the vacuum canister 1200 in the embodiment shown is a hollow cylindrical wall 1202 having an upper edge 1204 with a diameter somewhat greater than a diameter of a closed bottom 1206 thereof. Further, the bottom 1206 has an upwardly extending floor 1220 defining an interior mounting cavity 1222 having a shape substantially corresponding to an exterior surface of the boss 1120, which surface projects upwards from the lower platform 1112. The floor 1220 continues upwards along a neck 1224 and through further features that are explained in further detail below. In this exemplary embodiment, the two corresponding shapes are in the form of a funnel having an offset center. With such a shape, the vacuum canister 1200 rests on the boss 1120 and lower platform 1112 in one orientation. Alternatively, the vacuum canister 1200 is radially symmetrical and sits on a similarly cylindrical boss on the flat horizontal lower platform 1112 of the console 1100. In an embodiment, the horizontal lower platform 1112 and the upper front wall 1104 are white in color. What is valuable in an embodiment having a non-concentric boss and a clear canister with adjacent white console walls is that the single orientation allows a user maximized visualization of all of the volume within the canister 1200 and to easily visualize a removed clot on the interior filter. Various features of the vacuum canister 1200 are explained with respect to FIGS. 98 to 109, as detailed herein, these drawings are not to scale.

The top of the canister 1200 is, in an embodiment, a removable lid 1210. The lid 1210 is also clear or at least transparent. A hollow vacuum inlet 1212 of the canister 1200 projects upwardly from a top surface of the lid 1210 and fluidically connects an interior volume 1208 of the canister 1200 to the environment surrounding the canister 1200. In use, a proximal end of the first tubing whip 722 is connected in a fluid tight manner (either removably or permanently) to the vacuum inlet 1212. In this manner, any clot drawn in through the aspiration lumen will pass through the vacuum inlet 1212 and into the interior volume 1208. The thickness of the lid 1210 can be formed with curved outer surfaces (convex and/or concave) to magnify contents therein and provide the user with easier visualization of any clot aspirated into the interior volume 1208. One suitable configuration is in the shape of a telescope dome having a height that projects above the upper edge 1204 for increased visibility. In the embodiments shown, the vacuum inlet 1212 is aligned substantially within a center of a circular perimeter. This orientation increases clot visualization when combined with an offset internal filter as described in further detail below. In an embodiment, the proximal exit of the vacuum inlet 1212 is approximately 13 mm (0.5") above the internal filter.

When aspirated blood passes through the vacuum inlet 1212 and enters the interior volume 1208, the blood exhibits bubbles and foaming, each of which can reduce visibility within the interior volume 1208. To eliminate or reduce the bubbles/foam, a blood foaming reduction nozzle is connected to the interior lumen of the vacuum inlet 1212. Bubbles or foam are/is created because the blood enters the interior volume 1208 in an unrestricted manner. By directing the blood in a controlled manner, for example, in a vortex or cyclone, bubbles and foam are reduced. Also, the inside surface of the lid 1210 can be provided with a textured mesh that attracts the bubbles as they form and spread them out radially.

A clot that is aspirated into the vacuum canister 1200 can be viewed easily as long as the volume of blood does not fill the interior volume 1208 or at least fill a lower portion of the interior volume 1208 such that visualization of the clot above the grating 1258 becomes problematic. In some prior art vascular vacuum aspiration surgical procedures, the volume of blood that is aspirated is greater than the capacity of the vacuum canister being used to collect the blood and trap the clot. In such circumstances, a clot can be aspirated and the surgeon will not know that this has occurred due to the presence of the clot under the surface of the similarly colored and opaque liquid blood. The vacuum canister 1200 contains features to limit the amount of liquid blood into the interior volume, which features are located at the upper portion of the floor 1220. In particular, the neck 1224 is hollow and forms an upper entry orifice fluidically entering the mounting cavity 1222. The orifice of the neck 1224 forms the vacuum exit of the vacuum canister 1200 and directs all exiting fluid (e.g., air) into an upper entry orifice 1121 at the top surface of the boss 1120. This upper entry orifice 1121 is fluidically connected and sealed to the ingress of a non-illustrated vacuum pump that is housed within the vacuum console 1100. To ensure a tight and leak-free seal between the upper entry orifice 1121 at the top of the boss 1120 and the vacuum canister 1200, a gasket 1226 (for example, of rubber, silicone, latex, or another polymer or combinations thereof) is provided in the neck 1224 of the floor 1220, as shown particularly well in FIG. 98.

It would be damaging to the vacuum pump/motor in the vacuum console 1100 if any liquid other than air was to make it past the gasket 1226. Accordingly, a valve sub-assembly 1230 is provided upstream of the gasket 1226. The upstream valve sub-assembly 1230 can be removably connected to, fixedly connected to, or integral with the neck 1224 of the floor. In the embodiment shown, in particular in FIG. 98, the valve sub-assembly 1230 is fixedly connected to the neck 1224 upstream of the gasket 1226. The valve sub-assembly 1230 includes, in an upstream-to-downstream fluid flow direction, an inlet chamber 1232, a flow over chamber 1234, and an exit chamber 1236. The inlet chamber 1232 has a cylindrical interior surface that defines one or more inlet orifices 1233, which in the exemplary embodiment are a set of parallel, annular slots extending from the bottom of the inlet chamber 1232 to the top of the inlet chamber 1232. The exit of the inlet chamber 1232 leads downstream into the entrance of the flow over chamber 1234, which is horizontal in the exemplary embodiment. The exit chamber 1236 is a vertical cylinder fluidically connecting to the interior of the neck 1224 and having an interior cross-sectional shape corresponding to a cross-sectional interior shape of the neck 1224 so that fluid flow across the joint therebetween has turbulence minimized. In an embodiment, the gasket 1226 has a central bore having a shape and size equal to the cross-sectional interior shapes of the neck 1224 and the exit chamber 1236 (at least at the bottom of the exit chamber 1236). In another embodiment, the gasket 1226 defines a central bore that is reduced in diameter with respect to the cross-sectional shape of the exit chamber 1236, which is substantially equal to the diameter of the vacuum inlet 1212 of the lid 1210 of the vacuum canister 1200. The weight of the vacuum canister 1200 alone is able to establish the seal between the floor 1220 and the boss 1120.

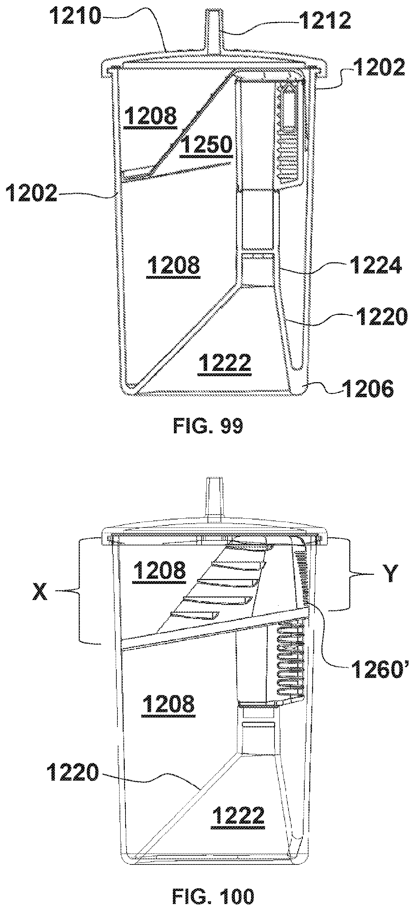

The inlet chamber 1232 contains therein a float valve 1240. The float valve 1240 is movable vertically within the inlet chamber 1232. In any operation of the vacuum, the float valve 1240 rests, by gravity, at the bottom of the inlet chamber 1232. As the interior volume 1208 fills with liquid, e.g., blood during thrombectomy surgery, that liquid raises the float valve 1240 within the inlet chamber 1232. The upper portion of the float valve 1240 is shaped to form a liquid-tight seal with either or both of a top/exit of the inlet chamber 1232 and an entrance of the flow over chamber 1234, indicated in FIG. 98 with reference numeral 1238, when the float valve 1240 is raised. This raised but not yet sealed position of the float valve 1240 is shown in FIGS. 98 and 99. When raised to the top position, the float valve 1240 prevents aspiration of fluid contained in the interior volume 1208 from entering into the vacuum pump. An alternative, non-illustrated, valve that prevents fluid ingress to the vacuum pump is a concentric, donut-shaped float valve surrounding a central post, such as the exit chamber 1236. As an alternative, or in addition, a water-impermeable membrane can serve as an ingress protection filter to the vacuum pump.

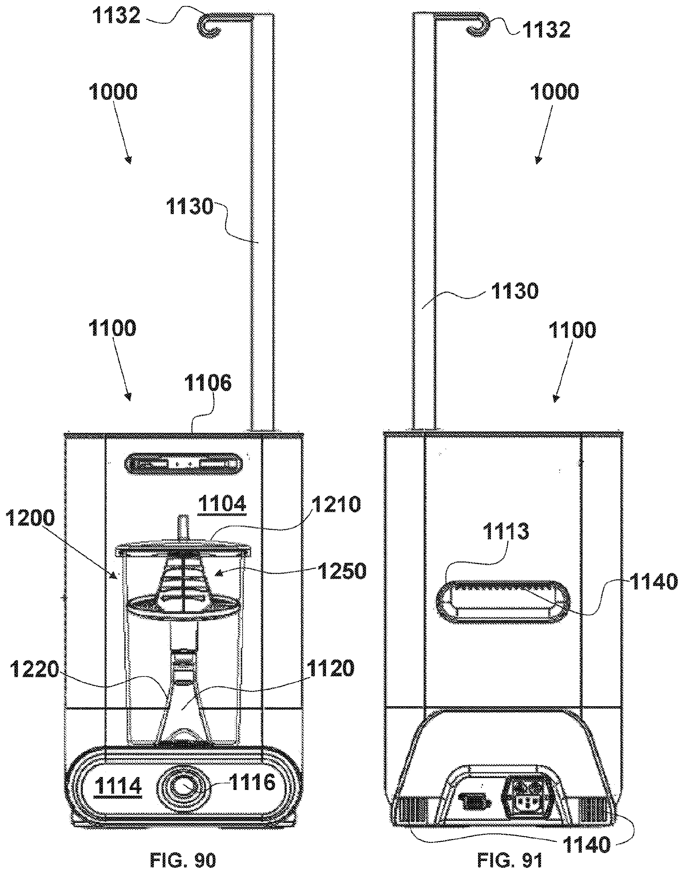

At various places herein, the vacuum pump is mentioned. Such pumps generate heat that may to be dissipated from the vacuum console 1100. While it would be beneficial to place heat-releasing vents on the top surface 1111 of the upper vertical rear section 1110, vent and/or purge fluid can be suspended directly above the top surface 1111 from the hanger 1132 - a position that would directly deposit liquid into the vacuum console 1100. As liquid in the vacuum console 1100 is to be avoided, heat-transfer vents 1140 can be provided on rear or lower-facing surfaces such as those on the lower horizontal front section 1102 in FIGS. 91 to 94 or in the ceiling of the handgrip 1113 as shown in FIG. 91.

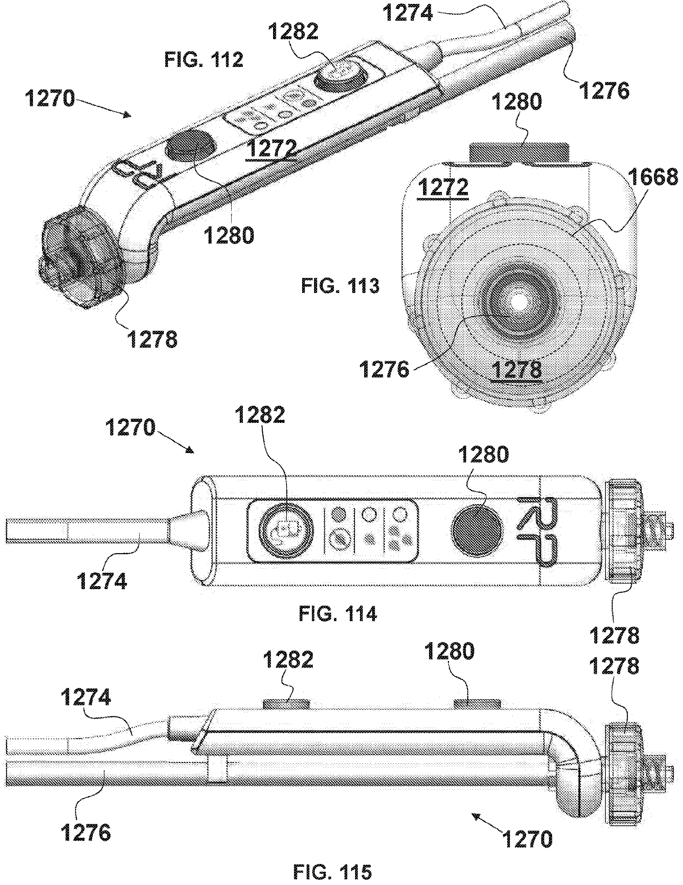

In FIGS. 56, 72, and 84, an extension line 674 is shown having a system control board 676 with remote controls 678. The remote controls 678 can duplicate, replace, or supplement the system controls 676 shown, for example, in FIG. 57. FIGS. 112 to 118 illustrate two additional embodiments of remote-control pendants 1270, 1290 of the aspiration thrombectomy systems, devices, and methods described herein. The remote-control pendants 1270, 1290 are part of the tube set 674 for remote operation from within the sterile field. Placing the pendants 1270, 1290 distal along the extension line 674 enables different design considerations for the vacuum console 1100. Because each pendant 1270, 1290 has all the controls necessary to operate the aspiration thrombectomy systems 400, 600, 1000, the housing of the vacuum console 1100 only needs a singular button to turn on the system and initiate the vacuum pump.

Shown in FIGS. 112 to 118 are wired embodiments of the pendants 1270, 1290. Each of the pendants 1270, 1290 respectively has an operator handle 1272, 1292, a conductor cable 1274, 1294 (having one or more conductors electrically connected to one or more components of the systems 400, 600, 1000), the hollow vacuum extension line 1276, 1296, a distal catheter connector 1278, 1290 (to connect to a catheter hub 661 of the ROAR catheter 660), and remote-control actuators 1280/1282, 1300/1302. The vacuum tubing and electrical cabling between the pendants 1270, 1290 and the system 400, 600, 1000 can be achieved in several ways. In a first exemplary embodiment, shown in FIGS. 112 and 114 to 118, each extension line 1276, 1296 is parallel to an adjacent multiconductor cable 1274, 1294. The conductors of each multiconductor cable 1274, 1294 can be bonded together over the entire length thereof, can be bonded together in small segments along the length, can be separately clipped along a length thereof, and/or each extension line 1276, 1296 has an extruded clip-like feature having a connector in which each multiconductor cable 1274, 1294 sits, respectively. Each multiconductor cable 1274, 1294 can be a multi-lumen extrusion in which wire are co-extruded or are pulled through an open lumen. Each multiconductor cable 1274, 1294 can be co-radial and/or coiled where the internal wires are bonded or extruded into the extension co-radially (a very flexible configuration).

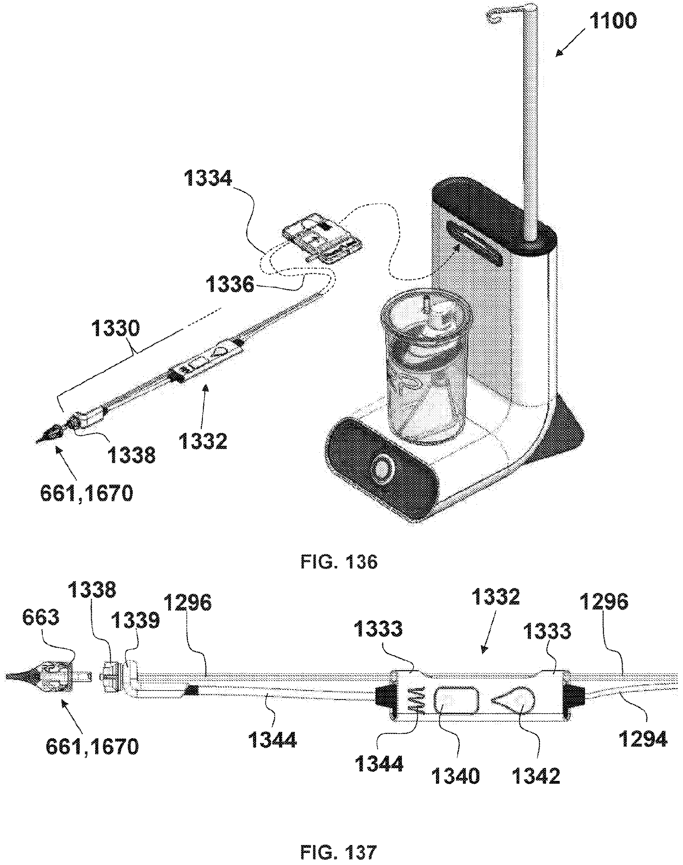

FIGS. 136 and 137 illustrate another exemplary embodiment of a wired remote control pendant 1330 of the aspiration thrombectomy systems, devices, and methods described herein. The remote control pendant 1330 is part of the tube set 674 (or it can be initially separated from but attached later) for remote operation from within the sterile field. Because the pendant 1330 has all of the controls necessary to operate the aspiration thrombectomy systems described herein (e.g., 400, 600, 1000), the housing of the vacuum console 1100 only needs a singular button to turn on the system and initiate the vacuum pump. The pendant 1330 has an operator handle 1332, a conductor cable 1334 (having one or more conductors electrically connected to one or more components of the systems 400, 600, 1000), the hollow vacuum extension line 1336, a distal catheter connector 1338 (to connect to a catheter hub 661, 1670 of the ROAR catheter 660), and remote-control actuators 1340, 1342. In this embodiment, the extension line 1336 is parallel to an adjacent multiconductor cable 1334. The conductors of the multiconductor cable 1334 can be bonded together over the entire length thereof, can be bonded together in small segments along the length, can be separately clipped along a length thereof, and/or the extension line 1336 has an extruded clip-like feature having a connector in which the multiconductor cable 1334 sits. The multiconductor cable 1334 can be a multi-lumen extrusion in which wire are co-extruded or are pulled through an open lumen. The multiconductor cable 1334 can be co-radial and/or coiled where the internal wires are bonded or extruded into the extension co-radially (a very flexible configuration). In comparison to the pendants 1270, 1290, the pendant 1330 separates the tag reader 1339 from the operator handle 1332 containing the two remote control actuators 1340, 1342. A distal tag reader portion of the pendant 1330 is connected to the distal catheter connector 1338, which is shaped to connect to the catheter hub 661, 1670 in a fluid tight manner. When connected, the identification tag 663 is sufficiently close to the tag reader 1339 to permit authentication of the ROAR catheter 660. As above, with proper identification established, ROAR operation is permitted and without only continuous aspiration is possible.

FIG. 137 shows the separation of the tag reader portion of the pendant 1330 from the operator handle 1332. The inventors have discovered that use of a remote-control pendant for the herein described systems can be improved to overcome the inherent cast that is present with tubing forming the vacuum extension line 674, 1376, 1296, 1336, 1674. Typically, the material forming the vacuum extension line 674, 1376, 1296, 1336, 1674 is created or stored or shipped in a coiled form. This manufacture or storage process imparts a biased pre-set shape into the vacuum extension line 674, 1376, 1296, 1336, 1674 that is referred to as cast. The cast typically remains in the material after assembly and causes the line and, thereby, the pendant to move in an undesirable way during use in the sterile field. More particularly, when the physician wants to rest the pendant on a flat surface and control the system by pressing on the two control buttons, the case in the vacuum extension line 674, 1376, 1296, 1336, 1674 prevents the pendant from lying flat against the table surface and, instead, the vacuum extension line 674, 1376, 1296, 1336, 1674 rotates the pendant at an angle requiring the surgeon or the surgical staff to hold down the pendant during use. This disadvantageous characteristic of the vacuum extension line 674, 1376, 1296, 1336, 1674 is resolved by the pendant 1330. Instead of being locked with respect to all movement of the vacuum extension line 674, 1376, 1296, 1336, 1674, the pendant 1330 remains longitudinally held with respect to the vacuum extension line 674, 1376, 1296, 1336, 1674 but is rotationally uncoupled from the vacuum extension line 674, 1376, 1296, 1336, 1674. Specifically, the flattened body of the operator handle 1332 has one or more lateral hollow guide tubes 1333 (e.g., two as shown in FIGS. 136 and 137). The vacuum extension line 674, 1376, 1296, 1336, 1674 passes through a lumen the guide tubes 1333 to remain connected laterally to the vacuum extension line 674, 1376, 1296, 1336, 1674. The lumen has a diameter that is sufficiently larger than the diameter of the vacuum extension line 674, 1376, 1296, 1336, 1674 so that the operator handle 1332 freely moves around the vacuum extension line 674, 1376, 1296, 1336, 1674. An ID conductor extension 1344 longitudinally connects the tag reader 1339 fixed at the end of the vacuum extension line 674, 1376, 1296, 1336, 1674 to a distal end of the operator handle 1332. The ID conductor extension 1344 carries the electrical conductors that carry the identification signal/state that changes when the identification tag 663 is positioned close enough to the tag reader 1339 to change the state for identifying the presence or not of a ROAR catheter 660. The ID conductor extension 1344 is flexible and, therefore, permits the operator handle 1332 to rotate about the vacuum extension line 674, 1376, 1296, 1336, 1674 even when the tag reader 1339 is rotationally fixed to the end of the vacuum extension line 674, 1376, 1296, 1336, 1674. In an alternative embodiment, the tag reader is also rotationally uncoupled from the vacuum extension line 674, 1376, 1296, 1336, 1674 and the distal catheter connector 1338.

Communication between the pendants 1270, 1290, 1330 and the systems 400, 600, 1000 can be wireless, e.g., by Bluetooth^{®} or other communication measures such as optically, audio, infrared, or radio frequency. In such a configuration, the pendants 1270, 1290, 1330 may be battery powered or an alternative configuration may include two-conductor wire for power and communications over wireless. The pendants 676, 1270, 1290, 1330 can have various forms. As described and shown herein, three buttons can be on the pendant 676 (PURGE, ROAR, OFF) to replicate the three buttons 677, 678, 679 found on previous embodiments of the system console. In another embodiment shown in FIGS. 112 to 115, the pendant comprises two activators/buttons: ASPIRATION 1280 and PURGE 1282. The PURGE button 1282 manages purging of the tube set (aspiration lumen) by either complete manual control or through automatic sequences performed by the system, which sequences are initiated by pressing the PURGE Button 1282. One sequence includes a toggling cycle between a full flow, a drip flow, and no flow/OFF. Another sequence toggles to initiate full flow and then automatically converts to a drip for a particular time or due to sensor feedback (e.g., sufficient flow is detected by a flow sensor or no bubbles are detected by bubble sensor), and then to press again to turn OFF flow. Another embodiment employs a so-called "dead-man" switch: full flow occurs when held down, release of the button converts to a drip flow, and then a further press again converts to no flow/OFF. Another embodiment includes pressing an actuator (with or without release) to perform a full flow first and then a drip flow for a given time, and then to turn OFF flow at a second given time after the first given time.

The ASPIRATION button 1280 manages initiation of either pulsing aspiration (ROAR) or continuous aspiration. One embodiment of activating ROAR aspiration includes pressing the ASPIRATION button 1280 and holding a dead-man switch. After release of the dead-man switch, continuous aspiration is applied, and another press of the switch turns off aspiration. Another embodiment includes pressing the ASPIRATION button 1280 to activate continuous aspiration and then subsequently pressing the ASPIRATION button 1280 again to turn off all aspiration. When either a ROAR-enabled catheter is connected or a ROAR-enabled catheter is connected and a purge sequence is completed/attempted, pressing the ASPIRATION button 1280 activates ROAR aspiration and subsequently pressing the ASPIRATION button 1280 again converts ROAR aspiration to continuous aspiration, and, finally, a third pressing of the ASPIRATION button 128 turns off all aspiration.

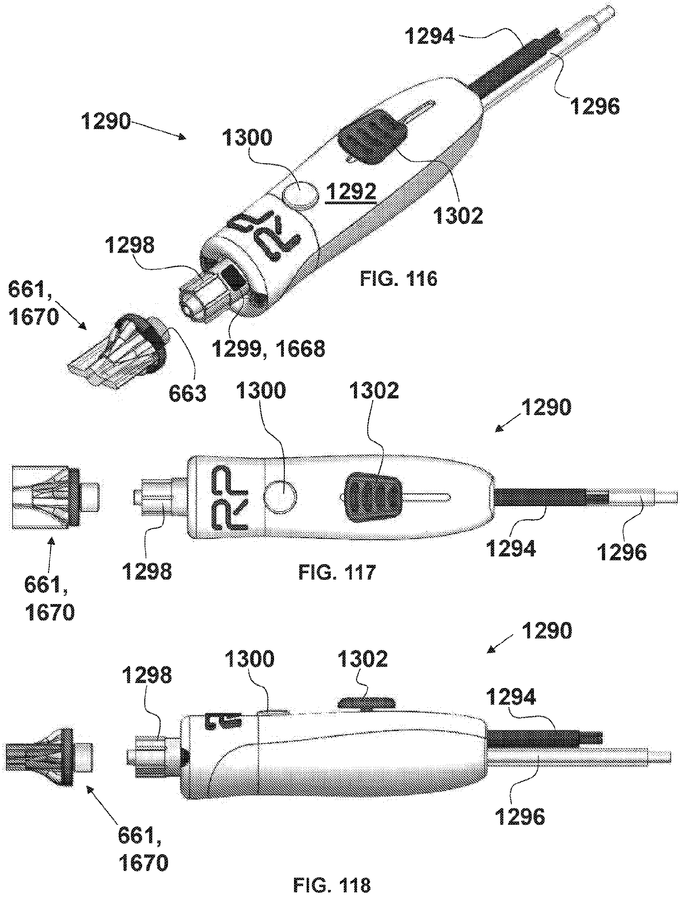

In still another embodiment shown in FIGS. 116 to 119, the pendant 1290 comprises a single button (ASPIRATION 1300) and a single proportional slider 1302 to control purging of the tube set. The slider 1302 has an OFF position in the sliding range, at least one DRIP position within the sliding range (e.g., 1 drip/sec, 1 drip/2-sec, 2 drips/sec, 4 drips/sec), and at least one open flow position within the sliding range (e.g., 50% open flow, 75% open flow, 100% open flow. The various drip capabilities in the pendants 676, 1270, 1290 give an operator the ability to better maintain meniscus-to-meniscus connection of catheter hub 661 and the extension lines 674, 1276, 1296 with a continuous dripping out of the extension lines 674, 1276, 1296. Positioning the pendants 676, 1270, 1290 at the distal end of the extension lines 674, 1276, 1296 (near the proximal end of the catheter 660/catheter hub 661), respectively, is advantageous for providing measures by which to catheter identification is possible. As indicated herein, ROAR pulsatile operation can be optimized by knowing various characteristics of the catheter 660. Placing an identification tag within the catheter hub 661, for example, minimizes a distance that would exist between a tag reader 1299 that is present at a distal end of the pendants 676, 1270, 1290. The identification tag 663 and the tag reader 1299 are shown diagrammatically in FIG. 116. With such features, therefore, the systems 400, 600, 1000 can have unique connectors that only permit a particular identified tube set(s) to be operated with ROAR aspiration; all unidentified or misidentified catheters being able to use the systems 400, 600, 1000 only with continuous aspiration. A positive catheter identification can be made known to a surgeon/user by turning on a light at the pendants 676, 1270, 1290 when a ROAR-enabled catheter is identified, verified, and/or detected. The identification can also be used to indicate to a user other characteristics of the systems 400, 600, 100, for example, it can indicate that the pendants 676, 1270, 1290 are connected to a proper catheter, it can indicate that the connection is a valid connection (e.g., there is no RHV in the aspiration line), it can indicate that the next press of the ROAR/ASPIRATION/GO buttons 1280, 1300 will administer a pulsed ROAR aspiration. This indicator, or another indicator, can blink, for example, while pulsed ROAR aspiration is occurring and the indicator can turn off when pulsing is complete. It is desirable to administer only one application of pulsed ROAR aspiration for each purging of a catheter/tube set. Therefore, the systems 400, 600, 1000 can be set or programmed to lock out any further pulsed ROAR aspiration until a re-purging of the tube set is attempted and/or until the catheter is disconnected and then reconnected. In an embodiment, each pendant 676, 1270, 1290 has visual indicators (e.g., lights in the form of LEDs) that aid a user in meniscus-to-meniscus connection visualization by illuminating the area of connection between the distal end of the extension line and the proximal end of the catheter hub and facilitating in visualization of bubbles.

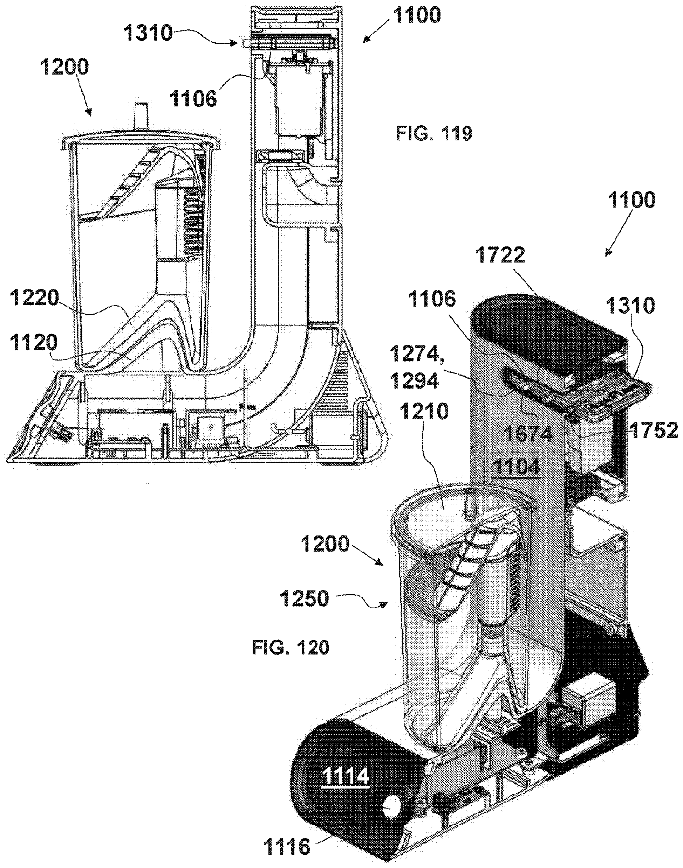

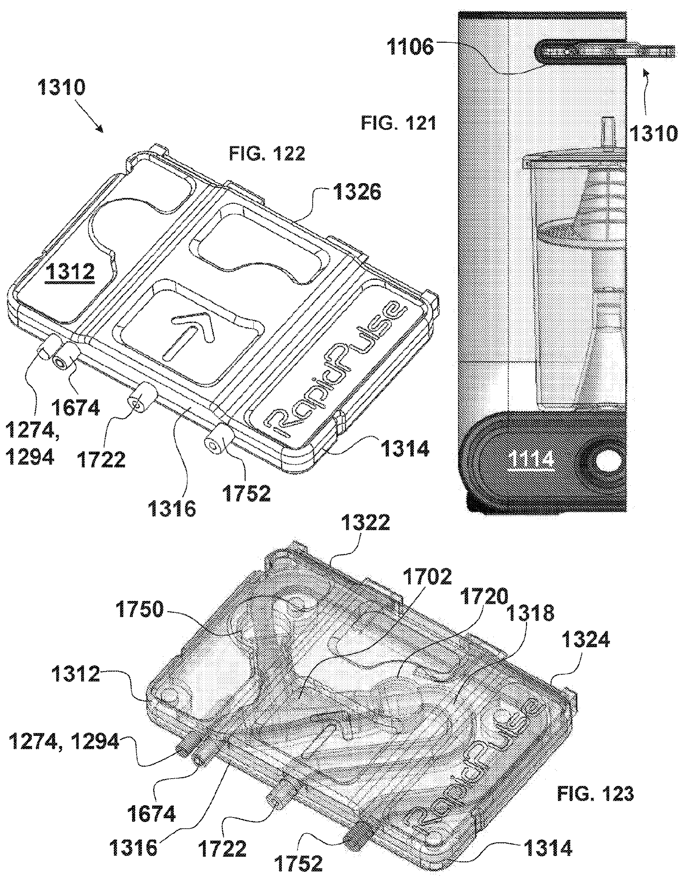

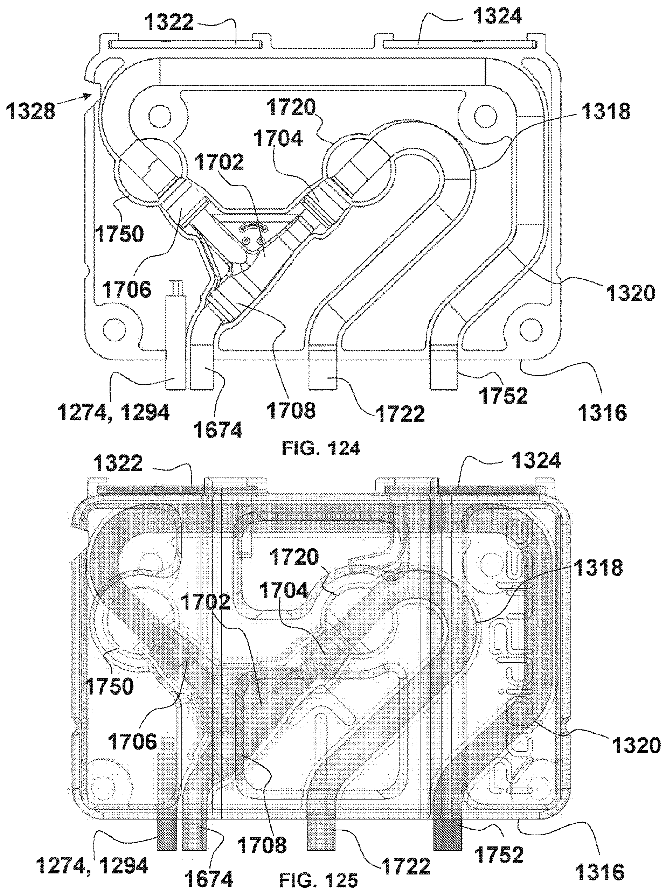

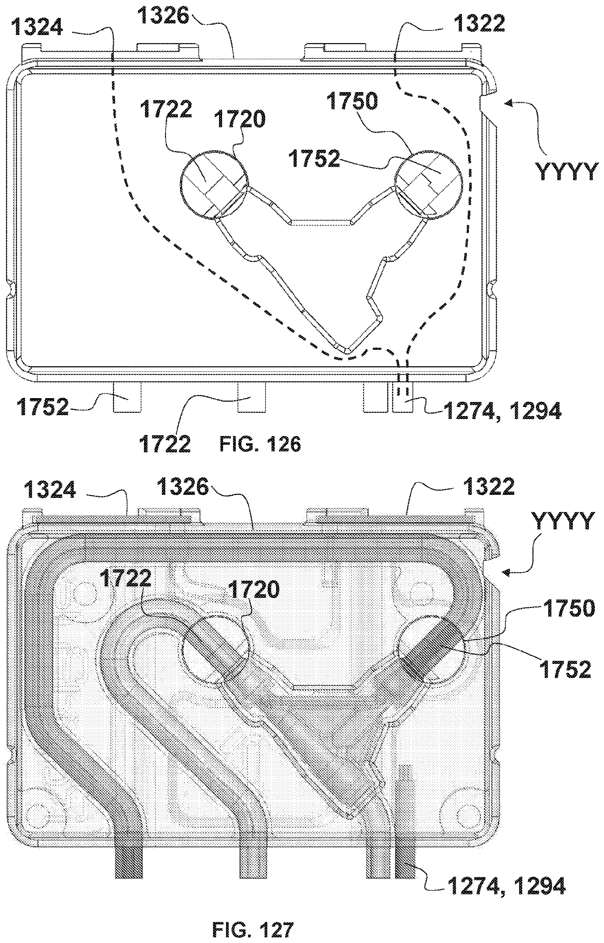

Another embodiment of a cassette connection assembly and cassette of the systems, devices, and methods is shown in FIGS. 119 to 128. This cassette assembly 1310 is sized and shaped to fit within a cassette slot 1106 within the upper front wall 1104 of the vacuum console 1000 shown, for example, in FIGS. 88 and 90, and in the cross-sectional views of FIGS. 119 to 121. To permit ease of entry of the cassette assembly 1310 into the cassette slot 1106, the cassette slot 1106 is disposed above the vacuum inlet 1212 of the lid 1210. The embodiment of the cassette assembly 1310 has a clamshell set of upper and lower cassette body parts 1312, 1314 that define various tracks therein to accommodate the first and second tubing whips 1722, 1752, a downstream junction 1702, an extension line 1674, and the vacuum and vent valve actuators 1620, 1650, which actuators 1620, 1650 form, with the first and second tubing whips 1722, 1752, each of the vacuum and vent valves of the system 1000. As shown in FIGS. 123 to 126, the first tubing whip 1722 (forming the vacuum line) enters a front face 1316 of the cassette assembly 1310, traverses a vacuum track 1318, and fluidically seals to a vacuum line entry port 1704 of the downstream junction 1702. Between the front face 1316 and the vacuum line entry port 1704, the cassette assembly 1310 defines a vacuum valve area 1720 into which the vacuum valve actuator 1620 is sized to project and, by interacting with the first tubing whip 1722 of the vacuum line, selectively opens, closes, partially closes, and/or over pushes the vacuum valve of the system 1000. The second tubing whip 1752 (forming the vent line) enters the front face 1316 of the cassette assembly 1310, traverses a vent track 1320, and fluidically seals to a vent line entry port 1706 of the downstream junction 1702. Between the front face 1316 and the vent line entry port 1706, the cassette assembly 1310 defines a vent valve area 1750 into which the vent valve actuator 1650 is sized to project and, by interacting with the second tubing whip 1752 of the vent line, selectively opens, closes, partially closes, and/or over pushes the vent valve of the system 1000. The exemplary embodiment of the downstream junction 1702 is a T-conduit defining the fluidically communicating and sealed connection of the first and second whips 1722, 1752 to the extension line 1674.

The configuration of the easily removable cassette assembly 1310 simplifies and reduces the time required to set up the system 1000, and this ease of setup is favorable for stroke procedures, where a concerted effort is taken to reduce procedure time. The tube set, comprising the junction 1702, the first and second tubing whips 1722, 1752, and the extension line 1674, is referred to collectively as a manifold and the manifold can be placed inside the cassette body (comprising the upper and lower cassette body parts 1312, 1314 in a two-part clam shell embodiment). The cassette body can also be a single piece or it can be over molded to the manifold. The cassette body is received by the cassette slot 1106 in the console 1100 to quickly connect the tube set to the console 1100 and accurately and repeatedly align the tube whips 1722, 1752 with the valve actuators 1620, 1650. In another embodiment, the cassette assembly 1310 can be placed onto a post with a rotary locking knob (e.g., as in FIGS.

57 to 71). In yet another non-illustrated embodiment, the cassette assembly 1310 can be placed in an open part or drawer of the console 1000 and be secured in place by a door with a latch. In an embodiment of the whips 1722, 1752, connection of the vacuum whip 1722 to the vacuum source 610 and/or connection of the vent whip 1752 to the vent fluid source 640 is done separately by a user. In another embodiment, both whips 1722, 1752 are connected done automatically when inserting the cassette assembly 1310 into the console 1100.

The cassette assembly 1310 not only houses the junction 1702 and distal sections of each of the first and second whips 1722, 1752, it also contains electrical connections for communication between the pendants 1270, 1290 and the vacuum console 1100. In the configuration of the cassette assembly 710 of FIGS. 68 to 71, the electrical connections 718 are on a bottom surface thereof. In the configuration of the cassette assembly 1310 of FIGS. 119 to 128, the electrical connections 1322, 1324, comprise a pair of plates on a rear surface 1326 thereof opposite the front face 1316. Inside the cassette slot 1106 are respective electrical leads that electrically connect with the electrical connections 1322, 1324 when the cassette assembly 1310 is releasably inserted into the cassette slot 1106. Accordingly, the cassette assembly 1310 is provided with a releasable lock part 1328 that is, in some embodiments, at least one orifice or indentation against which a detent or second lock part within the cassette slot 1106 interacts. For example, the console 1100 has an ejection button (electronic or mechanical) to move the detent and, thereby, release the cassette assembly 1310. The lock parts act as a retention system preventing the cassette assembly 1310 from being removed from within the cassette slot 1106 prematurely or when not specifically desired or from being moved improperly within the cassette slot 1106. The detent can be a ball detent or a latch, for example, as a mechanical measure for detecting and insuring proper seating connection. Alternatively, or additionally, a microswitch can be present in the cassette slot 1106 that detects proper seating of the cassette assembly 1310 and indicates to the user a proper seating state, for example, with a green "cartridge loaded" indicator, such as an LED. After the vacuum pump 80 has been used and a differential pressure may exist within the tube set, the system can include a vacuum dump valve to relieve vacuum pressure before releasing the cassette assembly 1310 from the console 1100. An alternative to a cartridge eject button is that there is no eject button and the cassette assembly 1310 is held within the cassette slot 1106 until power is turned off.

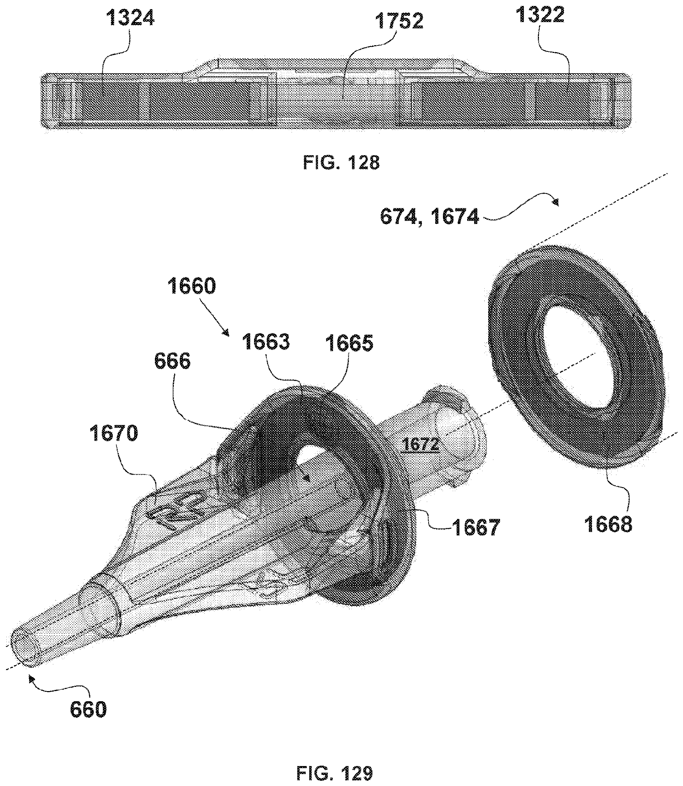

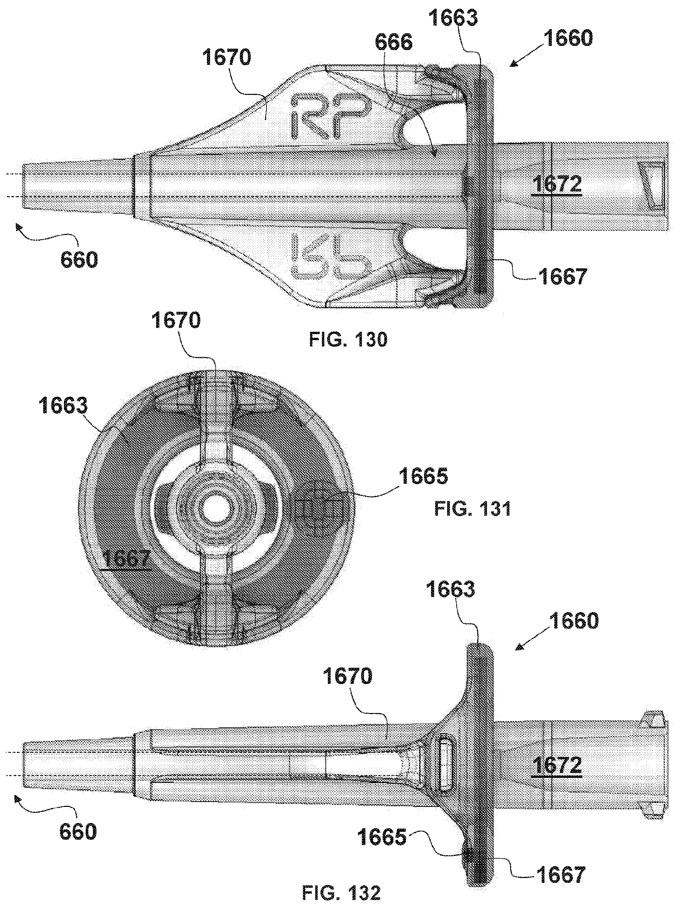

Described herein are various embodiments of catheter identification. One exemplary embodiment is shown in FIG. 54 and described with the manifold 630 of the vacuum valve 620, the ROAR ID sub-assembly 680, and the fitting 672. Another embodiment of a catheter identification assembly utilizing RFID is shown in FIGS. 129 to 135. A ROAR identification (ID) sub-assembly 1660 is secured to or is integral with a proximal manifold connector assembly 1670 at the proximal end 666 of a ROAR catheter 660, which connector assembly also can be referred to as a catheter hub. The ROAR ID sub-assembly 1660 includes a passive RF object tag 1663. The object tag 1663 has an RFID chip 1665 operatively connected to an antenna 1667 (e.g., a PCB antenna). The ROAR ID sub-assembly 1660 can be constructed by any one or more of over molded plastic, resin encapsulation, epoxy encapsulation, and/or polyurethane encapsulation completely encapsulate the RFID chip 1665 and the antenna 1667.

When an RFID transceiver antenna 1668 queries another RFID antenna (such as antenna 1667) within range, the hardware and/or software (e.g., the controller 700) can determine a unique ID of the object tag 1663 and, thereby, determine if a catheter to which the catheter hub 1670 is connected is a ROAR catheter. From this identification, the controller 700 can run the system 1000 in an optimal way for the particular catheter, for example, using ROAR aspiration if the catheter is a ROAR catheter 600. In some embodiments, the distal end of the extension lines 674, 1674 or the distal catheter connectors 1278, 1298 are provided with a transceiving antenna 1668 (shown in dashed lines in FIG. 113 and diagrammatically in FIG. 116) that is electrically connected to the controller 700 for communicating with any RFID object tag 1663 that inductively couples to the transceiving antenna 1668. A significant benefit of the configuration of the ROAR ID sub-assembly 1660 and how it is positioned perpendicularly on the catheter hub 1670 is that it creates clear visibility for a user to see through and inside a meniscus window 1672 present at the proximal end of the catheter hub 1670. This meniscus window 1672 allows the user/surgeon to determine if a bubble entered the aspiration lumen when the catheter hub 1670 was connected to the distal end of the extension line 674 or the distal catheter connector 1278, 1298 of the pendant 1270, 1290.

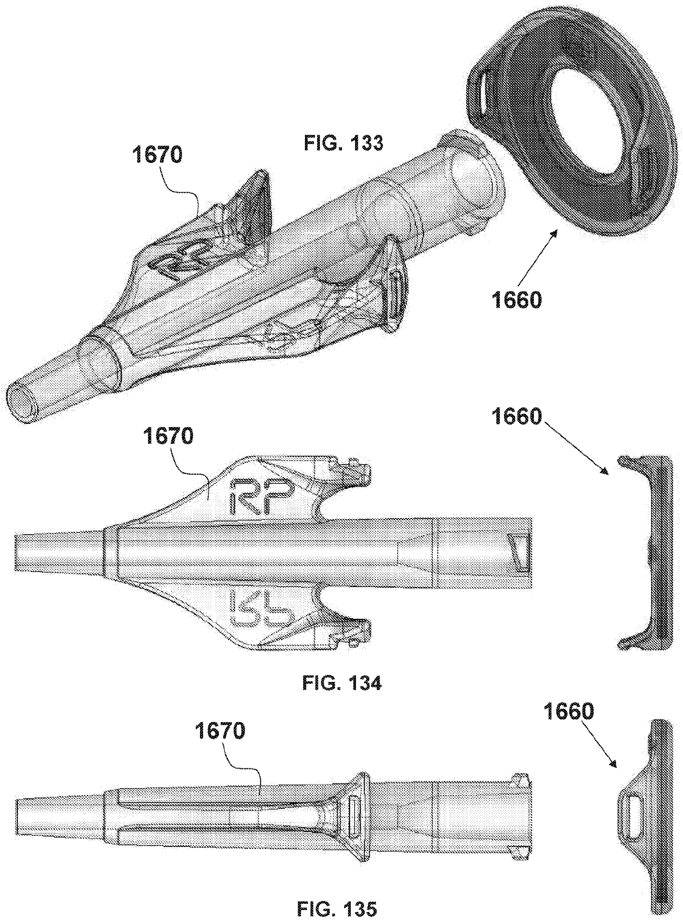

FIGS. 133 to 135 show one connection process for securing the ROAR ID sub-assembly 1660 to the catheter hub 1670. With the shape and form shown, the ROAR ID sub-assembly 1660 is clipped on to the catheter hub 1670 and is then permanently attached, for example, with a one-way barb, an adhesive, a press fit, and/or an ultrasonic weld, to name a few.

A method for utilizing a ROAR aspiration catheter of the aspiration thrombectomy system 1000 includes opening a sterile package of the tube set (e.g., the first and second tube whips 1722, 1752 and the extension line 1674) in a sterile field. Where each cassette body 1312, 1314 is packaged together along with the tube set, the connection area of the tube set (e.g., at the junction 1702) is either already inserted within a cassette body 1312, 1314 or is separate and, therefore, is installed within a cassette body 1312, 1314. The vacuum console 1100 is present outside the sterile field. Accordingly, a cassette body 1312, 1314 is handed out of the sterile field to a non-sterile operator to be inserted into the cassette slot 1106. A suction connector of the tube set (the proximal end of the first tubing whip 1722) is attached to the vacuum inlet 1212 of the lid 1210 of the vacuum canister 1200. A vent connector of the tube set (the proximal end of the second tubing whip 1752, e.g., in the form of a Vent IV Spike) is connected to a sterile saline bag removably secured to the hanger 1132 on the vacuum console's IV pole 1130. A non-sterile operator presses the power button (e.g., the user interface 1116) to power on the vacuum pump, which performs a cartridge detection sequence. Upon a positive result, the vacuum canister 1200 is brought to an acceptable minimum vacuum pressure level. All user interfaces (e.g., buttons, LED lights) on console 1100 or pendants 1270, 1290 become active. As there are different configurations for the pendant, some of which are illustrated 1270, 1290 and some of which are not, performance of a tube set purge is explained below for a few of the exemplary embodiments of the pendant.

In an embodiment where the pendant has three buttons (e.g., FIG. 56), to perform a purge of the tube set, the middle "PURGE" button 678 is pressed. The surgeon or operator waits approximately eight seconds for the system 1000 to send purge fluid distally through the tube set. The system 1000 automatically shuts off flow when the preselected time is complete or the system 1000 converts the purge to a slow positive drip rate. Alternatively, the operator can be permitted to wait until the operator subjectively determines that the tube set is completely purged and that no visible bubbles are present and, when that state occurs, the operator presses the PURGE button 678 again to either shut off flow or convert the flow to a slow positive drip rate. Alternatively, the operate holds the PURGE button 678 down until the tube set is completely purged (subjective) and no visible bubbles are present. To shut off flow or convert the purge flow to a slow drip rate, the operator releases the PURGE button.

In an embodiment where the pendant 1270 has two buttons with one button having three drip states as in FIG. 112, for example, to perform a purge of the tube set, the bottom PURGE button 1282 is pressed. The operator waits approximately eight seconds for system 100 to send purge fluid distally through the tube set. The system 1000 automatically shuts off flow when the preselected time is complete or the system 1000 converts the purge to a slow positive drip rate. Alternatively, the operator can be permitted to wait until the operator subjectively determines that the tube set is completely purged and that no visible bubbles are present and, when that state occurs, the operator presses the PURGE button 1282 again to convert the flow to a slow positive drip rate (middle drip indicator) or presses the PURGE button 1282 a second time to shut off flow (bottom no drip indicator). Alternatively, the operator simply holds the PURGE button 1282 down until the tube set is completely purged (subjective) and no visible bubbles are present.

In an embodiment where the pendant 1270 has two buttons but no indicators like, e.g., FIG. 112, to perform a purge of the tube set, the PURGE button is pressed. The operator waits approximately eight seconds for system to purge fluid distally through the tube set. The system 1000 automatically converts to a slow positive drip rate when complete. Alternatively, the operator can be permitted to wait until tube set is completely purged and no visible bubbles are present (subjective) before pressing the PURGE button again to convert to a slow positive drip rate.

In an embodiment where the pendant 1290 has one button 1300 and a slider 1302 (e.g., FIG. 116), to perform a purge of the tube set the operator moves the slider 1302 to introduce a flow of fluid distally through the tube set at a preferred variable flowrate and waits until the tube set is completely purged and no visible bubbles are present (subjective). Then, the operator moves the slider 1302 to a no-flow/off position or to convert to a slow positive drip rate. Alternatively, where the slider 1302 is spring loaded, the operator puts the slider in a spring-loaded section of the slider travel, which initiates a high-flowrate, timed purge of the tube set (e.g., approximately eight seconds). At the end of the period, the flow is either automatically shut off or is converted to a slow positive drip rate.

To operate the system described herein, e.g., the vacuum console 1100, with the pendant 1330 of FIGS. 136 and 137, the user places the cassette assembly 1310 within the cassette slot 1106 of the console 1100 and turns on the console 1100 by pressing the power button of the user interface 1116. A member of the surgical team in the sterile field presses the purge actuator 1342 to cause fluid to fill the vacuum extension line 1336. In an embodiment, the purge actuator 1342 pulses until the line 1336 is free of air and then lights solid. Another press of the purge actuator 1342 causes a manual stop of the vacuum extension line purging. A catheter is connected to the distal catheter connector 1338. If the catheter is a ROAR catheter 660, then a ROAR catheter indicator 1344 signals to the surgical staff that a ROAR catheter 660 is present and, therefore, ROAR operation of the catheter 660 as set forth herein is enabled (e.g., by lighting up the ROAR catheter indicator 1344). If a ROAR catheter 660 is not present, a different signal or no signal is indicated to the user. For example, the ROAR catheter indicator 1344 remains dark. After connection of the catheter, if ROAR operation is enabled, a pressing of the aspiration button 1340 causes ROAR operation with the catheter 660. ROAR operation can continue for a given amount of time, for example, between approximately 5 and approximately 20 seconds, or between approximately 10 seconds and approximately 17 seconds, in particular, for approximately 15 seconds. If there is no preset time or if the surgeon wishes to stop ROAR operation sooner, a second press of the aspiration button 1340 will turn ROAR operation off to cease all vacuum by the console 1100. If ROAR operation is disabled, a pressing of the aspiration button 1340 causes continuous vacuum operation with the catheter.

At this point in a thrombectomy, for example, treating a clot in the brain, a distal end of a ROAR catheter 660 has been tracked up to and is adjacent a proximal side of a clot. In a typical thrombectomy procedure, at least one device is within the ROAR catheter 660, such as a microcatheter, a guidewire, and/or a stentriever, to have guided the ROAR catheter 660 to the clot. In such a procedure, a Rotating Hemostasis Valve (RHV) is fluidically sealed at the proximal end thereof of the ROAR catheter 660 and the internal device(s) passes through a port of the RHV. The internal device(s) is(are) removed from within the lumen of the ROAR catheter 660 while the RHV is supplied with an open flush drip line connected to a side port thereof. To ensure that no air enters the lumen of the ROAR catheter 660 while such removal occurs, the drip remains and the internal device(s) is(are) slowly withdrawn out of the ROAR catheter 660. The RHV is then removed from the proximal end of the ROAR catheter 660. The proximal end of the ROAR catheter 660 is connected to the distal end of the extension line 1674 using a meniscus-to-meniscus technique that ensures no bubbles are introduced into either lumen during this connection. Upon detection of a ROAR enabled catheter, the PURGE is automatically transitioned to the OFF-flow state, regardless of what state the user had selected during connection (ex: full flow, partial flow, drip), ensuring fluid is not pushed distally into the vasculature.

In an embodiment where the ROAR catheter 660 has an identification tag/identifier 663 that identifies the ROAR catheter 660 (or any other non-ROAR catheter) to the system 1000 upon connection to the system 1000, the system 1000 changes the valve timing parameters accordingly and automatically. The valve timing parameters can be any one or more of:
- stored in the controller 700 and associated with that particular ROAR catheter 660;
- communicated to the controller 700 upon connection to the system 1000 and associated with that particular ROAR catheter 660; and
- indicated to the system 1000 that a ROAR-enabled catheter is connected to the system 1000 and the controller 700 uses the stored valve timing parameters.

Upon identification and/or authentication of a ROAR catheter 600, the ROAR/ASPIRATION/GO button (e.g., on the Pendant) is enabled (and the RP light illuminates) to perform ROAR pulsed aspiration when the button 678, 1280, 1300 is next pressed. Upon actuation of the button 678, 1280, 1300, ROAR aspiration is initiated, the distal end of the ROAR catheter 660 being adjacent the proximal side of the clot. The surgeon slowly advances the ROAR catheter 660 distally until resistance is met - indicating that the distal end abuts the clot. The surgeon waits a prescribed time (e.g., 5-sec, 15-sec, 20-sec, 30-sec, 1-min) before pressing the button 678, 1280, 1300 again to convert from ROAR aspiration to continuous aspiration. Alternatively, the surgeon slowly advances the ROAR catheter 660 distally until resistance is met - indicating that the distal end abuts the clot. The surgeon waits until the system 1000 automatically converts from ROAR aspiration to continuous aspiration after a prescribed time (e.g., 5-sec, 15-sec, 20-sec, 30-sec, 1-min) before or based upon sensor feedback (e.g., pressure, flowrate, temperature, sound, motion, some of which are described herein). Alternatively, the surgeon continues to hold the button 678, 1280, 1300 pressed while slowly advancing the catheter distally until resistance is met. The surgeon waits a prescribed time (e.g., 5-sec, 15-sec, 20-sec, 30-sec, 1-min) before releasing the button 678, 1280, 1300 to convert from ROAR aspiration to continuous aspiration.

When pulsed aspiration ends and continuous aspiration begins, the primary way to determine if the procedure was successful is to look at blood flow at or near where the procedure took place. In particular, the surgeon checks two points in the vessel to determine flow and, thereby, access the result/status of the procedure just conducted. The first point is located at the most distal location where the ROAR catheter 660 traveled. The second point is located proximal to the clot at a site where free flow existed prior to conducting pulsed aspiration. In summary, if free flow is detected at the first point, then the procedure was successful and the catheter can be removed. Many times during thrombectomies the surgeon does not see flow at the clot site. Therefore, the surgeon waits for a time and then slowly retracts the distal end of the catheter to a point where treatment was started or even further proximal -- where open flow occurred before advancing to the clot. If there is open flow where aspiration started, it is now safe to perform an angiogram with contrast and to conclude the procedure. If there still is no flow, then it can be determined that the catheter is corked by the clot and the surgeon needs to pull out the clot manually by retracting the catheter. From this, a flow assessment and decision tree can be created.

To perform flow assessment at the distal-most position where the ROAR catheter 660 traveled in the vessel either the surgeon assesses flow in the tube set or vacuum canister 1200 visually or the system 1000 assesses flow in the tube set or vacuum canister 1200 using sensors (e.g., pressure, flowrate, visual, motion), providing feedback to the surgeon as to the flow state within the aspiration lumen of the system 1000. There are two flow options that can exist at this point. If no flow is present, the surgeon waits for a period of time (e.g., 5-sec, 15-sec, 20-sec, 30-sec, 1-min, 1.5-min, 2-min, 2.5-min, 3-min, 5-min) while continuous aspiration occurs before slowly retracting the distal end of the ROAR catheter 660 to, at, or slightly proximal of the original clot face location. If free flow is present, the surgeon slowly retracts the distal end to, at, or slightly proximal of the original clot face location. A second assessment is then performed. More particularly, flow is assessed at the proximal position where aspiration started. The surgeon assesses flow in the tube set or vacuum canister visually or the system 100 assesses flow in the tube set or vacuum canister 1200 using sensors (e.g., pressure, flowrate, visual, motion), providing feedback to the surgeon as to the flow state within the aspiration lumen of the system 1000. Again, there are two flow options that can exist at this proximal position. If no flow is present, the surgeon slowly retracts the ROAR catheter 660 out of the patient completely (possibly into the guide catheter if present); this action manually pulls out a clot that is corked to the distal end of the ROAR catheter 660. On the other hand, if flow is present, the surgeon can press the button 678, 1280, 1300 again (or let go of the button) to shut off all aspiration. The surgeon is then able to perform a contrast run in this proximal location through the ROAR catheter 660 if desired.

The present technology is illustrated, for example, according to various aspects described below. Various examples of aspects of the present technology are described as numbered examples (1, 2, 3, etc.) for convenience. These are provided as examples and do not limit the present technology. It is noted that any of the dependent examples may be combined in any combination and placed into a respective independent example. The other examples can be presented in a similar manner.
1. An aspiration system for removing thromboembolic material from a blood vessel using a catheter system including an aspiration catheter having a proximal portion, a distal end and a lumen, the aspiration system comprising:
   a vacuum source configured to supply vacuum to the catheter system, wherein the vacuum source has a maximum steady state vacuum level;
   a vacuum valve fluidically coupled to the vacuum source and configured to be fluidically coupled to the proximal portion of the aspiration catheter;
   a vent valve configured to be fluidically coupled to (a) a vent fluid source containing a vent fluid and (b) the proximal portion of the aspiration catheter; and
   a controller configured to cyclically open and close the vacuum valve and the vent valve in a predetermined cycle based on the catheter system including -
      (a) a vacuum phase in which the vacuum valve is open and the vent valve is closed such that when thromboembolic material blocks the distal end of the aspiration catheter a super-vacuum level at the distal end of the aspiration catheter occurs for a temporary super-vacuum period before reducing to the maximum steady state vacuum level of the vacuum source, wherein the super-vacuum level is greater than the maximum steady state vacuum level of the vacuum source,
      (b) a vent phase in which the vent valve is open and the vacuum valve is closed to impart a distal shift of a fluid column in the lumen of the aspiration catheter, wherein the vent phase is timed start before the temporary super-vacuum reduces to the maximum steady state vacuum of the vacuum source, and
      (c) the vent phase is terminated and the vacuum phase is reapplied thereby quelling the distal shift of the fluid column such that an exit flow out from the distal end of the aspiration catheter during each cycle is in a range from at least approximately zero to a limited predetermined volume of liquid whereby the reapplied vacuum recaptures the thrombus before the thrombus is uncontrollably ejected from the distal end of the aspiration catheter.
2. The aspiration system of example 1, further comprising a vent fluid source, and wherein:
   the vent fluid source is configured to provide vent fluid at a pressure greater than body pressure; and
   the controller is configured to cyclically open and close the vacuum valve and the vent valve to alternate between a negative pressure and a positive pressure at the distal end of the aspiration catheter during each cycle.
3. The aspiration system of any of examples 1-2 wherein the controller is configured to cyclically open and close the vacuum valve and the vent valve such that movement of the fluid column at the distal end of the aspiration catheter is a positive amount of exit flow limited to less than 20 microliters before fluid is drawn back into the catheter lumen.
4. The aspiration system of any of examples 1-3 wherein the controller is configured to cyclically open and close the vacuum valve and the vent valve in a cycle comprising a vacuum-only state in which the vacuum valve is open and the vent valve is closed, an off-off state in which the vacuum valve is closed while the vent valve is closed for a controlled period of time, and a vent-only state in which the vacuum valve is closed and the vent valve is open.
5. The aspiration system of example 4 wherein the vacuum-only state is 40-60% of the cycle, the vent-only state is approximately 20-25% of the cycle, and the off-off state is the remainder of the cycle.
6. The aspiration system of any of examples 1-5 wherein:
   the controller is configured to cyclically open and close the vacuum valve and the vent valve in a cycle comprising (a) a first off-off state in which the vacuum valve is closed while the vent valve is closed, (b) a vacuum-only state following the first off-off state in which the vacuum valve is open while the vent valve is closed, (c) a second off-off state following the vacuum-only state in which the vacuum valve is closed while the vent valve is closed, and (d) a vent-only state following the second off-off state in which the vacuum valve is closed while the vent valve is open; and
   the cycle is repeated.
7. The aspiration system of any of examples 1-6 wherein the cycle is repeated at a frequency of 6 Hz to 16 Hz.
8. The aspiration system of example 7 wherein during each cycle a pressure differential at the distal end of the aspiration catheter is at least approximately 15 inHg to 25 inHg in a time of not greater than 20 ms to 50 ms.
9. The aspiration system of example 8 wherein the cycle is repeated at a frequency of 8 Hz to 12 Hz and during each cycle the pressure differential at the distal end of the aspiration catheter is at least approximately 20 inHg in a time of not greater than approximately 20 ms.
10. The aspiration system of any of examples 1-9 wherein the controller is configured to cyclically open and close the vacuum valve and the vent valve in a predetermined cycle comprising:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists; and
   initiating the vent phase by opening the vent valve such that vent fluid is introduced to the aspiration catheter while the super-vacuum level exists at the distal end of the aspiration catheter.
11. The aspiration system of example 10 wherein initiating the vacuum phase by opening the vacuum valve while the vent valve is closed comprises rapidly increasing the vacuum to the super-vacuum level in no greater than approximately 10 ms.
12. The aspiration system of example 11 wherein the cycle is repeated at a frequency of 6 Hz to 16 Hz.
13. The aspiration system of example 12 wherein during each cycle a pressure differential at the distal end of the aspiration catheter is at least approximately 15 inHg to 25 inHg in a time of not greater than 20 ms to 50 ms.
14. The aspiration system of example 11 wherein the cycle is repeated at a frequency of 8 Hz to 12 Hz and during each cycle the pressure differential at the distal end of the aspiration catheter is at least approximately 20 inHg in a time of not greater than approximately 20 ms.
15. The aspiration system of any of examples 1-9 wherein the controller is configured to cyclically open and close the vacuum valve and the vent valve in a predetermined cycle comprising:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists;
   a second double-off state in which the vacuum valve is off while the vent valve is off; and
   initiating the vent phase by opening the vent valve such that vent fluid is introduced to the aspiration catheter while the super-vacuum level exists at the distal end of the aspiration catheter.
16. The aspiration system of example 15 wherein initiating the vacuum phase by opening the vacuum valve while the vent valve is closed comprises rapidly increasing the vacuum to the super-vacuum level in no greater than approximately 10 ms.
17. The aspiration system of example 16 wherein the cycle is repeated at a frequency of 6 Hz to 16 Hz.
18. The aspiration system of example 17 wherein during each cycle a pressure differential at the distal end of the aspiration catheter is at least approximately 15 inHg to 25 inHg in a time of not greater than 20 ms to 50 ms.
19. The aspiration system of example 15 wherein the cycle is repeated at a frequency of 8 Hz to 12 Hz and during each cycle the pressure differential at the distal end of the aspiration catheter is at least approximately 20 inHg in a time of not greater than approximately 20 ms.
20. A method of removing thromboembolic material from a blood vessel, comprising:
   positioning a distal end of an aspiration catheter at least proximate to a mass of thromboembolic material in a blood vessel;
   cyclically opening and closing a vacuum valve in fluid communication with a vacuum source and the aspiration catheter and a vent valve in fluid communication with a vent fluid and the aspiration catheter in a predetermined cycle based on the aspiration catheter including -
      (a) a vacuum phase in which the vacuum valve is open and the vent valve is closed such that when thromboembolic material blocks the distal end of the aspiration catheter a super-vacuum level at the distal end of the aspiration catheter occurs for a temporary super-vacuum period before reducing to the maximum steady state vacuum level of the vacuum source, wherein the super-vacuum level is greater than the maximum steady state vacuum level of the vacuum source,
      (b) a vent phase in which the vent valve is open and the vacuum valve is closed to impart a distal shift of a fluid column in the lumen of the aspiration catheter, wherein the vent phase is timed to start before the temporary super-vacuum period reduces to the maximum steady state vacuum of the vacuum source, and
      (c) the vent phase is terminated and the vacuum phase is reapplied thereby quelling the distal shift of the fluid column such that an exit flow out from the distal end of the aspiration catheter during each cycle is in a range from at least approximately zero to a limited predetermined volume of liquid whereby the reapplied vacuum recaptures the thrombus before the thrombus is uncontrollably ejected from the distal end of the aspiration catheter.
21. The method of example 20 wherein the predetermined cycle further comprises:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists; and
   initiating the vent phase by opening the vent valve such that vent fluid is introduced to the aspiration catheter while the super-vacuum level exists at the distal end of the aspiration catheter.
22. The method of any of examples 20-21 wherein initiating the vacuum phase by opening the vacuum valve while the vent valve is closed comprises rapidly increases the vacuum to the super-vacuum level in no greater than approximately 10 ms.
23. The method of any of examples 20-21 wherein the cycle is repeated at a frequency of 6 Hz to 16 Hz.
24. The method of example 23 wherein during each cycle a pressure differential at the distal end of the aspiration catheter is at least approximately 15 inHg to 25 inHg in a time of not greater than 20 ms to 50 ms.
25. The method of example 23 wherein the cycle is repeated at a frequency of 8 Hz to 12 Hz and during each cycle the pressure differential at the distal end of the aspiration catheter is at least approximately 20 inHg in a time of not greater than approximately 20 ms.
26. The method of any of examples 20-25 wherein the predetermined cycle further comprises:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists;
   a second double-off state in which the vacuum valve is off while the vent valve is off; and
   initiating the vent phase by opening the vent valve such that vent fluid is introduced to the aspiration catheter while the super-vacuum level exists at the distal end of the aspiration catheter.
27. The method of example 26 wherein initiating the vacuum phase by opening the vacuum valve while the vent valve is closed comprises rapidly increasing the vacuum to the super-vacuum level in no greater than approximately 10 ms.
28. The method of example 27 wherein the cycle is repeated at a frequency of 6 Hz to 16 Hz.
29. The method of example 28 wherein during each cycle a pressure differential at the distal end of the aspiration catheter is at least approximately 15 inHg to 25 inHg in a time of not greater than 20 ms to 50 ms.
30. The method of example 27 wherein the cycle is repeated at a frequency of 8 Hz to 12 Hz and during each cycle the pressure differential at the distal end of the aspiration catheter is at least approximately 20 inHg in a time of not greater than approximately 20 ms.
31. An aspiration system for removing thromboembolic material from a blood vessel, comprising:
   a catheter system comprising an aspiration catheter including a proximal portion, a distal portion, and a lumen;
   a console including vacuum source, a vacuum valve fluidically coupled to the vacuum source, a vent valve configured to be fluidically coupled to a vent fluid source containing a vent fluid, and a controller configured to cyclically open and close the vacuum valve and the vent valve in a predetermined cycle based on the catheter system; and
   a remote-control pendant coupled to the console and the catheter system, the wired pending including -
      (a) a handle having at least one remote control actuator, and
      (b) a vacuum extension line configured to be fluidically coupled to the aspiration catheter, the vacuum valve, and the vent valve, wherein the vacuum extension line extends through the handle such that the handled remains at a longitudinal location along the vacuum extension line while the vacuum extension line can rotate relative to the handle.

It is noted that various individual features of the inventive processes and systems may be described only in one exemplary embodiment herein. The particular choice for description herein with regard to a single exemplary embodiment is not to be taken as a limitation that the particular feature is only applicable to the embodiment in which it is described. All features described herein are equally applicable to, additive, or interchangeable with any or all of the other exemplary embodiments described herein and in any combination or grouping or arrangement. In particular, use of a single reference numeral herein to illustrate, define, or describe a particular feature does not mean that the feature cannot be associated or equated to another feature in another drawing figure or description. Further, where two or more reference numerals are used in the figures or in the drawings, this should not be construed as being limited to only those embodiments or features, they are equally applicable to similar features or not a reference numeral is used or another reference numeral is omitted.

The foregoing description and accompanying drawings illustrate the principles, exemplary embodiments, and modes of operation of the systems, apparatuses, and methods. However, the systems, apparatuses, and methods should not be construed as being limited to the particular embodiments discussed above. Additional variations of the embodiments discussed above will be appreciated by those skilled in the art and the above-described embodiments should be regarded as illustrative rather than restrictive. Accordingly, it should be appreciated that variations to those embodiments can be made by those skilled in the art without departing from the scope of the systems, apparatuses, and methods as defined by the following examples.
One non-limiting embodiment of the present invention will now be described by way of reference to the following clauses in which:
1. An aspiration system for removing thromboembolic material from a blood vessel using a catheter system including an aspiration catheter having a proximal portion, a distal end and a lumen, the aspiration system comprising:
   a vacuum source configured to supply vacuum to the catheter system, wherein the vacuum source has a maximum steady state vacuum level;
   a vacuum valve fluidically coupled to the vacuum source and configured to be fluidically coupled to the proximal portion of the aspiration catheter;
   a vent valve configured to be fluidically coupled to (a) a vent fluid source containing a vent fluid and (b) the proximal portion of the aspiration catheter; and
   a controller configured to cyclically open and close the vacuum valve and the vent valve in a predetermined cycle based on the catheter system including -
      (a) a vacuum phase in which the vacuum valve is open and the vent valve is closed such that when thromboembolic material blocks the distal end of the aspiration catheter a super-vacuum level at the distal end of the aspiration catheter occurs for a temporary super-vacuum period before reducing to the maximum steady state vacuum level of the vacuum source, wherein the super-vacuum level is greater than the maximum steady state vacuum level of the vacuum source,
      (b) a vent phase in which the vent valve is open and the vacuum valve is closed to impart a distal shift of a fluid column in the lumen of the aspiration catheter, wherein the vent phase is timed start before the temporary super-vacuum reduces to the maximum steady state vacuum
         of the vacuum source, and
      (c) the vent phase is terminated and the vacuum phase is reapplied thereby quelling the distal shift of the fluid column such that an exit flow out from the distal end of the aspiration catheter during each cycle is in a range from at least approximately zero to a limited predetermined volume
         of liquid whereby the reapplied vacuum recaptures the thrombus before the thrombus is uncontrollably ejected from the distal end of the aspiration catheter.
2. The aspiration system of clause 1, further comprising a vent fluid source, and wherein:
   the vent fluid source is configured to provide vent fluid at a pressure greater than
   body pressure; and
      the controller is configured to cyclically open and close the vacuum valve and the vent valve to alternate between a negative pressure and a positive pressure at the distal end of the aspiration catheter during each cycle.
3. The aspiration system of clause 2 wherein the controller is configured to cyclically open and close the vacuum valve and the vent valve such that movement of the fluid column at the distal end of the aspiration catheter is a positive amount of exit flow limited to less than 20 microliters before fluid is drawn back into the catheter lumen.
4. The aspiration system of clause 1 wherein the controller is configured to cyclically open and close the vacuum valve and the vent valve in a cycle comprising a vacuum-only state in which the vacuum valve is open and the vent valve is closed, an off-off state in which the vacuum valve is closed while the vent valve is closed for a controlled period of time, and a vent-only state in which the vacuum valve is closed and the vent valve is open.
5. The aspiration system of clause 4 wherein the vacuum-only state is 40-60% of the cycle, the vent-only state is approximately 20-25% of the cycle, and the off-off state is the remainder of the cycle.
6. The aspiration system of clause 1 wherein:
   the controller is configured to cyclically open and close the vacuum valve and the vent valve in a cycle comprising (a) a first off-off state in which the vacuum valve is closed while the vent valve is closed, (b) a vacuum-only state following the first off-off state in which the vacuum valve is open while the vent valve is closed, (c) a second off-off state following the vacuum-only state in which the vacuum valve is closed while the vent valve is closed, and (d) a vent-only state following the second off-off state in which the vacuum valve is closed while the vent valve is open; and
   the cycle is repeated.
7. The aspiration system of clause 1 wherein the cycle is repeated at a frequency of 6 Hz to 16 Hz.
8. The aspiration system of clause 7 wherein during each cycle a pressure differential at the distal end of the aspiration catheter is at least approximately 15 inHg to 25 inHg in a time of not greater than 20 ms to 50 ms.
9. The aspiration system of clause 8 wherein the cycle is repeated at a frequency of 8 Hz to 12 Hz and during each cycle the pressure differential at the distal end of the aspiration catheter is at least approximately 20 inHg in a time of not greater than approximately 20 ms.
10. The aspiration system of clause 1 wherein the controller is configured to cyclically open and close the vacuum valve and the vent valve in a predetermined cycle comprising:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists; and
   initiating the vent phase by opening the vent valve such that vent fluid is introduced to the aspiration catheter while the super-vacuum level exists at the distal end of the aspiration catheter.
11. The aspiration system of clause 10 wherein initiating the vacuum phase by opening the vacuum valve while the vent valve is closed comprises rapidly increasing the vacuum to the super-vacuum level in no greater than approximately 10 ms.
12. The aspiration system of clause 11 wherein the cycle is repeated at a frequency of 6 Hz to 16 Hz.
13. The aspiration system of clause 12 wherein during each cycle a pressure differential at the distal end of the aspiration catheter is at least approximately 15 inHg to 25 inHg in a time of not greater than 20 ms to 50 ms.
14. The aspiration system of clause 11 wherein the cycle is repeated at a frequency of 8 Hz to 12 Hz and during each cycle the pressure differential at the distal end of the aspiration catheter is at least approximately 20 inHg in a time of not greater than approximately 20 ms.
15. The aspiration system of clause 1 wherein the controller is configured to cyclically open and close the vacuum valve and the vent valve in a predetermined cycle comprising:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists;
   a second double-off state in which the vacuum valve is off while the vent valve is off; and
   initiating the vent phase by opening the vent valve such that vent fluid is introduced to the aspiration catheter while the super-vacuum level exists at the distal end of the aspiration catheter.
16. The aspiration system of clause 15 wherein initiating the vacuum phase by opening the vacuum valve while the vent valve is closed comprises rapidly increasing the vacuum to the super-vacuum level in no greater than approximately 10 ms.
17. The aspiration system of clause 16 wherein the cycle is repeated at a frequency of 6 Hz to 16 Hz.
18. The aspiration system of clause 17 wherein during each cycle a pressure differential at the distal end of the aspiration catheter is at least approximately 15 inHg to 25 inHg in a time of not greater than 20 ms to 50 ms.
19. The aspiration system of clause 15 wherein the cycle is repeated at a frequency of 8 Hz to 12 Hz and during each cycle the pressure differential at the distal end of the aspiration catheter is at least approximately 20 inHg in a time of not greater than approximately 20 ms.
20. A method of removing thromboembolic material from a blood vessel, comprising:
   positioning a distal end of an aspiration catheter at least proximate to a mass of thromboembolic material in a blood vessel;
   cyclically opening and closing a vacuum valve in fluid communication with a vacuum source and the aspiration catheter and a vent valve in fluid communication with a vent fluid and the aspiration catheter in a predetermined cycle based on the aspiration catheter including -
      (a) a vacuum phase in which the vacuum valve is open and the vent valve is closed such that when thromboembolic material blocks the distal end of the aspiration catheter a super-vacuum level at the distal end of the aspiration catheter occurs for a temporary super-vacuum period before reducing to the maximum steady state vacuum level of the vacuum source, wherein the super-vacuum level is greater than the maximum steady state vacuum level of the vacuum source,
      (b) a vent phase in which the vent valve is open and the vacuum valve is closed to impart a distal shift of a fluid column in the lumen of the aspiration catheter, wherein the vent phase is timed to start before the temporary super-vacuum period reduces to the maximum steady state vacuum of the vacuum source, and
      (c) the vent phase is terminated and the vacuum phase is reapplied thereby quelling the distal shift of the fluid column such that an exit flow out from the distal end of the aspiration catheter during each cycle is in a range from at least approximately zero to a limited predetermined volume of liquid whereby the reapplied vacuum recaptures the thrombus before the thrombus is uncontrollably ejected from the distal end of the aspiration catheter.
21. The method of clause 20 wherein the predetermined cycle further comprises:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists; and
   initiating the vent phase by opening the vent valve such that vent fluid is introduced to the aspiration catheter while the super-vacuum level exists at the distal end of the aspiration catheter.
22. The method of clause 21 wherein initiating the vacuum phase by opening the vacuum valve while the vent valve is closed comprises rapidly increases the vacuum to the super-vacuum level in no greater than approximately 10 ms.
23. The method of clause 21 wherein the cycle is repeated at a frequency of 6 Hz to 16 Hz.
24. The method of clause 23 wherein during each cycle a pressure differential at the distal end of the aspiration catheter is at least approximately 15 inHg to 25 inHg in a time of not greater than 20 ms to 50 ms.
25. The method of clause 22 wherein the cycle is repeated at a frequency of 8 Hz to 12 Hz and during each cycle the pressure differential at the distal end of the aspiration catheter is at least approximately 20 inHg in a time of not greater than approximately 20 ms.
26. The method of clause 20 wherein the predetermined cycle further comprises:
   a first double-off state in which the vacuum valve is off while the vent valve is off;
   initiating the vacuum phase by opening the vacuum valve while the vent valve is closed such that vacuum rapidly increases to the super-vacuum level in no greater than approximately 20 ms and terminating the vacuum phase by closing the vacuum valve while the super-vacuum level exists;
      a second double-off state in which the vacuum valve is off while the vent valve is off; and
   initiating the vent phase by opening the vent valve such that vent fluid is introduced to the aspiration catheter while the super-vacuum level exists at the distal end of the aspiration catheter.
27. The method of clause 26 wherein initiating the vacuum phase by opening the vacuum valve while the vent valve is closed comprises rapidly increasing the vacuum to the super-vacuum level in no greater than approximately 10 ms.
28. The method of clause 27 wherein the cycle is repeated at a frequency of 6 Hz to 16 Hz.
29. The method of clause 28 wherein during each cycle a pressure differential at the distal end of the aspiration catheter is at least approximately 15 inHg to 25 inHg in a time of not greater than 20 ms to 50 ms.
30. The method of clause 27 wherein the cycle is repeated at a frequency of 8 Hz to 12 Hz and during each cycle the pressure differential at the distal end of the aspiration catheter is at least approximately 20 inHg in a time of not greater than approximately 20 ms.
31. An aspiration system for removing thromboembolic material from a blood vessel, comprising:
   a catheter system comprising an aspiration catheter including a proximal portion, a distal portion, and a lumen;
   a console including vacuum source, a vacuum valve fluidically coupled to the vacuum source, a vent valve configured to be fluidically coupled to a vent fluid source containing a vent fluid, and a controller configured to cyclically open and close the vacuum valve and the vent valve in a predetermined cycle based on the catheter system; and
   a remote-control pendant coupled to the console and the catheter system, the wired pending including -
      (a) a handle having at least one remote control actuator, and
      (b) a vacuum extension line configured to be fluidically coupled to the aspiration catheter, the vacuum valve, and the vent valve, wherein the vacuum extension line extends through the handle such that the handled remains at a longitudinal location along the vacuum extension line while the vacuum extension line can rotate relative to the handle.

## Claims

1. A cassette assembly configured for use with an aspiration thrombectomy console, the cassette assembly comprising:
a cassette body;
a manifold positioned within the cassette body, wherein the manifold includes-
a first tubing whip,
a second tubing whip,
a junction of the first tubing whip and the second tubing whip, and
an extension line extending from the junction;
a vacuum valve area sized and shaped to receive a vacuum valve actuator of the aspiration thrombectomy console for interacting with the first tubing whip to selectively open, close, or partially close the first tubing whip; and
a vent valve area sized and shaped to receive a vent valve actuator of the aspiration thrombectomy console for interacting with the second tubing whip to selectively open, close, or partially close the second tubing whip,
wherein the cassette body is sized and shaped to be releasably received within a slot of the aspiration thrombectomy console.

2. The cassette assembly of claim 1 wherein the cassette body includes an upper cassette body part and a lower cassette body part having a clamshell arrangement.

3. The cassette assembly of claim 1 wherein the cassette body includes tracks to accommodate the manifold.

4. The cassette assembly of claim 1 wherein the first tubing whip and the second tubing whip extend through a front face of the cassette body.

5. The cassette assembly of claim 1 wherein the junction includes a t-shaped conduit.

6. The cassette assembly of claim 1, further comprising electrical connectors on a rear surface of the cassette body configured to form an electrical connection with the aspiration thrombectomy console when the cassette body is positioned within the slot.

7. The cassette assembly of claim 6 wherein the electrical connectors include a first electrical plate and a second electrical plate.

8. The cassette assembly of claim 1, further comprising a releasable lock part to releasably retain the cassette assembly within the slot.

9. The cassette assembly of claim 8 wherein the releasable lock part includes an orifice or indentation in the cassette body.

10. An aspiration thrombectomy system, comprising:
the cassette assembly of any of claims 1-9, and
the aspiration thrombectomy console.

11. The aspiration thrombectomy system of claim 10 wherein, when the cassette assembly is positioned within the slot, the vacuum valve actuator projects into the vacuum valve area and the vent valve actuator projects into the vent valve area.

12. The aspiration thrombectomy system of claim 10 wherein the cassette assembly includes the releasable lock part of claim 8, and wherein the aspiration thrombectomy console includes a second lock part within the slot for interacting with the releasable lock part.

13. The aspiration thrombectomy system of claim 12 wherein the releasable lock part and the second lock part include a ball detent and/or a latch.

14. The aspiration thrombectomy system of claim 12 wherein the aspiration thrombectomy console includes an ejection button for releasing the cassette assembly from the slot.

15. The aspiration thrombectomy system of claim 10 wherein the aspiration thrombectomy console includes a microswitch in the slot configured to detect proper seating of the cassette assembly within the slot.
